# EUROPEAN PATENT APPLICATION

(11) **EP 4 538 260 A1**
(43) Date of publication of application: **16.04.2025**
(21) Application number: 23854140.3
(22) Date of filing: 04.07.2023
(51) Int. Cl.: C07D 253/075, A61K 31/53, A61K 31/192, A61P 5/16, A61P 5/00

(54) **POLYCYCLIC THYROID HORMONE ß RECEPTOR AGONIST AND USE THEREOF**

(30) Priority: 19.08.2022 CN 202210999229
(71) Applicant: Cascade Pharmaceuticals, Inc., Shanghai 201422 (CN)
(72) Inventor: SHI, Jingjing, Shanghai 201422 (CN); ZHAO, Yishuang, Shanghai 201422 (CN); ZHANG, Zhenwei, Shanghai 201422 (CN); YANG, Shengsheng, Shanghai 201422 (CN); GONG, Linpei, Shanghai 201422 (CN); WANG, Peng, Shanghai 201422 (CN); ZHU, Xin, Shanghai 201422 (CN); ZHANG, Zheng, Shanghai 201422 (CN)
(74) Representative: Hoefer & Partner Patentanwälte mbB
(86) International application number: PCT/CN2023/105635
(87) International publication number: WO 2024/037235

(57) **Abstract**

Provided are a polycyclic thyroid hormone β receptor agonist and the use thereof. Specifically, the present invention relates to a compound as represented by formula (1) or a pharmaceutically acceptable form thereof, a pharmaceutical composition containing same, and a preparation method therefor and the use thereof. The compound or pharmaceutical composition can be used for the preparation of a drug for preventing, treating or alleviating diseases regulated by the thyroid hormone β receptor.

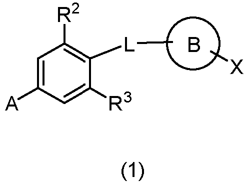

## Description

### CROSS-REFERENCE TO RELATED APPLICATION

The present application claims the priority to and the benefit of Chinese Patent Application No. 202210999229.5 filed with the National Intellectual Property Administration, PRC on August 19, 2022, the entire contents of which are incorporated herein by reference.

### TECHNICAL FIELD

The present disclosure belongs to the field of pharmaceutical chemistry, and relates to a polycylic compound as a thyroid hormone β receptor agonist, a pharmaceutical composition comprising the same, a preparation method therefor, and use thereof in the preparation of a medicament for preventing, treating or alleviating a disease regulated by a thyroid hormone β receptor.

### BACKGROUND

Thyroid hormone (TH) is synthesized in thyroid in response to the thyroid stimulating hormone (TSH) secreted by pituitary. Thyroxine plays a very important role in regulating body growth, development, metabolism, and homeostasis. Thyroid hormone exerts its functions by binding to thyroid hormone receptors (THRs). THRs belong to the nuclear receptor superfamily. Nuclear receptor, together with its common ligand - retinoid X receptor, forms a heterodimer that functions as a ligand-induced transcription factor. Like the other nuclear receptors, THRs have a ligand-binding domain and a DNA-binding domain, and regulate gene expression through their ligand-dependent interactions with the DNA response elements (thyroid hormone response elements, THREs).

There are currently two subtypes of THR: THRα and THRβ. THRα is mainly distributed in the heart tissue and plays an important regulatory role in the functions of the heart. THRβ is expressed primarily in the liver and hypophysis cerebri, regulates the metabolism of fatty acids and cholesterol, and regulates secretion of the thyroid stimulating hormone. Both THRα and THRβ are expressed in the brown adipose tissue (BAT), and play crucial roles in regulating basal oxygen consumption, fat storage, adipogenesis, and lipolysis (Oppenheimer et al., J. Clin. Invest. 87(1): 125-32 (1991)).

THR agonist may increase the metabolic rate, oxygen consumption, and thermogenesis, promote the metabolism of cholesterol into bile acids, and may additionally reduce the lipoprotein level associated with atherosclerosis. The liver and heart are the main target organs of the THR agonist. In the liver, the THR agonist primarily regulates the genes related to the synthesis and metabolism of fatty acids and cholesterol, and exerts impacts on carbohydrates by increasing glycogenolysis and glyconeogenesis and lowering the effect of insulin. In the heart, it may reduce systemic vascular resistance, increase blood volume, and generate inotropic and chronotropic effects.

THRβ agonist can also improve cellular lipid metabolism and exert the effects of lowering cholesterol and blood lipids. Therefore, it is of great significance to research and develop a THRβ agonist for treating and/or preventing diseases regulated by thyroid hormone receptors.

### SUMMARY

After extensive studies, the present disclosure has discovered a series of polycyclic compounds as thyroid hormone β receptor agonists having potential value in preventing and/or treating diseases regulated by thyroid hormone β receptors.

In the first aspect, the present disclosure provides a compound having a structure of formula (1) or a pharmaceutically acceptable form thereof: 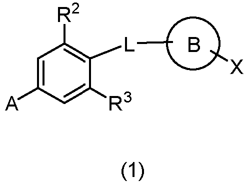 wherein
A is 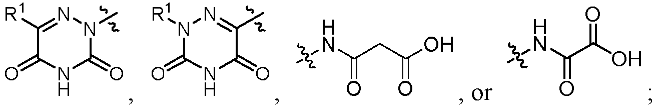
R¹ is H, halogen, -CN, -NH₂, -NO₂, -OH, or C₁₋₆ alkyl, said C₁₋₆ alkyl is optionally substituted with one or more substituents that are independently deuterium, halogen, -CN, -NH₂, -NO₂, or -OH;
R² and R³ are independently H, halogen, -CN, -NH₂, -NO₂, -OH, or C₁₋₆ alkyl, said C₁₋₆ alkyl is optionally substituted with one or more substituents that are independently halogen, -CN, -NH₂, -NO₂, or -OH;
L is -(C₁₋₄ alkylene)-, -(C₁₋₄ alkylene)-O-, -(C₁₋₄ alkylene)-S-, -(C₁₋₄ alkylene)-NH-, -O-(C₁₋₄ alkylene)-, -S-(C₁₋₄ alkylene)-, -NH-(C₁₋₄ alkylene)-, or -CH=CH-; said alkylene is optionally substituted with one or more substituents that are independently deuterium, halogen, -CN, -NH₂, -NO₂, or -OH;
ring B is a benzene ring, a naphthalene ring, a furan ring, a thiophene ring, or a pyrrole ring; ring B is optionally substituted with one or more R⁴;
each R⁴ is independently H, halogen, -CN, -NH₂, -NO₂, -OH, C₁₋₆ alkyl, C₁₋₆ alkoxy, 5- to 10-membered heteroaryl, C₅₋₈ cycloalkenyl, or C₃₋₈ cycloalkyl, said C₁₋₆ alkyl, C₁₋₆ alkoxy, 5-to 10-membered heteroaryl, or C₃₋₈ cycloalkyl is optionally substituted with one or more substituents that are independently halogen, -CN, -NH₂, -NO₂, or -OH;
X is -C(=O)NR⁵R⁶, -COOH, or 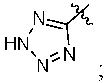
R⁵ and R⁶ are independently H, -OH, -S(=O)₂R⁷, C₁₋₆ alkyl, C₁₋₆ alkoxy, C₆₋₁₀ aryl, or C₃₋₈ cycloalkyl, said -S(=O)₂R⁷, C₁₋₆ alkyl, C₁₋₆ alkoxy, C₆₋₁₀ aryl, or C₃₋₈ cycloalkyl is optionally substituted with one or more substituents that are independently deuterium, halogen, -CN, -NH₂, -NO₂, or -OH;
R⁷ is H or C₁₋₆ alkyl; and
the pharmaceutically acceptable form is selected from the group consisting of a pharmaceutically acceptable salt, ester, stereoisomer, tautomer, solvate, nitrogen oxide, isotope-labelled compound, metabolite, and prodrug.

In some embodiments, the present disclosure provides a compound having a structure of formula (1) or a pharmaceutically acceptable form thereof: 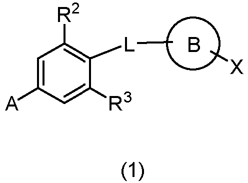 wherein 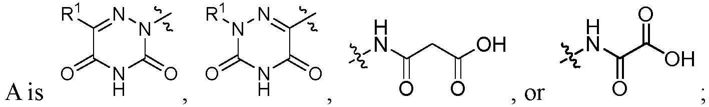
R¹ is H, halogen, -CN, -NH₂, -NO₂, -OH, or C₁₋₆ alkyl, said C₁₋₆ alkyl is optionally substituted with one or more substituents that are independently halogen, -CN, -NH₂, -NO₂, or -OH;
R² and R³ are independently H, halogen, -CN, -NH₂, -NO₂, -OH, or C₁₋₆ alkyl, said C₁₋₆ alkyl is optionally substituted with one or more substituents that are independently halogen, -CN, -NH₂, -NO₂, or -OH;
L is -(C₁₋₄ alkylene)-, -(C₁₋₄ alkylene)-O-, -(C₁₋₄ alkylene)-S-, -(C₁₋₄ alkylene)-NH-, -O-(C₁₋₄ alkylene)-, -S-(C₁₋₄ alkylene)-, -NH-(C₁₋₄ alkylene)-, or -CH=CH-; said alkylene is optionally substituted with one or more substituents that are independently deuterium, halogen, -CN, -NH₂, -NO₂, or -OH;
ring B is a benzene ring, a naphthalene ring, a furan ring, a thiophene ring, or a pyrrole ring; ring B is optionally substituted with one or more R⁴;
each R⁴ is independently H, halogen, -CN, -NH₂, -NO₂, -OH, C₁₋₆ alkyl, C₁₋₆ alkoxy, 5- to 10-membered heteroaryl, C₅₋₈ cycloalkenyl, or C₃₋₈ cycloalkyl, said C₁₋₆ alkyl, C₁₋₆ alkoxy, 5-to 10-membered heteroaryl, or C₃₋₈ cycloalkyl is optionally substituted with one or more substituents that are independently halogen, -CN, -NH₂, -NO₂, or -OH;
X is -C(=O)NR⁵R⁶, -COOH, or 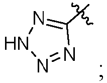
R⁵ and R⁶ are independently H, -OH, -S(=O)₂R⁷, C₁₋₆ alkyl, C₁₋₆ alkoxy, C₆₋₁₀ aryl, or C₃₋₈ cycloalkyl, said -S(=O)₂R⁷, C₁₋₆ alkyl, C₁₋₆ alkoxy, C₆₋₁₀ aryl, or C₃₋₈ cycloalkyl is optionally substituted with one or more substituents that are independently deuterium, halogen, -CN, -NH₂, -NO₂, or -OH;
R⁷ is H or C₁₋₆ alkyl; and
the pharmaceutically acceptable form is selected from the group consisting of a pharmaceutically acceptable salt, ester, stereoisomer, tautomer, solvate, nitrogen oxide, isotope-labelled compound, metabolite, and prodrug.

In some embodiments, R¹ is H, F, Cl, Br, -CN, -NH₂, or C₁₋₄ alkyl, said C₁₋₄ alkyl is optionally substituted with one or more substituents that are independently deuterium, F, Cl, Br, -CN, -NH₂, or -OH.

In some preferred embodiments, R¹ is H, -CN, -NH₂, -CH₃, -CH₂F, -CHF₂, -CDF₂, or -CF₃.

In some embodiments, R¹ is H, F, Cl, Br, -CN, -NH₂, or C₁₋₄ alkyl, said C₁₋₄ alkyl is optionally substituted with one or more substituents that are independently F, Cl, Br, -CN, -NH₂, or -OH.

In some preferred embodiments, R¹ is H, -CN, -NH₂, -CH₃, -CH₂F, -CHF₂, or -CF₃.

In some embodiments, A is

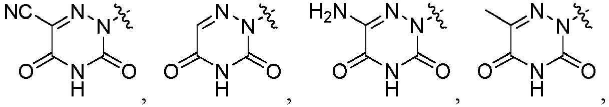

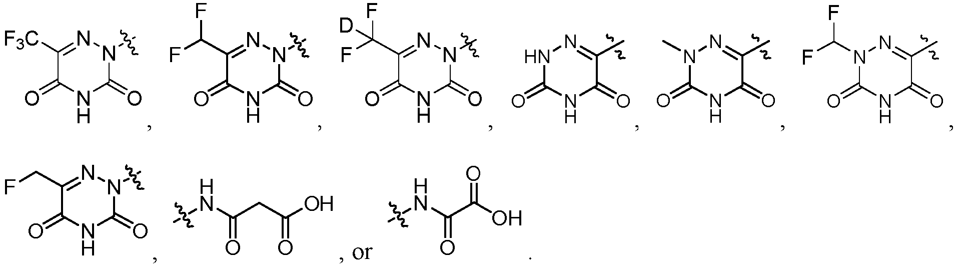

In some embodiments, A is

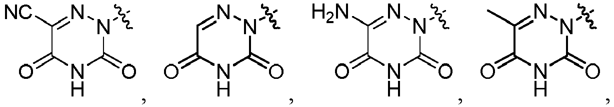

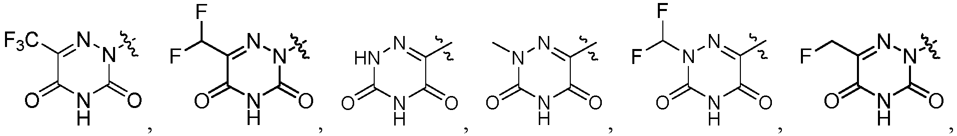

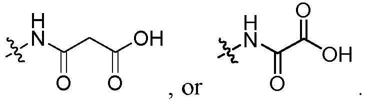

In some embodiments, R² and R³ are independently H, F, Cl, Br, -CN, -NH₂, or C₁₋₄ alkyl, said C₁₋₄ alkyl is optionally substituted with one or more substituents that are independently F, Cl, Br, -CN, -NH₂, -NO₂, or -OH.

In some preferred embodiments, R² and R³ are independently H, F, Cl, Br, or -CH₃.

In some embodiments, L is -(C₁₋₃ alkylene)-, -(C₁₋₃ alkylene)-O-, -(C₁₋₃ alkylene)-S-, -(C₁₋₃ alkylene)-NH-, -O-(C₁₋₃ alkylene)-, -S-(C₁₋₃ alkylene)-, -NH-(C₁₋₃ alkylene)-, or -CH=CH-; said alkylene is optionally substituted with one or more substituents that are independently deuterium, F, Cl, Br, or -OH.

In some preferred embodiments, L is -C(D)H-O-, -CD₂-O-, -CH₂-O-, -CH₂-S-, -CH₂-NH-, -CH₂-CH₂-, -O-CH₂-, -S-CH₂-, -NH-CH₂-, or -CH=CH-.

In some embodiments, L is -CD₂-O-, -CH₂-O-, -CH₂-S-, -CH₂-NH-, -CH₂-CH₂-, -O-CH₂-, -S-CH₂-, -NH-CH₂-, or -CH=CH-.

In some embodiments, ring B is a benzene ring, a naphthalene ring, or a thiophene ring; ring B is optionally substituted with one or more R⁴.

In some preferred embodiments, ring B is 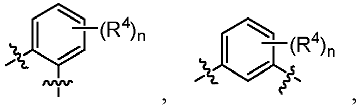 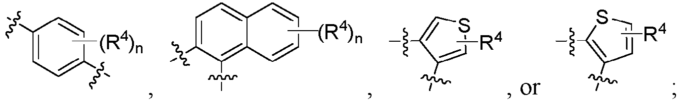 n is 0, 1, 2 or 3.

In some embodiments, each R⁴ is independently H, F, Cl, Br, -CN, -NH₂, C₁₋₄ alkyl, C₁₋₄ alkoxy, 5- to 8-membered heteroaryl, C₅₋₈ cycloalkenyl, or C₃₋₆ cycloalkyl, said C₁₋₄ alkyl, C₁₋₄ alkoxy, 5- to 8-membered heteroaryl, C₅₋₈ cycloalkenyl, or C₃₋₆ cycloalkyl is optionally substituted with one or more substituents that are independently F, Cl, Br, -CN, -NH₂, or -OH.

In some preferred embodiments, each R⁴ is independently H, F, Cl, Br, -CN, -CH₃, -OCH₃,

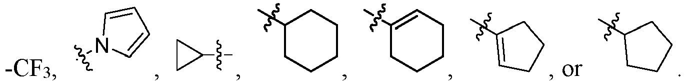

In some embodiments, R⁵ and R⁶ are independently H, -OH, -S(=O)₂R⁷, C₁₋₄ alkyl, C₁₋₄ alkoxy, C₆₋₁₀ aryl, or C₃₋₆ cycloalkyl, said -S(=O)₂R⁷, C₁₋₄ alkyl, C₁₋₄ alkoxy, C₆₋₁₀ aryl, or C₃₋₆ cycloalkyl is optionally substituted with one or more substituents that are independently deuterium, F, Cl, Br, -CN, -NH₂, or -OH; R⁷ is H or C₁₋₄ alkyl.

In some preferred embodiments, R⁵ and R⁶ are independently H, -CH₃, -CD₃, -CH(CH₃)₂, -CH₂CH₃, -OCH₃, -OH, -S(=O)₂CH₃,

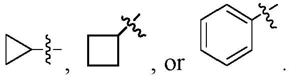

In some embodiments, the compound of formula (1) or the pharmaceutically acceptable form thereof as described above is a compound having a structure of formula (2), formula (3), formula (4) or formula (5), or a pharmaceutically acceptable form thereof: 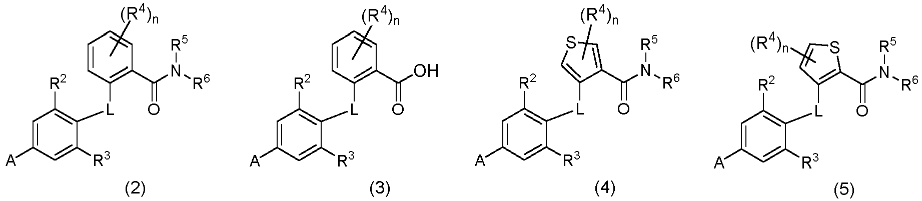 wherein A, R², R³, R⁴, L, n, R⁵, and R⁶ are as defined in formula (1).

In some embodiments, the compound of formula (1) or the pharmaceutically acceptable form thereof as described above is a compound having a structure of formula (6), formula (7), formula (8), formula (9) or formula (10), or a pharmaceutically acceptable form thereof: 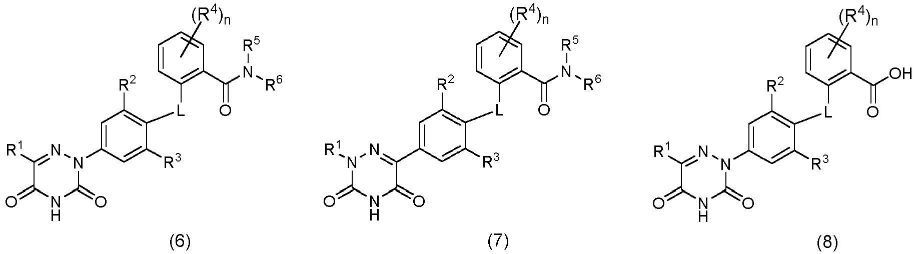 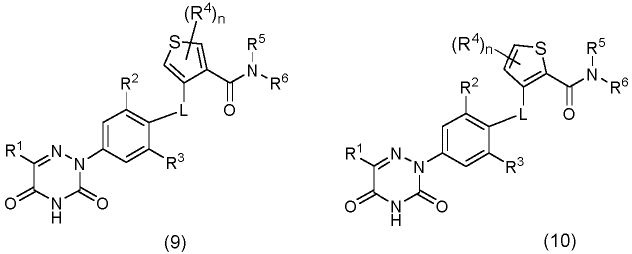 wherein R¹, R², R³, R⁴, L, n, R⁵, and R⁶ are as defined in formula (1).

In some embodiments, the compound of formula (1) or the pharmaceutically acceptable form thereof as described above is a compound having a structure of formula (11), formula (12) or formula (13), or a pharmaceutically acceptable form thereof: 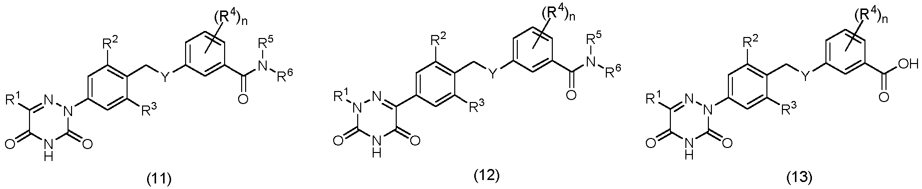 wherein Y is CH₂, O, S or NH, and R¹, R², R³, R⁴, n, R⁵, and R⁶ are as defined in formula (1).

A person skilled in the art shall appreciate that the present disclosure encompasses the compounds obtained by arbitrarily combining various embodiments. The embodiments obtained by combining the technical features or preferred technical features of one embodiment with the technical features or preferred technical features of another embodiment are also included within the scope of the present disclosure.

In the second aspect, the present disclosure further provides a compound, or a pharmaceutically acceptable salt, ester, stereoisomer, tautomer, solvate, nitrogen oxide, isotope-labelled compound, metabolite, or prodrug thereof, wherein the compound is selected from the group consisting of: 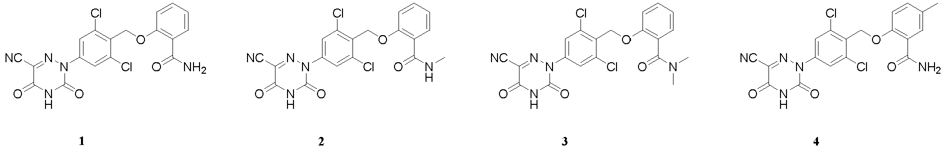 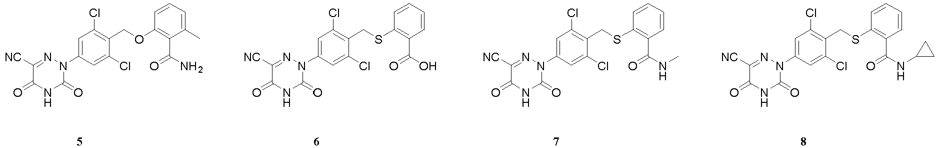 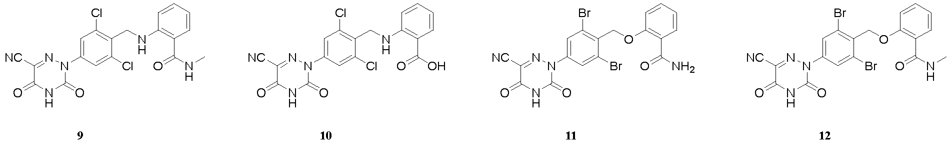 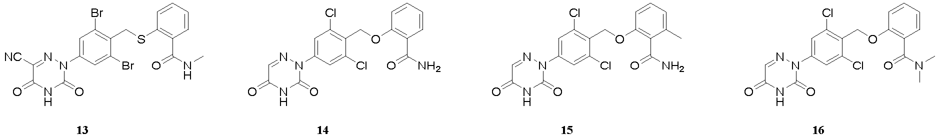 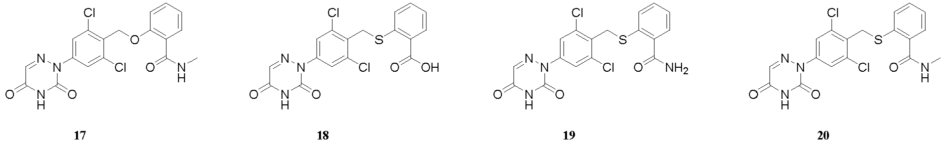 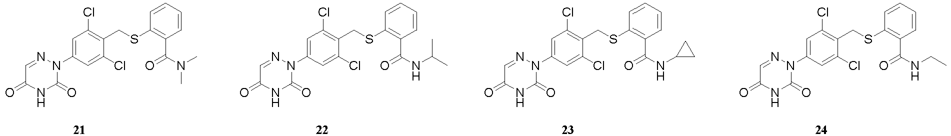 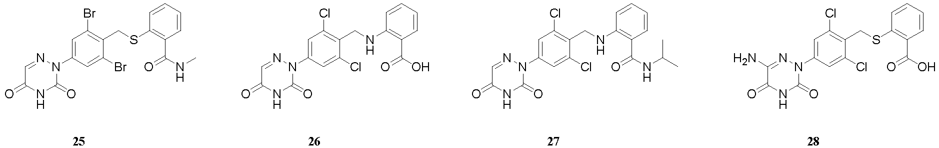 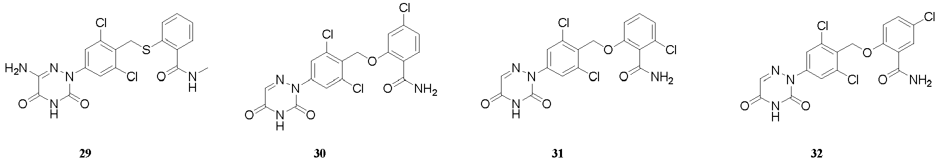 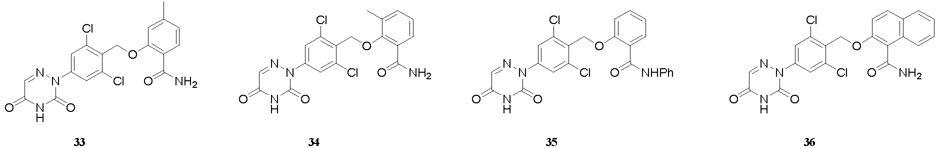 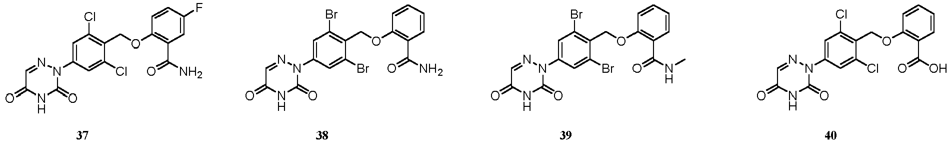 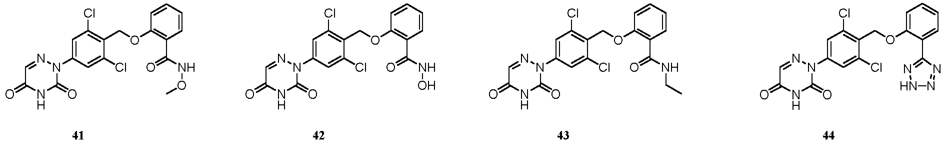 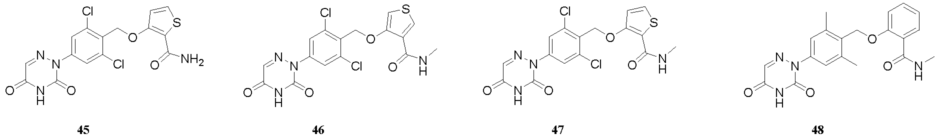 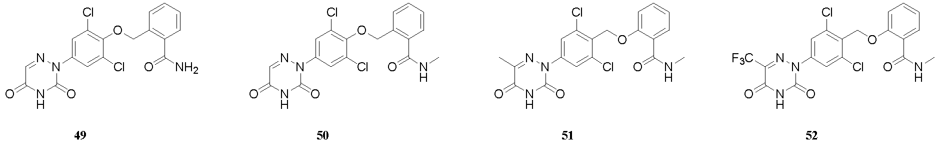 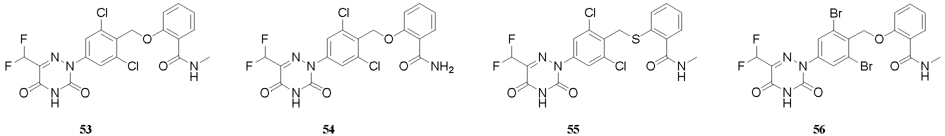 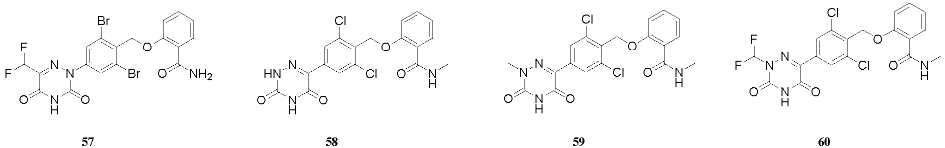 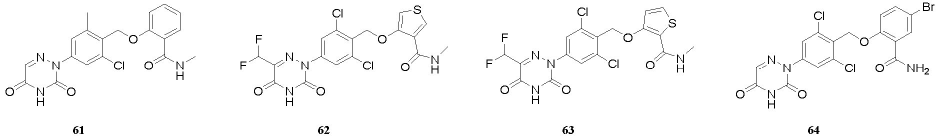 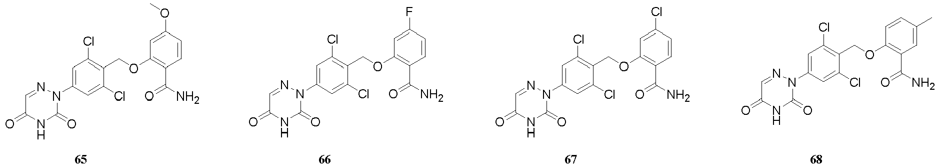 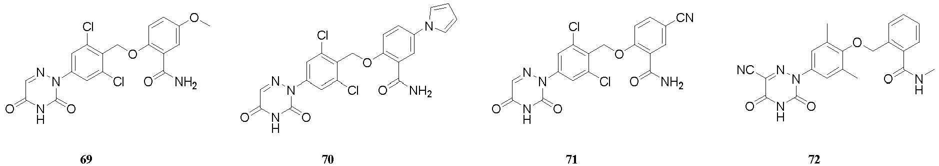 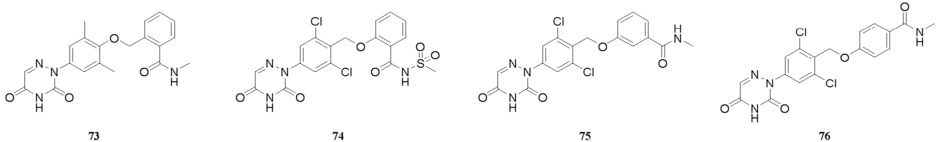 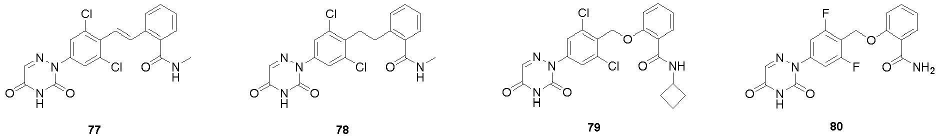 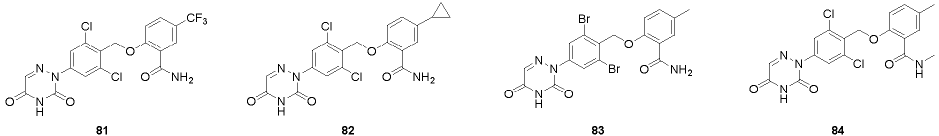 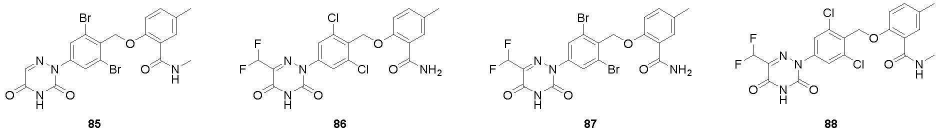 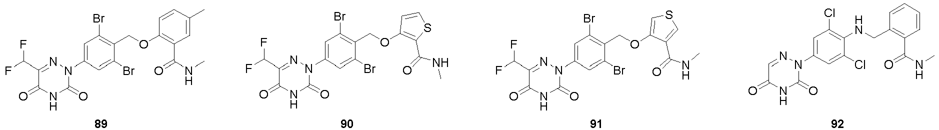 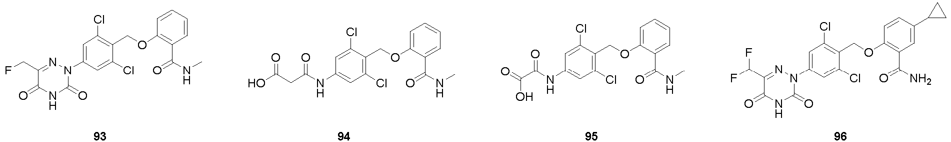 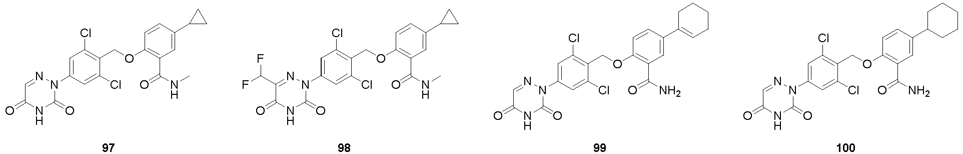 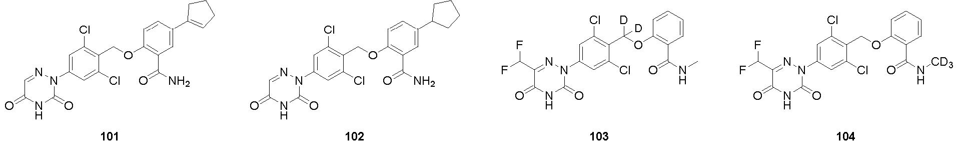 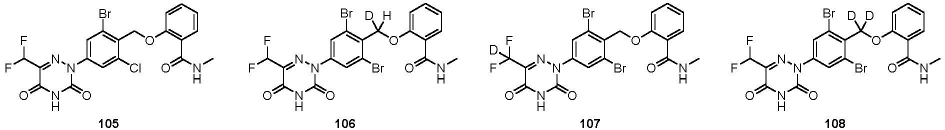 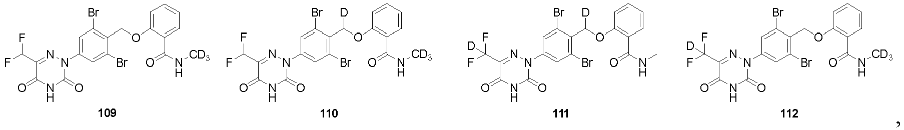 and

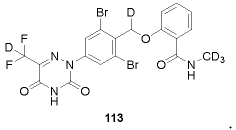

In the third aspect, the present disclosure provides a preparation method for a compound represented by formula (11), comprising the following steps:

### Step 1: Synthesis of Intermediate M1

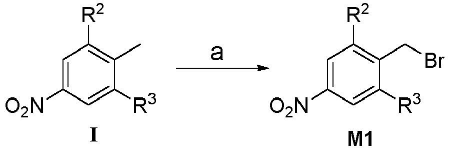

(a) A compound represented by general formula I is used as a starting material and reacts under the action of N-bromosuccinimide and a radical initiator to afford a compound represented by general formula M1.

In some embodiments, (a) in the above step 1 is conducted in the presence of a radical initiator selected from the group consisting of azobisisobutyronitrile, azobisisoheptanenitrile, cumene hydroperoxide, tert-butyl hydroperoxide, p-menthane hydroperoxide, dibenzoyl peroxide, dodecanoyl peroxide, di-tert-butyl peroxide, and dicumyl peroxide, preferably azobisisobutyronitrile.

### Step 2: Synthesis of Intermediate M2

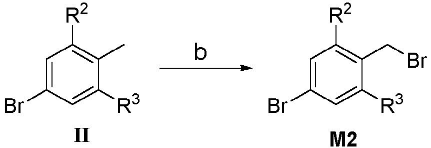

(b) A compound represented by general formula II is used as a starting material and reacts under the action of N-bromosuccinimide and a radical initiator to afford a compound represented by general formula M2.

In some embodiments, (b) in the above step 2 is conducted in the presence of a radical initiator selected from the group consisting of azobisisobutyronitrile, azobisisoheptanenitrile, cumene hydroperoxide, tert-butyl hydroperoxide, p-menthane hydroperoxide, dibenzoyl peroxide, dodecanoyl peroxide, di-tert-butyl peroxide, and dicumyl peroxide, preferably azobisisobutyronitrile.

### Step 3: Synthetic Method I for Compound Represented by Formula (11)

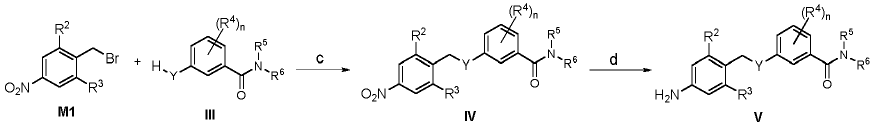

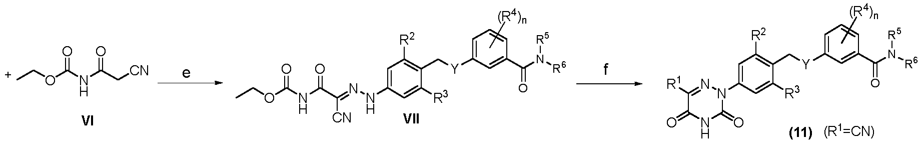

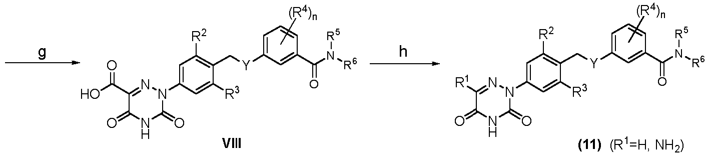

(c) the compound represented by general formula M1 is used as a starting material and reacts with a compound represented by general formula III under the action of a base to afford a compound represented by general formula IV;
(d) the compound represented by general formula IV reacts with a reducing agent to afford a compound represented by general formula V;
(e) the compound represented by general formula V is used as a starting material and reacts with an aqueous sodium nitrite solution under the action of an acid to form a diazonium salt, and then the diazonium salt reacts with a compound represented by general formula VI to afford a compound represented by general formula VII;
(f) the compound represented by general formula VII yields a compound represented by formula (11) (wherein R¹=CN) under the action of a base;
(g) the compound represented by formula (11) yields a compound represented by general formula VIII under the action of an acid; and
(h) the compound represented by general formula VIII yields a compound represented by formula (11) (wherein R¹=H or NH₂) under a different condition.

In some embodiments, (c) in the above step 3 is conducted in the presence of a base selected from the group consisting of triethylamine, N,N-diisopropylethylamine, pyridine, imidazole, 1,8-diazabicycloundec-7-ene, sodium carbonate, potassium carbonate, cesium carbonate, lithium hydroxide, sodium hydroxide, potassium hydroxide, sodium methoxide, sodium ethoxide, potassium ethoxide, potassium acetate, and sodium acetate, preferably potassium carbonate.

In some embodiments, (d) in the above step 3 is conducted in the presence of a reducing agent selected from the group consisting of iron, zinc, Raney nickel, sodium dithionite, Pd/C, Pt/C, sodium sulfide, sodium disulfide, lithium aluminum tetrahydride, and sodium borohydride, preferably sodium dithionite.

In some embodiments, (e) in the above step 3 is conducted in the presence of an acid selected from the group consisting of hydrochloric acid, acetic acid, formic acid, and sulfuric acid, preferably acetic acid.

In some embodiments, (f) in the above step 3 is conducted in the presence of a base selected from the group consisting of triethylamine, N,N-diisopropylethylamine, pyridine, imidazole, 1,8-diazabicycloundec-7-ene, sodium carbonate, potassium carbonate, cesium carbonate, lithium hydroxide, sodium hydroxide, potassium hydroxide, sodium methoxide, sodium ethoxide, potassium ethoxide, potassium acetate, and sodium acetate, preferably potassium acetate.

In some embodiments, (g) in the above step 3 is conducted in the presence of an acid selected from the group consisting of hydrochloric acid, acetic acid, formic acid, and sulfuric acid, preferably hydrochloric acid.

In some embodiments, (h) in the above step 3 is conducted in the presence of a decarboxylating agent, and the decarboxylating agent is mercaptoacetic acid, mercaptopropionic acid or mercaptobutyric acid, preferably mercaptoacetic acid.

In some embodiments, (h) in the above step 3 is conducted by reacting with diphenylphosphoryl azide in the presence of a base selected from the group consisting of triethylamine, N,N-diisopropylethylamine, pyridine, imidazole, 1,8-diazabicycloundec-7-ene, sodium carbonate, potassium carbonate, cesium carbonate, lithium hydroxide, sodium hydroxide, potassium hydroxide, sodium methoxide, sodium ethoxide, potassium ethoxide, potassium acetate, and sodium acetate, preferably triethylamine; and then undergoing a Boc deprotection step that is conducted in the presence of trifluoroacetic acid.

### Step 3': Synthetic Method II for Compound Represented by Formula (11)

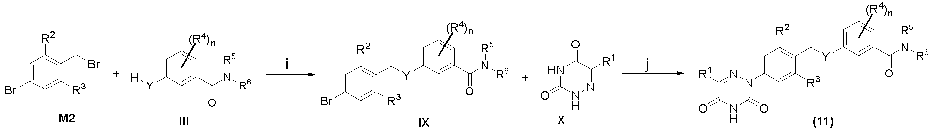

(i) the compound represented by general formula M2 is used as a starting material and reacts with the compound represented by general formula III under the action of a base to afford a compound represented by general formula IX; and
(j) the compound represented by general formula IX is used as a starting material and reacts with a compound represented by general formula X under the action of a base and a copper catalyst to afford the compound represented by formula (11).

In some embodiments, (i) in the above step 3' is conducted in the presence of a base selected from the group consisting of triethylamine, N,N-diisopropylethylamine, pyridine, imidazole, 1,8-diazabicycloundec-7-ene, sodium carbonate, potassium carbonate, cesium carbonate, lithium hydroxide, sodium hydroxide, potassium hydroxide, sodium methoxide, sodium ethoxide, potassium ethoxide, potassium acetate, and sodium acetate, preferably potassium carbonate.

In some embodiments, (j) in the above step 3' is conducted in the presence of a base selected from the group consisting of triethylamine, N,N-diisopropylethylamine, pyridine, imidazole, 1,8-diazabicycloundec-7-ene, sodium carbonate, potassium carbonate, cesium carbonate, lithium hydroxide, sodium hydroxide, potassium phosphate, potassium hydroxide, sodium methoxide, sodium ethoxide, and potassium ethoxide, preferably potassium phosphate and potassium carbonate.

In some embodiments, (j) in the above step 3' is conducted in the presence of a copper catalyst selected from the group consisting of cuprous oxide, cuprous chloride, cuprous iodide, cuprous thiocyanate, copper acetate, cuprous bromide, copper, copper oxide, copper chloride, copper bromide, and copper iodide, preferably cuprous iodide.

In the fourth aspect, the present disclosure provides a pharmaceutical composition, comprising at least one of the compounds of formula (1) to formula (13) or pharmaceutically acceptable forms thereof as described above, and one or more pharmaceutically acceptable carriers.

In the fifth aspect, the present disclosure provides the compounds of formula (1) to formula (13) or pharmaceutically acceptable forms thereof as described above, or the pharmaceutical composition described above, as a thyroid hormone β receptor agonist, for use in prevention and/or treatment of a disease or condition at least partially mediated by a thyroid hormone β receptor.

In the sixth aspect, the present disclosure provides use of the compounds of formula (1) to formula (13) or pharmaceutically acceptable forms thereof as described above, or the pharmaceutical composition described above in the preparation of a medicament for preventing and/or treating a disease or condition at least partially mediated by a thyroid hormone β receptor (for example, a metabolic disease such as a nonalcoholic fatty liver disease, dyslipidemia, atherosclerosis, or hypothyroidism).

In the seventh aspect, the present disclosure provides a method for preventing and/or treating a disease or condition at least partially mediated by a thyroid hormone β receptor, comprising a step of administering, to a subject in need thereof, a prophylactically and/or therapeutically effective amount of the compounds of formula (1) to formula (13) or pharmaceutically acceptable forms thereof as described above, or the pharmaceutical composition described above.

The present disclosure is not limited to the particular embodiments described herein. It should also be understood that the terms used herein are intended only to describe rather than limit the particular embodiments.

### Definitions of Terms

Unless otherwise specified, the meanings of following terms in the present disclosure are as follows:
The term "including", "comprising", "having" or "containing" or any other variant thereof is intended to encompass non-exclusive or open-ended inclusions. For example, a composition, method or apparatus including a series of elements is not necessarily limited only to the elements that have been explicitly recited, and may further include other elements that are not explicitly recited or the elements innate in the above composition, method or apparatus.

When the lower limit and the upper limit of a numerical range are disclosed, it means that any value or sub-range within this range is specifically disclosed. In particular, each of the numerical ranges (*e.g.,* in the form of "from about a to b", or equivalently "from approximately a to b", or equivalently "about a-b") of the parameters disclosed herein is to be understood to encompass each of the values and sub-ranges therein. For example, "C₁₋₄" should be understood to encompass any sub-range and each point value therein, *e.g.,* C₂₋₄, C₃₋₄, C₁₋₂, C₁₋₃, or C₁₋₄, etc., and C₁, C₂, C₃, C₄, etc. For another example, "5- to 10-membered" should be understood to encompass any sub-range and each point value therein, *e.g.,* 5- to 6-membered, 5- to 7-membered, 5- to 8-membered, 5- to 9-membered, 6- to 7-membered, 6- to 8-membered, etc., and 5-, 6-, 7-, 8-, 9-, 10-membered, etc.

The term "substitute" and other variations thereof used herein mean that one or more (such as 1, 2, 3, or 4) atoms or atomic groups (such as a hydrogen atom) linked to the designated atom are replaced with additional equivalent(s), provided that the valence of the designated atom or atomic group in the current situation can be kept normal and that a stable compound can be formed. If an atom or atomic group is described as being "optionally substituted with ...", it may be either substituted or unsubstituted. Unless otherwise specified, a linking position of a substituent described herein may be at any appropriate position of the substituent. When a linking bond in a substituent is shown as a chemical bond across two interconnected atoms in a ring system, it means that the substituent may be linked to any of the ring atoms in the ring system.

The term "pharmaceutical composition" refers to a composition that can be used as a medicament, comprising an active pharmaceutical ingredient (or a therapeutic agent) and one or more optional pharmaceutically acceptable carriers. The term "pharmaceutically acceptable carrier" refers to an excipient administered together with a therapeutic agent, which is suitable, within the scope of reasonable medical judgment, for contact with the tissue of human beings and/or other animals without undue toxicity, irritation, allergic response or other problems or complications commensurate with a reasonable benefit/risk ratio. Pharmaceutically acceptable carriers usable in the present disclosure include, but are not limited to, a) diluents; b) lubricants; c) binders; d) disintegrants; e) absorbents, colorants, flavoring agents, and/or sweetening agents; f) emulsifiers or dispersants; and/or g) substances that enhance absorption of the compounds.

The above-mentioned pharmaceutical compositions may act systemically and/or topically. To this end, they may be administered via suitable routes, *e.g*., parenteral, topical, intravenous, oral, subcutaneous, intra-arterial, intradermal, transdermal, rectal, intracranial, intraperitoneal, intranasal, or intramuscular route, or administered as inhalants.

The routes of administration described above can be achieved by suitable dosage forms. The dosage form usable in the present disclosure includes, but is not limited to, tablets, capsules, troches, dragees, powders, sprays, emulsions, ointments, suppositories, gels, pastes, lotions, ointments, aqueous suspensions, injectable solutions, elixirs, syrups, etc.

When administered orally, the above-mentioned pharmaceutical composition may be formulated into any orally acceptable dosage form, including but not limited to, tablets, capsules, aqueous solutions, aqueous suspensions, etc.

The above-mentioned pharmaceutical composition may also be administered in the form of a sterile injection including sterile injection water or oil suspensions or sterile injection water or oil solutions. The carriers that can be used include, but are not limited to, water, Ringer's solution, and isotonic sodium chloride solution. In addition, sterile non-volatile oils may also be used as solvents or suspension media, such as monoglycerides or diglycerides.

The above-mentioned pharmaceutical composition may comprise 0.01 mg to 1000 mg of at least one of the above compounds of formula (1) to formula (3) or pharmaceutically acceptable forms thereof.

The term "disease or condition at least partially mediated by a thyroid hormone β receptor" refers to the disease whose pathogenesis involves at least a part of factors associated with the thyroid hormone β receptor, *e.g.,* a metabolic disease such as a nonalcoholic fatty liver disease, dyslipidemia, atherosclerosis or hypothyroidism.

The term "effective amount" refers to a dose capable of inducing a biological or medical response in a cell, tissue, organ, or organism (*e.g*., a subject) and is sufficient to achieve the desired prophylactic and/or therapeutic effect.

The dosage regimen may be adjusted to provide an optimal response. For example, a single dose may be administered, divided doses may be administered over time, or the dose may be administered after it is proportionally reduced or increased as appropriate. It should be appreciated that for any particular subject, the specific dosage regimen should be adjusted according to the needs and the professional judgment of the administering practitioner.

The term "in need thereof" refers to a judgment made by a doctor or other paramedics on a subject who needs to or will benefit from the course of prevention and/or treatment, and the judgment is made based on various factors in the doctor or other paramedics' areas of expertise.

The term "subject" (or referred to as testee) refers to human beings or non-human animals. The subject of the present disclosure includes a subject (patient) with a disease and/or a condition and a normal subject. The non-human animals of the present disclosure include all vertebrates, e.g., non-mammals such as birds, amphibians, and reptiles, and mammals, e.g., non-human primates, livestock, and/or domesticated animals (such as sheep, dogs, cats, cows, and pigs).

The term "treatment" refers to alleviation or elimination of a target disease or condition. If a testee receives a therapeutic dose of the compound of the present disclosure or a pharmaceutically acceptable form thereof or the pharmaceutical composition of the present disclosure and at least one index or symptom of the testee shows observable and/or detectable alleviation and/or amelioration, this indicates that the testee has been successfully "treated". It is to be appreciated that the treatment includes not only complete treatment but also achievement of some biologically or medically relevant outcomes while not yet completing treatment. Specifically, "treatment" means that the compound of the present disclosure or a pharmaceutically acceptable form thereof or the pharmaceutical composition of the present disclosure can achieve at least one of the following effects: for example, (1) prevention of diseases occurring in animals that may be predisposed to diseases but have not yet experienced or showed pathological or symptomatological features of the diseases; (2) suppression of diseases *(i.e.,* prevention of further development in pathology and/or symptomatology) in animals that are experiencing or showing pathological or symptomatological features of the diseases; and (3) amelioration of diseases (*i.e.,* disease reversion in pathology and/or symptomatology) in animals that are experiencing or showing pathological or symptomatological features of the diseases.

The term "pharmaceutically acceptable salt" refers to salts of the compounds of the present disclosure that are substantially non-toxic to an organism. Pharmaceutically acceptable salts generally include, but are not limited to, salts formed by reacting the compounds of the present disclosure with a pharmaceutically acceptable inorganic/organic acid or inorganic/organic base, and such salts are also referred to as acid addition salts or base addition salts. For a review of suitable salts, please see, for example, Jusiak, Soczewinski, et al., Remington's Pharmaceutical Sciences [M], Mack Publishing Company, 2005 and Stahl, Wermuth, Handbook of Pharmaceutical Salts: Properties, Selection, and Use [M], Wiley-VCH, 2002**.** Methods for preparing pharmaceutically acceptable salts of the compounds of the present disclosure are known to a person skilled in the art.

The term "pharmaceutically acceptable ester" refers to esters substantially non-toxic to an organism and hydrolyzed in the organism into the compounds of the present disclosure or salts thereof. Pharmaceutically acceptable esters generally include, but are not limited to, esters formed by reacting the compounds of the present disclosure with a pharmaceutically acceptable carboxylic acid or sulfonic acid, and such esters are also known as carboxylic acid esters or sulfonic acid esters.

The term "isomer" refers to the compounds that have the same molecular weight because of the same number and type of atoms, but differ in the spatial arrangement or configuration of the atoms.

The term "stereoisomer" (or "optical isomer") refers to a stable isomer having a perpendicular and asymmetric plane due to the presence of at least one chiral element (including a chiral center, a chiral axis, and a chiral plane), thereby enabling rotation of plane-polarized light. Since the compounds of the present disclosure have asymmetric centers and other chemical structures that may lead to stereoisomerism, the present disclosure also includes these stereoisomers and mixtures thereof. Unless otherwise indicated, all stereoisomeric forms of the compounds of the present disclosure are within the scope of the present disclosure.

The term "tautomer" (or "tautomeric form") refers to structural isomers with different energies that may be interconvertible through a low energy barrier. If tautomerism is possible (*e.g.,* in a solution), a chemical equilibrium of tautomers can be achieved. For example, the causes of proton tautomers (or prototropic tautomers) include, but are not limited to, interconversion via proton migration, such as keto-enol tautomerism, imine-enamine tautomerism, and amide-iminol tautomerism. Unless otherwise indicated, all tautomeric forms of the compounds of the present disclosure are within the scope of the present disclosure.

The term "solvate" refers to a substance formed by the association of the compound of the present disclosure (or a pharmaceutically acceptable salt thereof) with at least one kind of solvent molecule through non-covalent intermolecular forces. For example, the solvate includes, but is not limited to, hydrates (including hemihydrates, monohydrates, dihydrates, trihydrates, etc.), ethanolates, and acetoneates.

The term "nitrogen oxide" refers to a compound formed by oxidation of a nitrogen atom in the structure of a tertiary amine or nitrogen-containing (aromatic) heterocyclic compound. For example, the nitrogen atom in the parent nucleus of the compound of formula I can form the corresponding nitrogen oxide.

The term "isotope-labelled compound" refers to a derivative compound formed by replacing a particular atom in the compound of the present disclosure with its isotopic atom. Unless otherwise indicated, the compounds of the present disclosure include various isotopes of H, C, N, O, F, P, S, and Cl, for example, but not limited to ²H(D), ³H(T), ¹³C, ¹⁴C, ¹⁵N, ¹⁷O, ¹⁸O, ¹⁸F, ³¹P, ³²P, ³⁵S, ³⁶S, and ³⁷Cl.

The term "metabolite" refers to a derivative compound formed after the compound of the present disclosure is metabolized. For further information on metabolism, please see Goodman and Gilman's: The Pharmacological Basis of Therapeutics (9th ed.) [M], McGraw-Hill International Editions, 1996. The present disclosure encompasses all possible metabolite forms of the compounds of the present disclosure, *i.e.,* substances formed in the body of a subject administered with the compound of the present disclosure. Metabolites of the compounds can be identified by techniques well known in the art, and their activities can be experimentally characterized.

The term "prodrug" refers to a derivative compound that, after administered to a subject, is capable of offering, directly or indirectly, the compound of the present disclosure. Particularly preferred derivative compounds or prodrugs are the compounds that, when administered to a subject, can increase the bioavailability of the compounds of the present disclosure (*e.g*., more readily absorbed into the blood), or the compounds that facilitate delivery of the parent compound to the site of action (*e.g.,* the lymphatic system). Unless otherwise indicated, all prodrug forms of the compounds of the present disclosure are within the scope of the present disclosure, and various prodrug forms are known in the art, see, *e.g.,* T. Higuchi, V. Stella, Pro-drugs as Novel Drug Delivery Systems [J], American Chemical Society, Vol. 14, 1975**.** In addition, the present disclosure further encompasses the compounds of the present disclosure containing a protective group. In any process of preparing the compounds of the present disclosure it may be necessary and/or desirable to protect sensitive or reactive groups on any of the molecules concerned, thereby forming a chemically protected form of the compounds of the present disclosure. This may be realized by conventional protective groups, such as those described in T. W. Greene, P. G. M. Wuts, Protective Groups in Organic Synthesis [M], John Wiley & Sons, 2006**.** These protective groups may be removed at an appropriate subsequent stage by the methods known in the art.

The term "independently" means that at least two groups (or ring systems) within the same or similar range in the structure may have the same or different meanings under particular circumstances. For example, if the substituent X and the substituent Y are independently hydrogen, halogen, hydroxyl, cyano, alkyl, or aryl, when the substituent X is hydrogen, the substituent Y may either be hydrogen or be halogen, hydroxyl, cyano, alkyl, or aryl; similarly, when the substituent Y is hydrogen, the substituent X may either be hydrogen or be halogen, hydroxyl, cyano, alkyl, or aryl.

When used herein alone or in combination with an additional group, the term "halogen" refers to fluorine (F), chlorine (Cl), bromine (Br), and iodine (I).

When used herein alone or in combination with an additional group, the term "alkyl" refers to linear or branched aliphatic hydrocarbyl. For example, the term "C₁₋₆ alkyl" used in the present disclosure refers to alkyl having 1 to 6 carbon atoms. For example, alkyl may be methyl, ethyl, n-propyl, isopropyl, n-butyl, sec-butyl, isobutyl, or tert-butyl.

When used herein alone or in combination with an additional group, the term "alkylene" refers to divalent, linear or branched, saturated aliphatic hydrocarbyl, and the two groups (or fragments) it links may be linked either to the same carbon atom or to different carbon atoms. For example, the term "C₁₋₄ alkylene" used herein refers to alkylene having 1 to 4 carbon atoms (such as methylene, 1,1-ethylene, 1,2-ethylene, 1,2-propylene, or 1,3-butylene).

When used herein alone or in combination with an additional group, the term "alkoxy" refers to alkyl linked to the remaining moiety of the molecule via an oxygen atom. For example, alkoxy may be methoxy, ethoxy, n-propoxy, isopropoxy, n-butoxy, isobutoxy, or tert-butoxy.

When used herein alone or in combination with an additional group, the term "cycloalkyl" refers to saturated monocyclic or polycyclic (such as bicyclic, *e.g*., fused rings, bridged rings, or spiro rings) nonaromatic hydrocarbonyl. For example, the term "C₃₋₆ cycloalkyl" used in the present disclosure refers to cycloalkyl having 3 to 6 carbon atoms. For example, cycloalkyl may be cyclopropyl, cyclobutyl, cyclopentyl, cyclohexyl, or bicyclo[2.2.1]heptyl. Cycloalkyl in the present disclosure is optionally substituted with one or more substituents described herein.

The term "C₅₋₈ cycloalkenyl" refers to monocyclic or polycyclic (such as bicyclic, e.g., fused rings, bridged rings, or spiro rings) nonaromatic hydrocarbonyl having one or more double bonds, which has 5 to 8 carbon atoms, *e.g.*, cyclopentenyl or cyclohexenyl.

When used herein alone or in combination with an additional group, the term "aryl" refers to monocyclic or fused polycyclic, aromatic hydrocarbonyl with a conjugated π-electron system. For example, the term "C₆₋₁₀ aryl" used in the present disclosure refers to aryl having 6 to 10 carbon atoms. For example, aryl may be phenyl, naphthyl, anthracenyl, phenanthrenyl, acenaphthenyl, azulenyl, fluorenyl, indenyl, or pyrenyl. The aryl of the present disclosure is optionally substituted with one or more substituents described herein.

When used herein alone or in combination with an additional group, the term "heteroaryl" refers to a monocyclic or fused polycyclic, aromatic group with a conjugated π-electron system, the ring atom of which consists of a carbon atom and at least one heteroatom selected from N, O, and S. If the valency requirements are met, the heteroaryl may be linked to the remaining moiety of the molecule via any one of the ring atoms. For example, the term "5- to 10-membered heteroaryl" used in the present disclosure refers to heteroaryl having 5 to 10 ring atoms. For example, heteroaryl may be thienyl, furyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl, triazinyl and benzene-fused derivatives thereof, pyrrolopyridinyl, pyrrolopyrazinyl, pyrazolopyridinyl, imidazopyridinyl, pyrrolopyrimidinyl, pyrazolopyrimidinyl, purinyl, etc. The heteroaryl of the present disclosure is optionally substituted with one or more substituents described herein (such as halogen or C₁₋₆ alkyl).

### DETAILED DESCRIPTION

In order to render the purposes and technical solutions of the present disclosure clearer, the embodiments of the present disclosure will be described in detail below with reference to the examples. A person skilled in the art will appreciate, however, that the following examples are intended only to illustrate the present disclosure, and should not be considered to limit the scope of the present disclosure.

The reagents or instruments used in the examples are all commercially-available conventional products. Where no specific conditions are specified, conventional conditions or those recommended by the manufacturers are followed. The term "room temperature" used herein refers to 20°C±5°C. When used to modify a value or a numerical range, the term "about" used herein is meant to include this value or numerical range and an error range of this value or numerical range acceptable to a person skilled in the art, for example, the error range is ±10%, ±5%, ±4%, ±3%, ±2%, ±1%, or ±0.5%.

The structures of the compounds described in the examples below are determined by nuclear magnetic resonance (NMR) and/or mass spectrometry (MS).

The measuring instrument for the nuclear magnetic resonance (NMR) is a Bruker 400 MHz nuclear magnetic resonance instrument. Solvents for assay are deuterated methanol (CD₃OD), deuterated chloroform (CDCl₃), and dimethyl sulfoxide-*d*₆ (DMSO-*d*₆). The internal standard is tetramethylsilane (TMS). In ¹H NMR, part of the hydrogen may not show peaks as a result of the interference from salts or solvents.

The abbreviations used for the nuclear magnetic resonance (NMR) data in the following examples have the following meanings:
s: singlet, d: doublet, t: triplet, q: quartet, dd: double doublet, qd: quartet doublet, ddd: double double doublet, ddt: double double triplet, dddd: double double double doublet, m: multiplet, br: broad, *J*: coupling constant, Hz: Hertz, *δ*: chemical shift.

All chemical shift (δ) values are given in parts per million (ppm).

The measuring instrument for mass spectrometry is an Agilent 6120B mass spectrometer with an ion source being an electrospray ion (ESI) source.

HPLC assay uses Agilent 1200 DAD high pressure liquid chromatograph (Sunfirc C18, 150X 4.6 mm, 5 µm chromatographic column) and Waters 2695-2996 high pressure liquid chromatograph (Gimini C18, 150X 4.6 mm, 5 µm chromatographic column).

Silica gel plates for thin layer chromatography are GF254 silica gel plates (Qingdao Haiyang Chemical Co., Ltd.). The silica gel plates used for the thin layer chromatography (TLC) have a thickness of 0.15 mm to 0.2 mm, and products are separated and purified by the thin layer chromatography using silica gel plates having a thickness of 0.4 mm to 0.5 mm.

Column chromatography generally uses 200 to 300-mesh silica gel (Qingdao Haiyang Chemical Co., Ltd.) as a carrier.

In the Examples, the reaction progress is monitored by thin layer chromatography (TLC). The system of the developing solvents used for the reactions includes A: dichloromethane - methanol system; and B: petroleum ether - ethyl acetate system. The volume ratio of solvents may be adjusted depending on the chemical polarity of the compounds.

The system of eluents for the column chromatography for purification of compounds and the system of the developing solvents for the thin layer chromatography include A: dichloromethane - methanol system; and B: petroleum ether - ethyl acetate system. The volume ratio of solvents may be adjusted depending on the chemical polarity of the compounds, or adjusted by adding a small amount of triethylamine and acid or alkaline reagents, etc.

### Synthesis of Compounds

### Synthesis Example 1: Synthesis of Intermediate M1a:

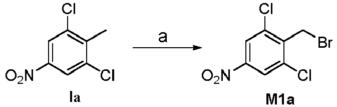

Step a: **Ia** (20 g, 50 mmol), N-bromosuccinimide (26.7 g, 150 mmol), and azobisisobutyronitrile (4 g, 25 mmol) were added to carbon tetrachloride (500 mL), heated to 80°C, and stirred overnight. Upon completion of the reaction, the resultant was directly concentrated to afford a crude product. Subsequently, the crude product was purified by column chromatography to afford compound **M1a** (26 g, yield 88.6%) as a white solid.

### Synthesis Example 2: Synthesis of Intermediate M2a:

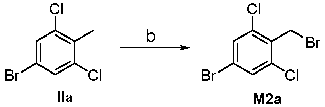

Step b: **IIa** (2.5 g, 10.4 mmol), N-bromosuccinimide (5.56 g, 32.1 mmol), and azobisisobutyronitrile (854 mg, 5.21 mmol) were added to carbon tetrachloride (40 mL), heated to 80°C, and stirred overnight. Upon completion of the reaction, the resultant was directly concentrated to afford a crude product. Subsequently, the crude product was purified by column chromatography to afford compound **M2a** (3.3 g, yield 99%) as a white solid.

¹H NMR (400 MHz, DMSO-d6) δ 7.86 (s, 2H), 4.72 (s, 2H).

### Synthesis Example 3: Synthesis of Intermediate M2b:

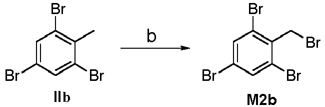

Step b: **IIb** (5 g, 15.2 mmol), N-bromosuccinimide (8.1 g, 45.7 mmol), and azobisisobutyronitrile (1.5 g, 9.1 mmol) were added to carbon tetrachloride (40 mL), heated to 80°C, and stirred overnight. Upon completion of the reaction, the resultant was directly concentrated to afford a crude product. Subsequently, the crude product was purified by column chromatography to afford compound **M2b** (5.3 g, yield 85.5%) as a white solid.

### Example 1: Synthesis of Compound 1

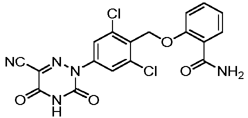

### Synthetic route:

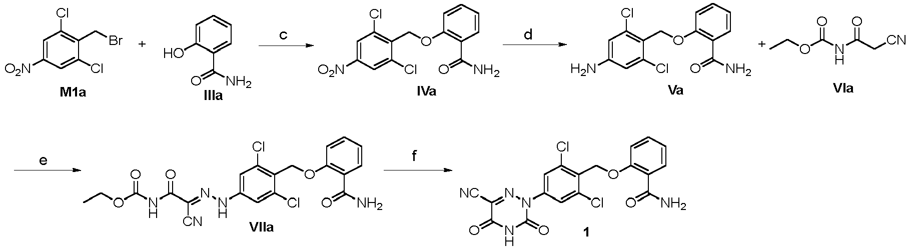

Step c: Compound **M1a** (800 mg, 2.86 mmol) and potassium carbonate (591 mg, 4.29 mmol) were added to N,N-dimethylformamide (10 mL), and then **IIIa** (391 mg, 2.86 mmol) was added to the reaction solution and stirred at room temperature for half an hour. Upon completion of the reaction, ethyl acetate (100 mL) was added. The organic phases were washed with saturated saline. The organic phases were combined and concentrated to afford a crude product. Thereafter, the crude product was purified by column chromatography to afford compound **IVa** (950 mg, yield 97.7%) as a yellow solid. MS(ESI, m/z): 341[M+H]⁺.

Step d: Compound **IVa** (950 mg, 2.79 mmol) and sodium dithionite (1.45 g, 8.36 mmol) were added to a mixed solution of tetrahydrofuran (20mL) and water (10 mL), heated to 50°C, and reacted for three hours. Upon completion of the reaction, ethyl acetate (100 mL) was added. The organic phases were washed with saturated saline. The organic phases were combined and concentrated to afford a crude product. Thereafter, the crude product was purified by column chromatography to afford a yellow oily product **Va** (794 mg, yield 91.7%). MS(ESI, m/z): 311[M+H]⁺.

Step e: Compound **Va** (794 mg, 2.55 mmol) and hydrochloric acid (280 mg, 7.66 mmol) were dissolved into acetic acid (10 mL), cooled to 0°C, and stirred for 10 min. The aqueous solution (2 mL) of sodium nitrite (194 mg, 2.81 mmol) was added dropwise and stirred at 0°C for 30 min, and then N-cyanoacetylurethane (438 mg, 2.81 mmol) was added to the reaction solution and reacted at room temperature for one hour. Upon completion of the reaction, ethyl acetate (200 mL) was added. The organic phases were washed with a saturated sodium bicarbonate solution and saturated saline. The organic phases were combined and concentrated to afford a crude product. Thereafter, the crude product was purified by column chromatography to afford a yellow oily product **VIIa** (600 mg, yield 50%), MS(ESI, m/z): 478[M+H]⁺.

Step f: Compound **VIIa** (600 mg, 1.26 mmol) and potassium acetate (148 mg, 1.51 mmol) were added to N,N-dimethylacetamide (10 mL) and stirred, heated to 110°C, and stirred overnight. Upon completion of the reaction, the resultant was directly purified by column chromatography to afford a white solid 1 (11 mg, yield 2.0%), MS(ESI, m/z): 432[M+H]⁺.

¹H NMR (400 MHz, DMSO-d6) δ 7.78 (d, *J =* 7.6 Hz, 1H), 7.75 (s, 2H), 7.53 (t, *J =* 7.2 Hz, 1H), 7.44 (s, 1H), 7.38 (d, *J =* 8.0 Hz, 1H), 7.21 (s, 1H), 7.10 (t, *J =* 7.6 Hz, 1H), 5.40 (s, 2H).

### Example 2: Synthesis of Compound 2

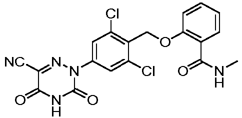

### Synthetic route:

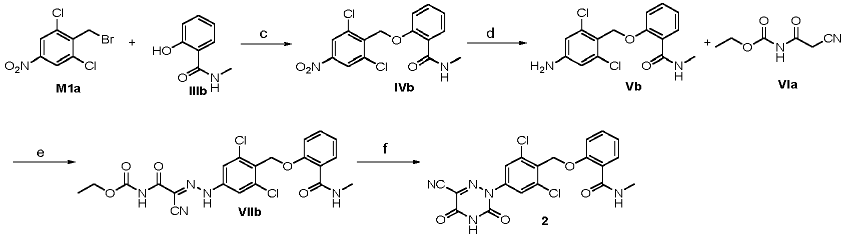

The raw material **IIIa** was replaced with **IIIb**, and then the synthetic method of Example **1** was followed to afford compound 2 (56 mg, yield 7.82%) as a yellow solid. MS(ESI, m/z): 446[M+H]⁺.

¹H NMR (400 MHz, DMSO-d6) δ 7.87 - 7.82 (m, 1H), 7.74 (s, 2H), 7.63 (dd, *J =* 7.6 Hz, 1.6 Hz, 1H), 7.51 - 7.47 (m, 1H), 7.37 (d, *J =* 8.4 Hz, 1H), 7.09 (t, *J =* 7.6 Hz, 1H), 5.37 (s, 2H), 2.68 (d, *J =* 4.8 Hz, 3H).

### Example 3: Synthesis of Compound 3

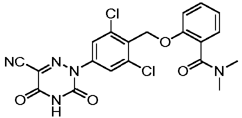

### Synthetic route:

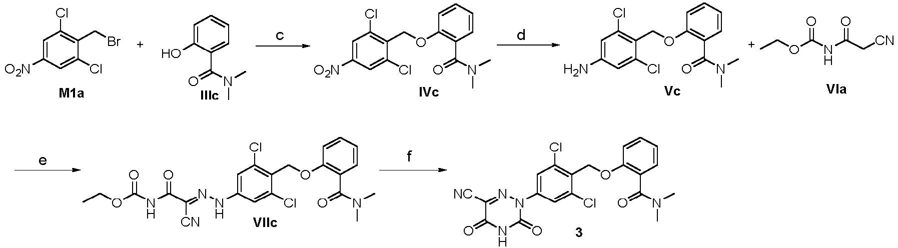

The raw material **IIIa** was replaced with **IIIc,** and then the synthetic method of Example **1** was followed to afford compound **3** (80.8 mg, yield 12.0%) as a yellow solid. MS(ESI, m/z): 460[M+H]⁺.

¹H NMR (400 MHz, DMSO-d6) δ 7.72 (s, 2H), 7.45 - 7.41 (m, 1H), 7.33 (d, *J =* 8.0 Hz, 1H), 7.16 (dd, *J =* 7.6 Hz, 1.6 Hz, 1H), 7.06 (t, *J =* 7.2 Hz, 1H), 5.28 (s, 2H), 2.84 (s, 3H), 2.69 (s, 3H).

### Example 4: Synthesis of Compound 4

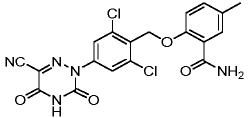

### Synthetic route:

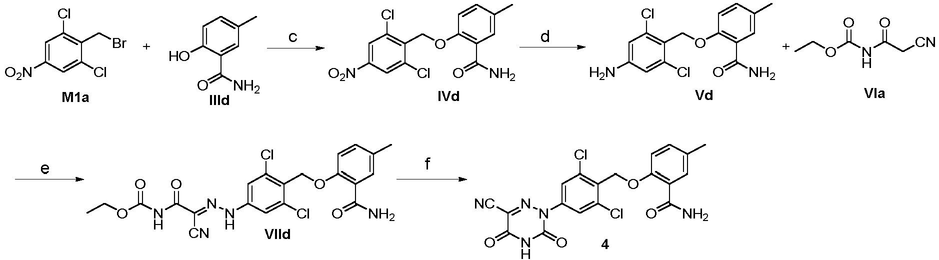

The raw material **IIIa** was replaced with **IIId**, and then the synthetic method of Example **1** was followed to afford compound **4** (14.8 mg, yield 15.0%) as a yellow solid. MS(ESI, m/z): 446[M+H]⁺.

¹H NMR (400 MHz, DMSO-d6) δ 7.74 (s, 2H), 7.60 (d, *J =* 2.0 Hz, 1H), 7.40 (s, 1H), 7.32 (dd, *J =* 7.6 Hz, 2.0 Hz, 1H), 7.26 (d, *J =* 8.0 Hz, 1H), 7.20 (s, 1H), 5.35 (s, 2H), 2.28 (s, 3H).

### Example 5: Synthesis of Compound 5

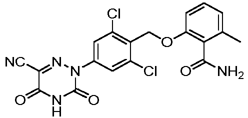

### Synthetic route:

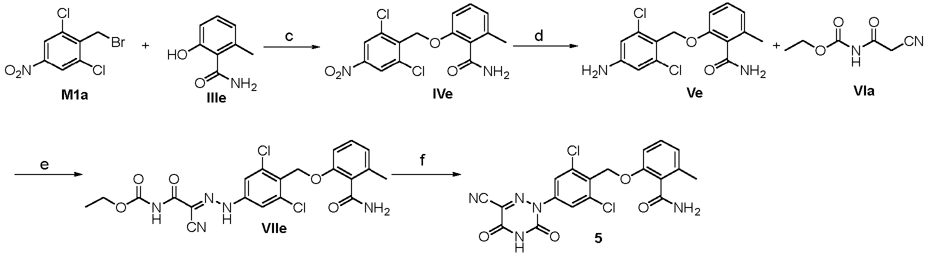

The raw material **IIIa** was replaced with **IIIe**, and then the synthetic method of Example **1** was followed to afford compound **5** (17.15 mg, yield 8.1%) as a yellow solid. MS(ESI, m/z): 446[M+H]⁺.

¹H NMR (400 MHz, DMSO-d6) δ 7.71 (s, 2H), 7.50 (s, 1H), 7.27 - 7.23 (m, 2H), 7.09 (d, *J =* 8.4 Hz, 1H), 6.87 (d, *J =* 7.6 Hz, 1H), 5.23 (s, 2H), 2.22 (s, 3H).

### Example 6: Synthesis of Compound 6

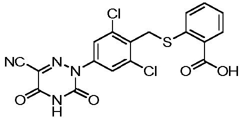

### Synthetic route:

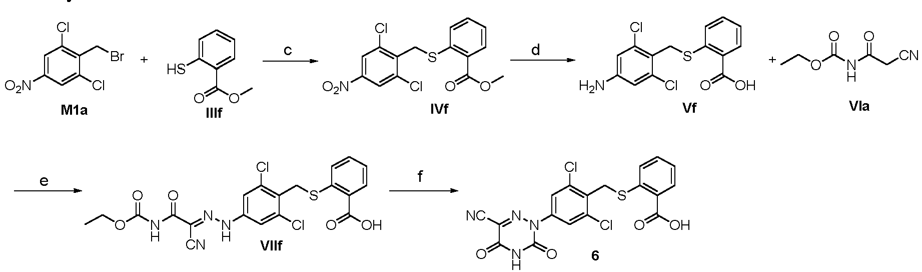

The raw material **IIIa** was replaced with **IIIf**, and then the synthetic method of Example **1** was followed to afford compound 6 (2.89 mg, yield 6.5%) as a yellow solid. MS(ESI, m/z): 449[M+H]⁺.

¹H NMR (400 MHz, DMSO-d6) δ 7.92 (d, *J =* 8.4 Hz, 1H), 7.70 (s, 2H), 7.60-7.57 (m, 2H), 7.31 - 7.26 (m, 1H), 4.40 (s, 2H).

### Example 7: Synthesis of Compound 7

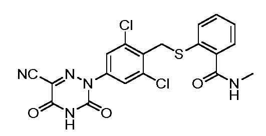

### Synthetic route:

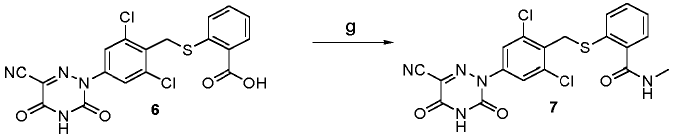

Step g: Compound **6** (50 mg, 0.112 mmol), methylamine hydrochloride (18 mg, 0.268 mmol), and HATU (51 mg, 0.134 mmol) were dissolved into N,N-dimethylformamide (2 mL). Afterwards, triethylamine (34 mg, 0.335 mmol) was added to the reaction solution and stirred at room temperature for one hour. Upon completion of the reaction, 1N diluted hydrochloric acid solution was added to adjust the pH value to 4 to 5, and ethyl acetate (100 mL) was added. The organic phases were washed with saturated saline. The organic phases were combined and concentrated to afford a crude product. Thereafter, the crude product was purified by column chromatography to afford compound **7** (35 mg, yield 68%) as a white solid, MS(ESI, m/z): 462[M+H]⁺.

¹H NMR (400 MHz, DMSO-d6) δ 8.28 - 8.23 (m, 1H), 7.65 (s, 2H), 7.51 - 7.49 (m, 1H), 7.45 - 7.43 (m, 2H), 7.31 - 7.27 (m, 1H), 4.38 (s, 2H), 2.71 (d, *J =* 4.0 Hz, 3H).

### Example 8: Synthesis of Compound 8

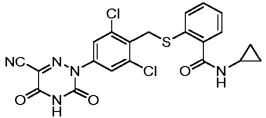

### Synthetic route:

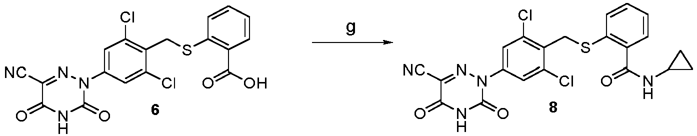

The synthetic method of Example 7 was followed to afford compound **8** (12 mg, yield 60%) as a white solid. MS(ESI, m/z): 488[M+H]⁺.

¹H NMR (400 MHz, DMSO-d6) δ 13.25 (s, 1H), 8.37 (d, *J =* 4.4 Hz, 1H), 7.66 (s, 2H), 7.51 - 7.49 (m,1H), 7.44 - 7.39 (m, 1H), 7.29 (t, *J =* 7.6 Hz, 1H), 4.38 (s, 2H), 2.80 - 2.75 (m, 1H), 0.67 - 0.63 (m, 2H), 0.52 - 0.50 (m, 2H).

### Example 9: Synthesis of Compound 9

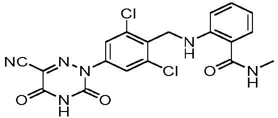

### Synthetic route:

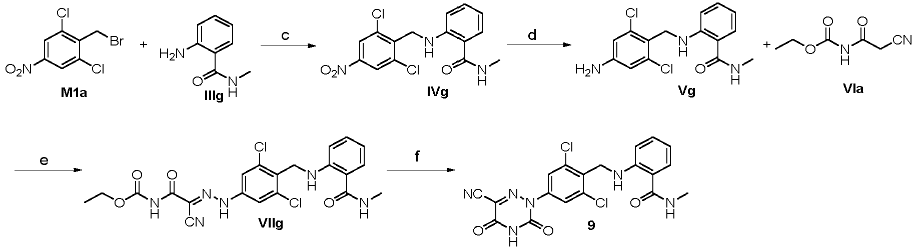

The raw material **IIIa** was replaced with **IIIg,** and then the synthetic method of Example **1** was followed to afford compound **9** (5 mg, yield 6.4%) as a brown solid. MS(ESI, m/z): 445[M+H]⁺.

¹H NMR (400 MHz, DMSO-d6) δ 8.29 (s, 1H), 8.12 (s, 1H), 7.69 (s, 2H), 7.52 (d, *J =* 8.0 Hz, 1H), 7.36 - 7.24 (m, 1H), 6.90 (d, *J =* 8.4 Hz, 1H), 6.60 (t, *J =* 7.6 Hz, 1H), 4.50 (d, *J* = 5.2 Hz, 2H), 2.67 (d, *J =* 4.4 Hz, 3H).

### Example 10: Synthesis of Compound 10

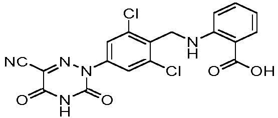

### Synthetic route:

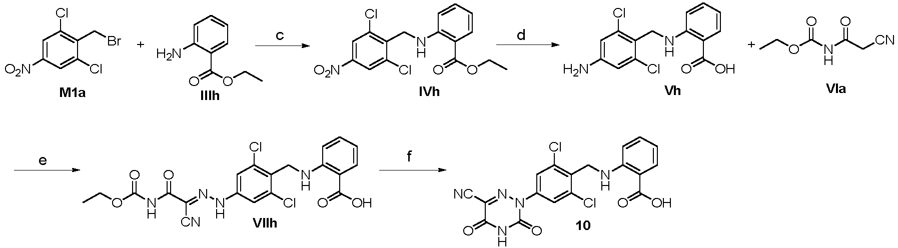

The raw material **IIIa** was replaced with **IIIh,** and then the synthetic method of Example **1** was followed to afford compound **10** (560 mg, yield: 99%) as a brown solid. MS(ESI, m/z): 431.9[M+H]⁺.

¹H NMR (400 MHz, DMSO-d6) δ 12.71 (s, 1H), 8.09 (s, 1H), 7.83 - 7.75 (m, 1H), 7.69 (s, 2H), 7.46 - 7.37 (m, 1H), 6.95 (d, *J =* 8.4 Hz, 1H), 6.62 (t, *J =* 7.6 Hz, 1H), 4.63 (d, *J =* 5.2 Hz, 2H).

### Example 11: Synthesis of Compound 11

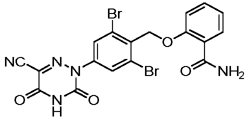

### Synthetic route:

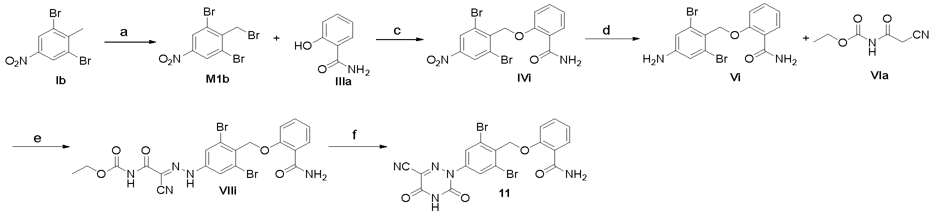

The raw material **M1a** was replaced with **M1b,** and the synthetic method of Example **1** was followed to afford compound **11** (6 mg, yield 1.2%) as a gray solid. MS(ESI, m/z): 522[M+H]⁺.

¹H NMR (400 MHz, DMSO-d6) δ 7.93 (s, 2H), 7.83 (dd, *J =* 7.6 Hz, 1.6 Hz, 1H), 7.54 (t, *J =* 8.0 Hz, 1H), 7.48 (brs, 1H), 7.40 (d, *J =* 8.4 Hz, 1H), 7.16 (brs, 1H), 7.11 (t, *J =* 7.6 Hz, 1H), 5.42 (s, 2H).

### Example 12: Synthesis of Compound 12

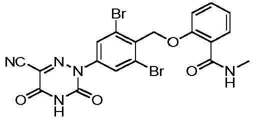

### Synthetic route:

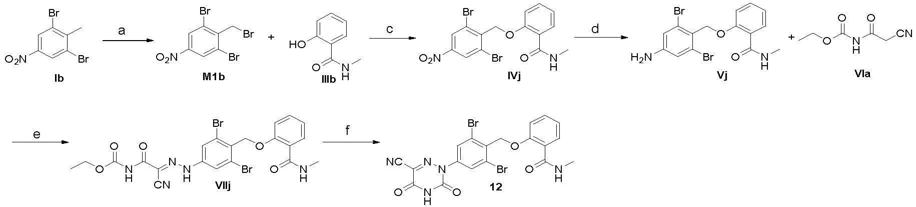

The raw material **M1a** was replaced with **M1b,** the raw material **IIIa** was replaced with **IIIb,** and the synthetic method of Example **1** was followed to afford compound **12** (64.83 mg, yield 21.5%) as a yellow solid. MS(ESI, m/z): 536[M+H]⁺.

¹H NMR (400 MHz, DMSO-d6) δ 7.95 (s, 2H), 7.80 - 7.76 (m, 1H), 7.71 (d, *J =* 7.2 Hz, 1H), 7.50 (t, *J =* 8.4 Hz, 1H), 7.39 (d, *J =* 8.4 Hz, 1H), 7.10 (t, *J =* 7.6 Hz, 1H), 5.38 (s, 2H), 2.69 (d, *J* = 4.4 Hz, 3H).

### Example 13: Synthesis of Compound 13

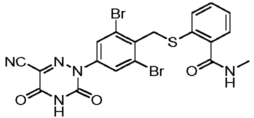

### Synthetic route:

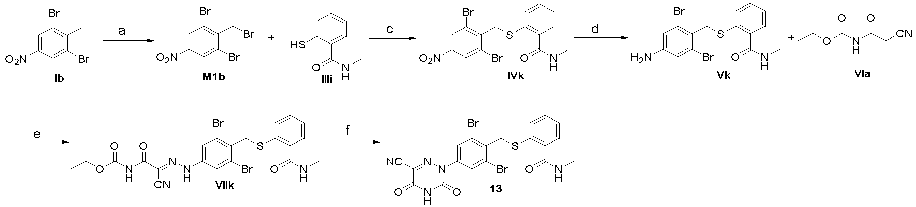

The raw material **M1a** was replaced with **M1b,** the raw material **IIIa** was replaced with **IIIi**, and the synthetic method of Example 1 was followed to afford compound **13** (64.83 mg, yield 21.5%) as a yellow solid. MS(ESI, m/z): 552[M+H]⁺.

¹H NMR (400 MHz, DMSO-d6) δ 13.24 (s, 1H), 8.29 - 8.26 (m, 1H), 7.83 (s, 2H), 7.51 (d, *J* = 7.6 Hz, 1H), 7.46 - 7.43 (m, 2H), 7.29 (d, *J =* 7.2 Hz, 1H), 4.44 (s, 2H), 2.72 (d, *J =* 4.4 Hz, 3H).

### Example 14: Synthesis of Compound 14

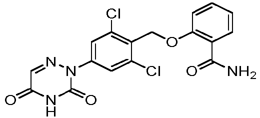

### Synthetic route:

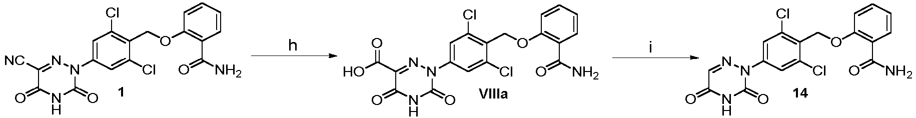

Step h: Compound **1** (410 mg, 0.95 mmol) was added to acetic acid (20 mL) and stirred. Afterwards, hydrochloric acid (1 mL) was added, heated to 120°C, and reacted overnight. Upon completion of the reaction, ethyl acetate (100 mL) was added. The organic phases were washed with saturated saline. The organic phases were combined and concentrated to afford a crude product **VIIIa** (550 mg, yield 99%), MS(ESI, m/z): 451[M+H]⁺.

Step i: Compound **VIIIa** (50 mg, 0.11 mmol) was added to mercaptoacetic acid (1 mL), heated to 170°C, and stirred for one hour. Upon completion of the reaction, the reaction solution was directly purified by column chromatography to afford a white solid **14** (3.75 mg, yield 8.3%), MS(ESI, m/z): 407[M+H]⁺.

¹H NMR (400 MHz, DMSO-d6) δ 12.49 (s, 1H), 7.81 - 7.79 (m, 3H), 7.69 (s, 1H), 7.53 (t, *J =* 8.4 Hz, 1H), 7.47 (s, 1H), 7.39 (d, *J =* 8.0 Hz, 1H), 7.22 (s, 1H), 7.11 (t, *J =* 7.6 Hz, 1H), 5.39 (s, 2H).

### Example 15: Synthesis of Compound 15

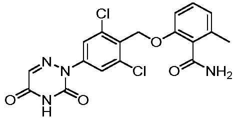

### Synthetic route:

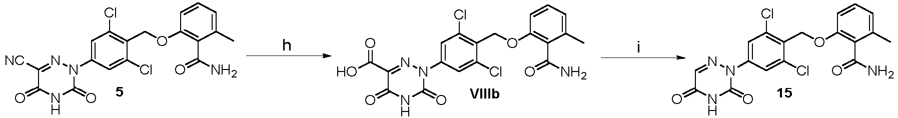

The raw material **1** was replaced with **5,** and then the synthetic method of Example **14** was followed to afford compound **15** (37.4 mg, yield 82.6%) as a yellow solid, MS(ESI, m/z): 421[M+H]⁺.

¹H NMR (400 MHz, DMSO-d6) δ 12.49 (s, 1H), 7.73 (s, 2H), 7.71 (s, 1H), 7.49 (s, 1H), 7.27 - 7.23 (m, 2H), 7.08 (d, *J =* 8.0 Hz, 1H), 6.86 (d, *J =* 7.6 Hz, 1H), 5.21 (s, 2H), 2.21 (s, 3H).

### Example 16: Synthesis of Compound 16

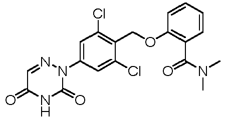

### Synthetic route:

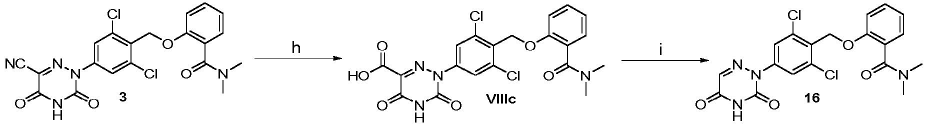

The raw material **1** was replaced with **3,** and then the synthetic method of Example **14** was followed to afford compound **16** (2.68 mg, yield 5.4%) as a yellow solid, MS(ESI, m/z): 435[M+H]⁺.

¹H NMR (400 MHz, DMSO-d6) δ 12.49 (s, 1H), 7.76 (s, 2H), 7.72 (s, 1H), 7.06 (t, *J =* 8.4 Hz, 1H), 7.34 (d, *J =* 8.0 Hz, 1H), 7.16 (dd, *J =* 7.6 Hz, 2.0 Hz, 1H), 7.06 (t, *J =* 7.2 Hz, 1H), 5.26 (s, 2H), 2.85 (s, 3H), 2.70 (s, 3H).

### Example 17: Synthesis of Compound 17

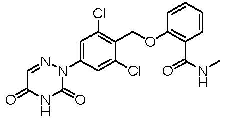

### Synthetic route:

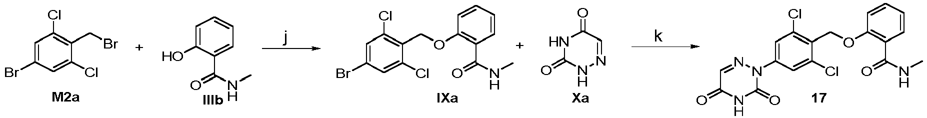

Step j: Compound **M2a** (456 mg, 1.43 mmol) and potassium carbonate (296 mg, 2.14 mmol) were added to N,N-dimethylformamide (5 mL), and then **IIIb** (216 mg, 1.43 mmol) was added to the reaction solution and stirred at room temperature for half an hour. Upon completion of the reaction, ethyl acetate (100 mL) was added. The organic phases were washed with saturated saline. The organic phases were combined and concentrated to afford a crude product. Thereafter, the crude product was purified by column chromatography to afford compound **IXa** (550 mg, yield 98.9%) as a yellow solid. MS(ESI, m/z): 388[M+H]⁺.

Step k: Compounds **IXa** (523 mg, 1.34 mmol) and **Xa** (152 mg, 1.34 mmol) were dissolved into a N,N-dimethylformamide (3 mL) solution. Thereafter, cuprous iodide (255 mg, 1.34 mmol), potassium phosphate (570 mg, 2.69 mmol), and N,N'-dimethylethylenediamine (118 mg, 1.34 mmol) were added, and heated to 120°C and reacted for two hours under nitrogen protection. The reaction solution was cooled to room temperature and directly purified by column chromatography to afford compound **17** (16 mg, yield 2.8%) as a white solid. MS(ESI, m/z): 421[M+H]⁺.

¹H NMR (400 MHz, DMSO-d6) δ 12.49 (s, 1H), 7.86 - 7.81 (m, 1H), 7.78 (s, 2H), 7.72 (s, 1H), 7.65 (dd, *J =* 7.6 Hz, 1.6 Hz, 1H), 7.49 (t, *J =* 8.0 Hz, 1H), 7.37 (d, *J =* 8.4 Hz, 1H), 7.09 (t, *J =* 7.6 Hz, 1H), 5.37 (s, 2H).

### Example 18: Synthesis of Compound 18

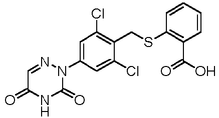

### Synthetic route:

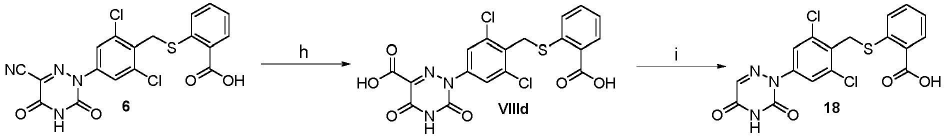

The raw material 1 was replaced with **6,** and then the synthetic method of Example **14** was followed to afford compound **18** (4.12 mg, yield 3.8%) as a yellow solid, MS(ESI, m/z): 424[M+H]⁺.

¹H NMR (400 MHz, DMSO-d6) δ 7.92 (d, *J* = 7.6 Hz, 1H), 7.73 (s, 2H), 7.70 (s, 1H), 7.60 - 7.57 (m, 2H), 7.31 - 7.26 (m, 1H), 4.39 (s, 2H).

### Example 19: Synthesis of Compound 19

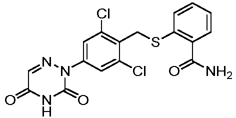

### Synthetic route:

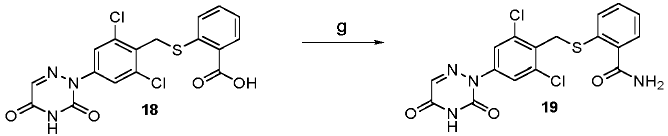

The synthetic method of Example 7 was followed to afford compound **19** (25 mg, yield 73%) as a white solid, MS(ESI, m/z): 423[M+H]⁺.

¹H NMR (400 MHz, DMSO-d6) δ 13.34 (s, 1H), 7.85 (s, 1H), 7.78 - 7.77 (m, 3H), 7.51 (t, *J= 7.6* Hz, 2H), 7.46 - 7.44 (m, 2H), 7.29 - 7.26 (m, 1H), 4.38 (s, 2H).

### Example 20: Synthesis of Compound 20

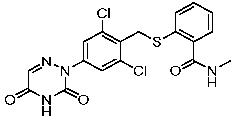

### Synthetic route:

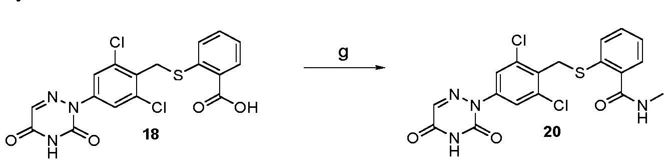

The synthetic method of Example **7** was followed to afford compound **20** (35 mg, yield 68%) as a white solid, MS(ESI, m/z): 437[M+H]⁺.

¹H NMR (400 MHz, DMSO-d6) δ 8.26 (s, 1H), 7.66 - 7.65 (m, 3H), 7.51 - 7.49 (m, 1H), 7.45 - 7.43 (m, 2H), 7.31 - 7.27 (m, 1H), 4.38 (s, 2H), 2.71 (d, *J =* 4.0 Hz, 3H).

### Example 21: Synthesis of Compound 21

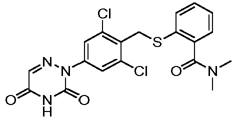

### Synthetic route:

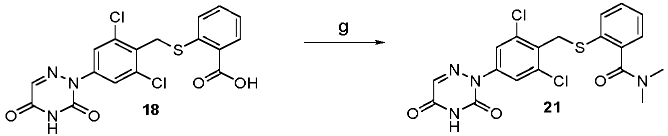

The synthetic method of Example **7** was followed to afford compound **21** (18 mg, yield 49%) as a white solid. MS(ESI, m/z): 451[M+H]⁺.

¹H NMR (400 MHz, DMSO-d6) δ 7.63 (s, 2H), 7.52 - 7.49 (m, 1H), 7.35 - 7.24 (m, 3H), 7.22 - 7.21 (m, 1H), 4.41 (s, 2H), 2.95 (s, 3H), 2.65 (s, 3H).

### Example 22: Synthesis of Compound 22

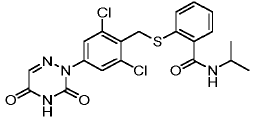

### Synthetic route:

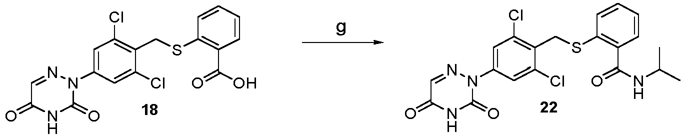

The synthetic method of Example **7** was followed to afford compound **22** (20 mg, yield 54%) as a white solid. MS(ESI, m/z): 465[M+H]⁺.

¹H NMR (400 MHz, DMSO-d6) δ 13.25 (s, 1H), 8.18 (d, *J =* 7.6 Hz, 1H), 7.66 - 7.65 (m, 3H), 7.50 (d, *J =* 8.0 Hz, 1H), 7.45 - 7.38 (m, 2H), 7.30 (t, *J =* 7.6 Hz, 1H), 4.38 (s, 2H), 4.03 - 3.95 (m, 1H), 1.11 (d, *J =* 6.8 Hz, 6H).

### Example 23: Synthesis of Compound 23

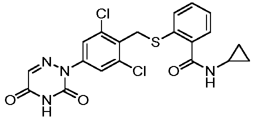

### Synthetic route:

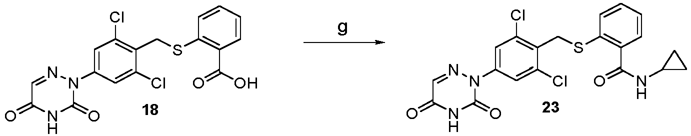

The synthetic method of Example **7** was followed to afford compound **23** (12 mg, yield 74%) as a white solid. MS(ESI, m/z): 463[M+H]⁺.

¹H NMR (400 MHz, DMSO-d6) δ 12.47 (s, 1H), 8.37 (d, *J =* 4.0 Hz, 1H), 7.70 - 7.69 (m, 3H), 7.50 (d, *J =* 8.0 Hz, 1H), 7.44 - 7.38 (m, 2H), 7.28 (t, *J =* 7.6 Hz, 1H), 4.37 (s, 2H), 2.80 - 2.74 (m, 1H), 0.67 - 0.62 (m, 2H), 0.52-0.48 (m, 2H).

### Example 24: Synthesis of Compound 24

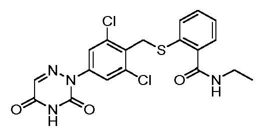

### Synthetic route:

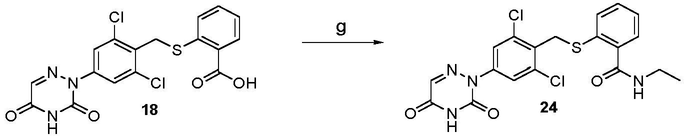

The synthetic method of Example **7** was followed to afford compound **24** (7.53 mg, yield 35%) as a white solid. MS(ESI, m/z): 451[M+H]⁺.

¹H NMR (400 MHz, DMSO-d6) δ 12.47 (s, 1H), 8.33 (t, *J =* 5.6 Hz, 1H), 7.70 - 7.69 (m, 3H), 7.50 (d, *J =* 7.6 Hz, 1H), 7.46 - 7.41 (m, 2H), 7.29 (t, *J =* 7.6 Hz, 1H), 4.37 (s, 2H), 3.32 - 3.17 (m, 2H), 1.08 (s, 3H).

### Example 25: Synthesis of Compound 25

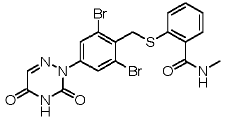

### Synthetic route:

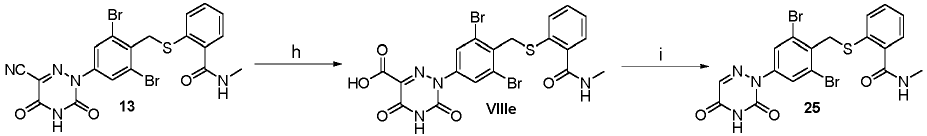

The raw material 1 was replaced with **13,** and then the synthetic method of Example **14** was followed to afford compound **25** (60 mg, yield 65%) as a white solid, MS(ESI, m/z): 527[M+H]⁺.

¹H NMR (400 MHz, DMSO-d6) δ 12.45 (s, 1H), 8.29 - 8.25 (m, 1H), 7.87 (s, 2H), 7.69 (s, 1H), 7.51 (d, *J =* 7.6 Hz, 1H), 7.46 - 7.43 (m, 2H), 7.28 (t, *J =* 7.6 Hz, 1H), 4.43 (s, 2H), 2.72 (d, *J* = 4.8 Hz, 3H).

### Example 26: Synthesis of Compound 26

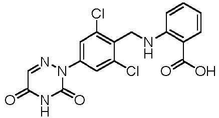

### Synthetic route:

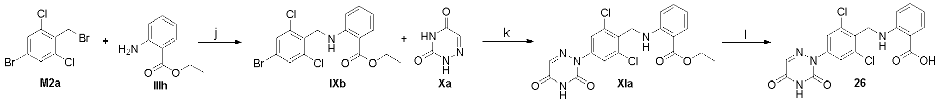

Steps j-k: The raw material **IIIb** was replaced with **IIIh,** and then the synthetic method of Example **17** was followed to afford compound **XIa** (200 mg, yield 61%) as a yellow solid, MS(ESI, m/z): 435[M+H]⁺.

Step l: Compound **XIa** (200 mg, 0.27 mmol) was dissolved into a mixed solution of ethanol (2 mL) and tetrahydrofuran (2 mL), and then a solution of lithium hydroxide (22 mg, 0.92 mmol) in water (2 mL) was added to the reaction solution, and heated to 65°C and reacted for six hours. Upon completion of the reaction, the pH value of the reaction solution system was adjusted to 2 to 3 with 1N diluted hydrochloric acid, and then ethyl acetate was added for extraction. The organic phases were washed with saturated saline. The organic phases were combined and concentrated to afford a crude product. Subsequently, the crude product was purified by column chromatography to afford compound **26** (10 mg, yield 9%) as a white solid, MS(ESI, m/z): 407[M+H]⁺.

¹H NMR (400 MHz, DMSO-d6) δ 11.69 (s, 1H), 8.16 - 8.07 (m, 1H), 7.80 (dd, *J =* 8.0 Hz, 1.6 Hz, 1H), 7.73 (s, 2H), 7.68 (s, 1H), 7.45 - 7.38 (m, 1H), 6.96 (d, *J =* 8.4 Hz, 1H), 6.65 - 6.59 (m, 1H), 4.62 (d, *J =* 4.4 Hz, 2H).

### Example 27: Synthesis of Compound 27

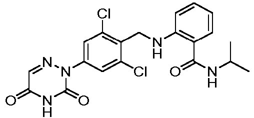

### Synthetic route:

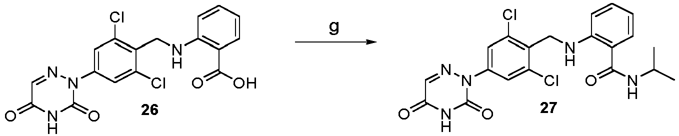

The synthetic method of Example **7** was followed to afford compound **27** (10 mg, yield 45.6%) as a white solid. MS(ESI, m/z): 448[M+H]⁺.

¹H NMR (400 MHz, DMSO-d6) δ 8.86 (s, 1H), 7.73 (s, 2H), 7.15 - 7.03 (m, 2H), 6.73 - 6.64 (m, 2H), 6.60 - 6.51 (m, 1H), 5.82 (t, *J =* 5.2 Hz, 1H), 4.39 (d, *J =* 5.2 Hz, 2H), 4.06 - 3.92 (m, 1H), 1.14 (d, *J =* 6.4 Hz, 6H).

### Example 28: Synthesis of Compound 28

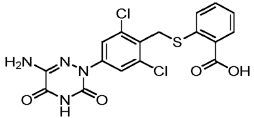

### Synthetic route:

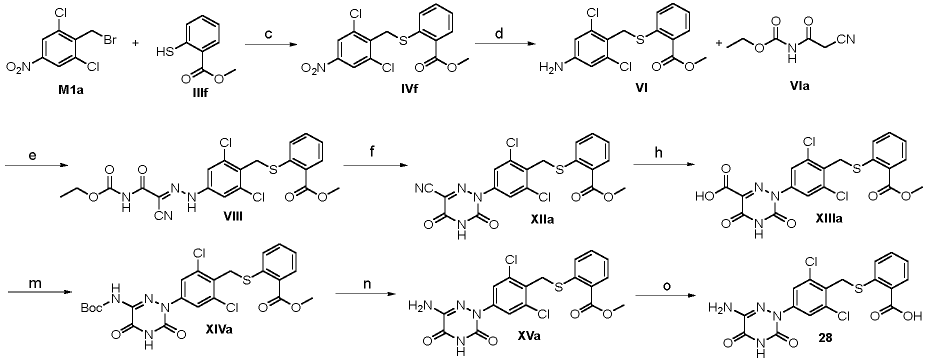

Steps c-f and step h: The raw material **IIIa** was replaced with **IIIf,** and then the synthetic methods of Example **1** and Example **14** were followed to afford compound **XIIIa** (47 mg, yield 22.6%) as a yellow solid, MS(ESI, m/z): 482[M+H]⁺.

Step m: Compound **XIIIa** (47 mg, 0.098 mmol), diphenylphosphoryl azide (83 mg, 0.30 mmol), and triethylamine (31 mg, 0.30 mmol) were added to a mixed solution of tert-butanol (10 mL) and tetrahydrofuran (3 mL), heated to 85°C, and reacted overnight. Upon completion of the reaction, the resultant was directly concentrated to afford a crude product. Thereafter, the crude product was purified by column chromatography to afford compound **XIVa** (60 mg, yield 99%) as a yellow oily product, MS(ESI, m/z): 553[M+H]⁺.

Step n: Compound **XIVa** (30 mg, 0.054 mmol) was added to dichloromethane (10 mL), and then trifluoroacetic acid (1 mL) was added and reacted at room temperature for three hours. Upon completion of the reaction, the resultant was directly concentrated to afford compound **XVa** (30 mg, yield 99%) as a crude product, MS(ESI, m/z): 453[M+H]⁺.

Step o: Compound **XVa** (30 mg, 0.054 mmol) was added to methanol (3 mL) and tetrahydrofuran (3 mL), and then an aqueous solution (0.5 mL) of sodium hydroxide (13 mg, 0.334 mmol) was added, heated to 45°C, and reacted overnight. Upon completion of the reaction, the resultant was directly concentrated to afford a crude product. Subsequently, the crude product was purified by column chromatography to afford compound **28** (4.2 mg, yield 14.5%) as a white solid, MS(ESI, m/z): 439[M+H]⁺.

¹H NMR (400 MHz, DMSO-d6) δ 7.91 (d, *J =* 7.2 Hz, 1H), 7.84 (s, 2H), 7.58 - 7.53 (m, 2H), 7.28 - 7.24 (m, 1H), 6.54 (s, 2H), 4.35 (s, 2H).

### Example 29: Synthesis of Compound 29

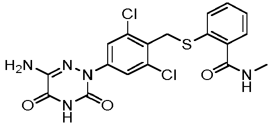

### Synthetic route:

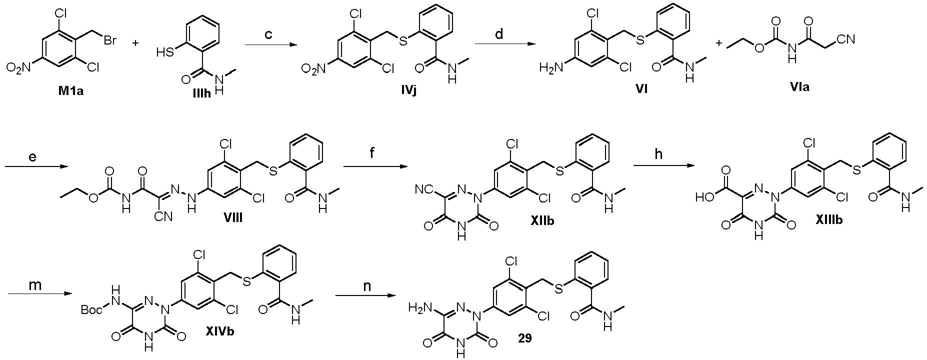

The raw material **IIIf** was replaced with **IIIh,** and then the synthetic method of Example **28** was followed to afford compound **29** (8.55 mg, yield 16%) as a white solid, MS(ESI, m/z): 452[M+H]⁺.

¹H NMR (400 MHz, DMSO-d6) δ 12.26 (s, 1H), 8.25 (s, 1H), 7.81 (s, 2H), 7.49 (d, *J =* 7.6 Hz, 1H), 7.44 - 7.41 (m, 2H), 7.28 (t, *J =* 8.0 Hz, 1H), 6.55 (s, 2H), 4.34 (s, 2H), 2.71 (d, *J* = 4.4 Hz, 3H).

### Example 30: Synthesis of Compound 30

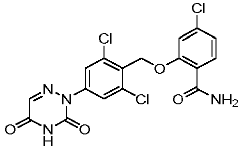

### Synthetic route:

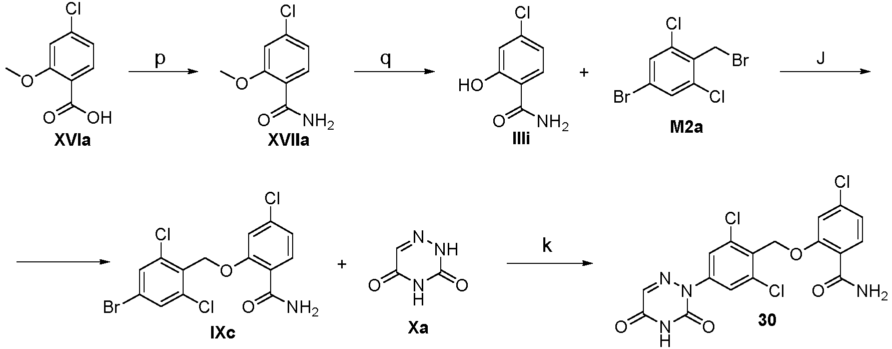

Step p: Compound **XVIa** (1 g, 5.36 mmol), 2-(7-azabenzotriazol-1-yl)-N,N,N',N'-tetramethyluronium hexafluorophosphate (HATU) (2.4 g, 6.43 mmol), and ammonium chloride (344 mg, 6.43 mmol) were added to N,N-dimethylformamide (30 mL), and then triethylamine (1.6 g, 16.07 mmol) was added to the reaction solution and stirred at room temperature for two hours. Upon completion of the reaction, the reaction solution was directly purified by column chromatography to afford compound **XVIIa** (991 mg, yield 98%) as a white solid, MS(ESI, m/z): 186[M+H]⁺.

Step q: Compound **XVIIa** (991 mg, 5.36 mmol) was added to dichloromethane (20 mL), and then boron tribromide (16 mL, 16 mmol) was slowly added under ice bath condition and stirred overnight at room temperature. Upon completion of the reaction, the reaction solution was quenched with water. The organic phases were separated and concentrated to afford a crude product. Subsequently, the crude product was purified by column chromatography to afford compound **IIIi** (800 mg, yield 99%) as a white solid, MS(ESI, m/z): 172[M+H]⁺.

Steps j-k: The raw material **IIIb** was replaced with **IIIi,** and then the synthetic method of Example **17** was followed to afford compound 30 (5 mg, yield 2.0%) as a white solid, MS(ESI, m/z): 441[M+H]⁺.

¹H NMR (400 MHz, DMSO-d6) δ 7.80 - 7.78 (m, 3H), 7.75 (d, *J =* 9.6 Hz, 1H), 7.67 (s, 1H), 7.54 (d, *J =* 1.6 Hz, 1H), 7.17 (dd, *J =* 6.4 Hz, 1.6Hz, 2H), 5.42 (s, 2H).

### Example 31: Synthesis of Compound 31

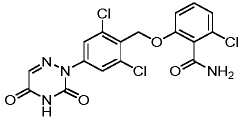

### Synthetic route:

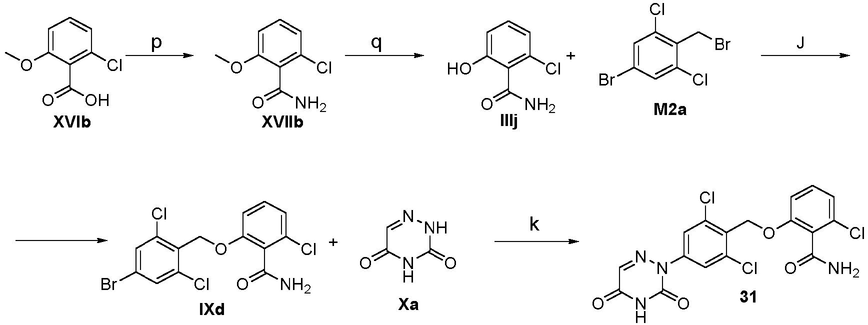

The raw material **XVIa** was replaced with **XVIb,** and then the synthetic method of Example **30** was followed to afford compound **31** (10 mg, yield 7.0%) as a white solid, MS(ESI, m/z): 441[M+H]⁺.

¹H NMR (400 MHz, DMSO-d6) δ 7.75 (s, 1H), 7.72 (s, 2H), 7.69 (s, 1H), 7.43 (s, 1H), 7.39 (t, *J=* 8.0 Hz, 1H), 7.28 (d, *J =* 8.0 Hz, 1H), 7.10 (d, *J=* 8.0 Hz, 1H), 5.26 (s, 2H).

### Example 32: Synthesis of Compound 32

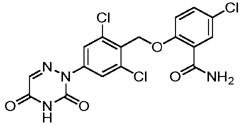

### Synthetic route:

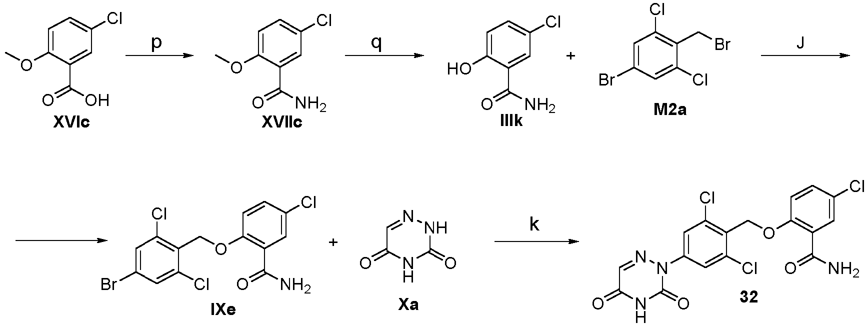

The raw material **XVIa** was replaced with **XVIc,** and then the synthetic method of Example **30** was followed to afford compound 32 (30.7 mg, yield 28.4%) as a white solid, MS(ESI, m/z): 441[M+H]⁺.

¹H NMR (400 MHz, DMSO-d6) δ 12.47 (s, 1H), 7.78 (s, 2H), 7.71 (s,1H), 7.70 (d, *J =* 2.8 Hz, 1H), 7.61 (s, 1H), 7.58 (dd, *J =* 8.8 Hz, 2.8 Hz, 1H), 7.43 (d, *J =* 9.2 Hz, 1H), 7.30 (s, 1H), 5.37 (s, 2H).

### Example 33: Synthesis of Compound 33

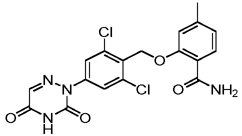

### Synthetic route:

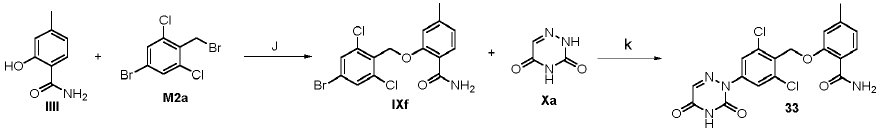

The raw material **IIIb** was replaced with **IIIl**, and then the synthetic method of Example **17** was followed to afford compound **33** (7 mg, yield 11.0%) as a white solid, MS(ESI, m/z): 421[M+H]⁺.

¹H NMR (400 MHz, DMSO-d6) δ 12.48 (s, 1H), 7.79 (s, 1H), 7.75 (d, *J* = 8.0 Hz,1H), 7.72 (s, 1H), 7.40 (s, 1H), 7.24 (s, 1H), 7.11 (s, 1H), 6.92 (d, *J =* 8.0 Hz, 2H), 5.39 (s, 2H), 2.38 (s, 3H).

### Example 34: Synthesis of Compound 34

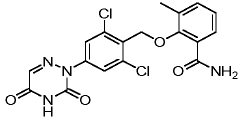

### Synthetic route:

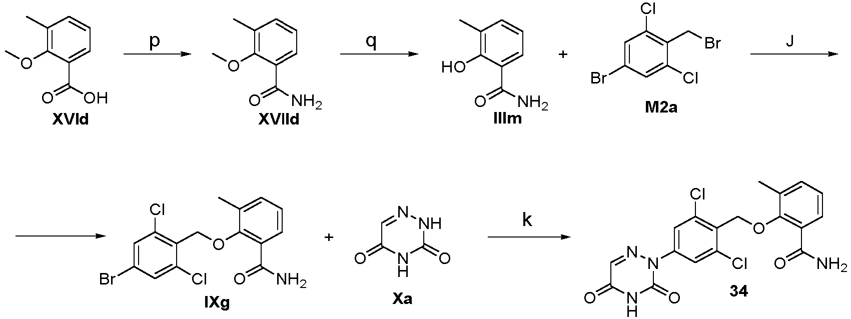

The raw material **XVIa** was replaced with **XVId,** and then the synthetic method of Example 30 was followed to afford compound **34** (5 mg, yield 4.0%) as a white solid, MS(ESI, m/z): 421[M+H]⁺.

¹H NMR (400 MHz, DMSO-d6) δ 7.71 (s, 2H), 7.69 (s, 1H), 7.53 (s, 1H), 7.34 (d, *J =* 8.8 Hz, 1H), 7.24 (d, *J =* 6.4 Hz, 1H), 7.06 (d, *J =* 8.0 Hz, 1H), 5.24 (s, 2H), 2.05 (s, 3H).

### Example 35: Synthesis of Compound 35

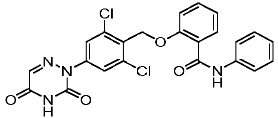

### Synthetic route:

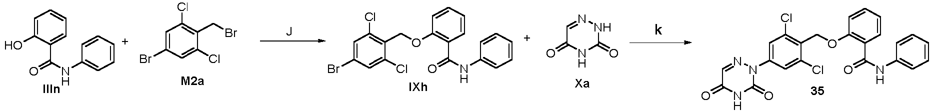

The raw material **IIIb** was replaced with **IIIn,** and then the synthetic method of Example **17** was followed to afford compound **35** (59.7 mg, yield 16.8%) as a white solid, MS(ESI, m/z): 483[M+H]⁺.

¹H NMR (400 MHz, DMSO-d6) δ 9.94 (s, 1H), 7.79 - 7.76 (m, 3H), 7.71 (s, 1H), 7.59 (t, *J* = 8.4 Hz, 1H), 7.46 (d, *J =* 8.4 Hz, 1H), 7.39 (d, *J =* 8.0 Hz, 2H), 7.26 (t, *J =* 8.0 Hz, 2H), 7.18 (t, *J =* 7.6 Hz, 1H), 7.03 (t, *J =* 7.2 Hz, 1H), 5.46 (s, 2H).

### Example 36: Synthesis of Compound 36

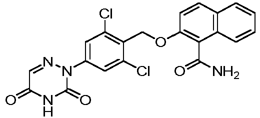

### Synthetic route:

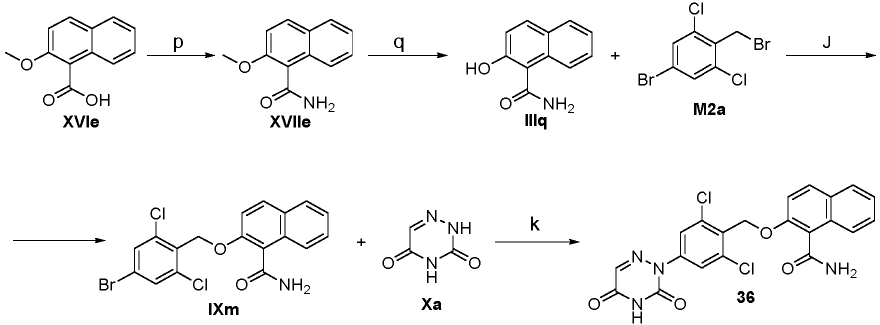

The raw material **XVIa** was replaced with **XVIe,** and then the synthetic method of Example **30** was followed to afford compound **36** (12 mg, yield 5.0%) as a white solid, MS(ESI, m/z): 457[M+H]⁺.

¹H NMR (400 MHz, DMSO-d6) δ 8.33 (s, 1H), 7.98 (d, *J =* 8.4 Hz, 1H), 7.91 (d, *J =* 8.0 Hz, 1H), 7.82 (s, 2H), 7.77 (s, 1H), 7.68 (s, 1H), 7.61 - 7.55 (m, 2H), 7.44 (t, *J =* 8.0 Hz, 1H), 7.39 (s, 1H), 5.48 (s, 2H).

### Example 37: Synthesis of Compound 37

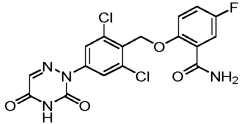

### Synthetic route:

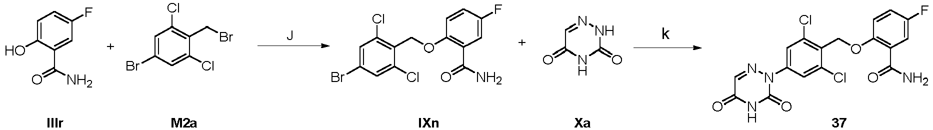

The raw material **IIIb** was replaced with **IIIr**, and then the synthetic method of Example 17 was followed to afford compound 37 (11 mg, yield 10.0%) as a white solid, MS(ESI, m/z): 425[M+H]⁺.

¹H NMR (400 MHz, DMSO-d6) δ 12.50 (s, 1H), 7.79 (s, 2H), 7.70 (s, 1H), 7.64 (s, 1H), 7.52 (dd, *J* = 9.2 Hz, 2.8 Hz, 1H), 7.45 - 7.39 (m, 2H), 7.33 (s, 1H), 5.38 (s, 2H).

### Example 38: Synthesis of Compound 38

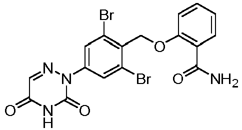

### Synthetic route:

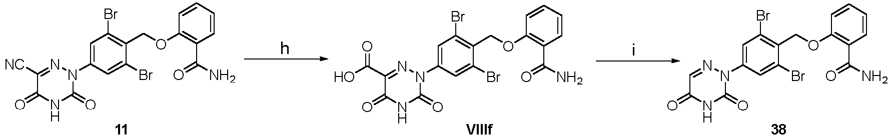

The raw material **1** was replaced with **11,** and then the synthetic method of Example **14** was followed to afford compound **38** (3.5 mg, yield 3.2%) as a white solid, MS(ESI, m/z): 497[M+H]⁺.

¹H NMR (400 MHz, DMSO-d6) δ 12.49 (s, 1H), 7.97 (s, 2H), 7.84 (dd, *J =* 7.2 Hz, 1.6 Hz, 1H), 7.71 (s, 1H), 7.55 (t, *J =* 8.0 Hz, 1H), 7.50 (s, 1H), 7.40 (d, *J =* 8.0 Hz, 1H), 7.17 (s, 1H), 7.12 (t, *J =* 7.6 Hz, 1H), 5.42 (s, 2H).

### Example 39: Synthesis of Compound 39

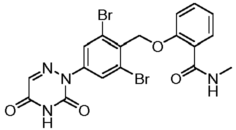

### Synthetic route:

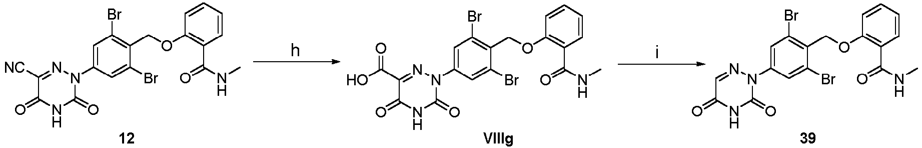

The raw material 1 was replaced with **12,** and then the synthetic method of Example **14** was followed to afford compound **39** (10.3 mg, yield 6.2%) as a white solid, MS(ESI, m/z): 511[M+H]⁺.

¹H NMR (400 MHz, DMSO-d6) δ 12.49 (s, 1H), 7.97 (s, 2H), 7.81-7.78 (m, 1H), 7.72 (s, 1H), 7.69 (d, *J* = 7.6 Hz, 1H), 7.51 (t, *J =* 7.6 Hz, 1H), 7.40 (d, *J =* 8.4 Hz, 1H), 7.11 (t, *J =* 7.2 Hz, 1H), 5.40 (s, 2H), 2.69 (d, *J =* 4.8 Hz, 3H).

### Example 40: Synthesis of Compound 40

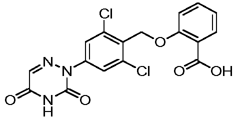

### Synthetic route:

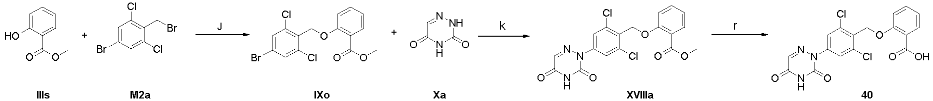

Steps j-k: The raw material **IIIb** was replaced with **IIIs**, and then the synthetic method of Example **17** was followed to afford compound **XVIIIa** (180 mg, yield 26.0%) as a white solid, MS(ESI, m/z): 422[M+H]⁺.

Step r: Compound **XVIIIa** (180 mg, 0.450 mmol) was added to methanol (10 mL), and then an aqueous solution (5 mL) of sodium hydroxide (126 mg, 3.15 mmol) was slowly added to the reaction solution and stirred overnight at room temperature. Upon completion of the reaction, 1N hydrochloric acid solution was added to adjust the pH value of the system to between 4 and 5. The resultant was filtered and dried to afford compound **40** (170 mg, yield 93.0%) as a white solid, MS(ESI, m/z): 408[M+H]⁺.

¹H NMR (400 MHz, DMSO-d6) δ 12.46 (s, 1H), 7.75 (s, 2H), 7.73 (s, 1H), 7.63 (d, *J* = 7.6 Hz, 1H), 7.54 (t, *J =* 7.2 Hz, 1H), 7.33 (d, *J =* 8.4 Hz, 1H), 7.07 (t, *J =* 7.6 Hz, 1H), 5.30 (s, 2H).

### Example 41: Synthesis of Compound 41

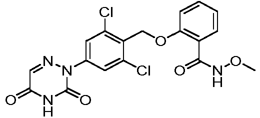

### Synthetic route:

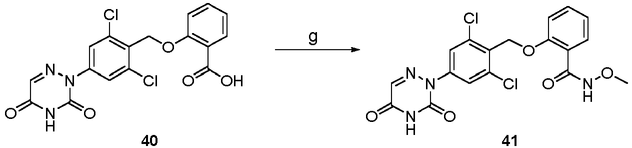

The synthetic method of Example 7 was followed to afford compound **41** (5 mg, yield 39%) as a white solid. MS(ESI, m/z): 437[M+H]⁺.

¹H NMR (400 MHz, DMSO-d6) δ 12.51 (s, 1H), 11.01 (s, 1H), 7.76 (s, 2H), 7.72 (s, 1H), 7.50 (t, *J =* 8.0 Hz, 1H), 7.44 (d, *J =* 6.8 Hz, 1H), 7.34 (d, *J =* 8.4 Hz, 1H), 7.07 (t, *J =* 7.2 Hz, 1H), 5.32 (s, 2H), 3.55 (s, 2H).

### Example 42: Synthesis of Compound 42

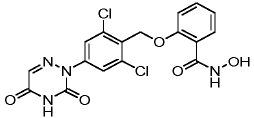

### Synthetic route:

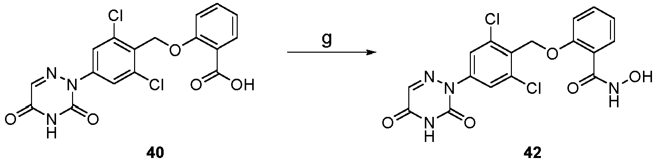

The synthetic method of Example **7** was followed to afford compound **42** (8 mg, yield 39%) as a white solid. MS(ESI, m/z): 423[M+H]⁺.

¹H NMR (400 MHz, DMSO-d6) δ 10.39 (s, 1H), 9.04 (s, 1H), 7.77 (s, 2H), 7.71 (s, 1H), 7.60 - 7.41 (m, 2H), 7.33 (d, *J =* 8.0 Hz, 1H), 7.07 (t, *J =* 7.6 Hz, 1H), 5.32 (s, 2H).

### Example 43: Synthesis of Compound 43

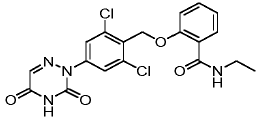

### Synthetic route:

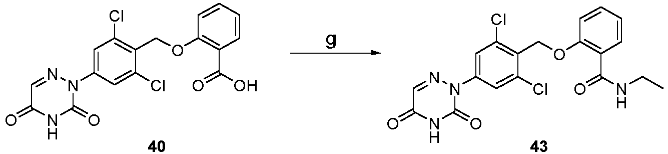

The synthetic method of Example **7** was followed to afford compound **43** (15 mg, yield 61%) as a white solid. MS(ESI, m/z): 435[M+H]⁺.

¹H NMR (400 MHz, DMSO-d6) δ 12.49 (s, 1H), 7.79 (s, 2H), 7.76 - 7.62 (m, 3H), 7.59 - 7.45 (m, 1H), 7.38 (d, *J =* 8.4 Hz, 1H), 7.11 (t, *J =* 7.6 Hz, 1H), 5.40 (s, 2H), 3.23 - 3.03 (m, 2H), 0.88 (t, *J =* 7.2 Hz, 3H).

### Example 44: Synthesis of Compound 44

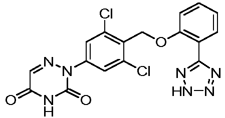

### Synthetic route:

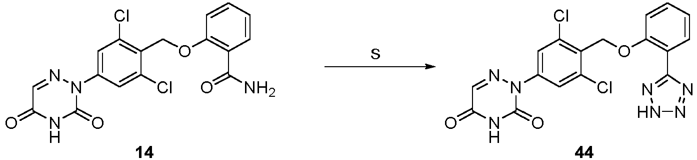

Step s: Compound **14** (44 mg, 0.108 mmol) and diphenylphosphoryl azide (200 mg, 0.727 mmol) were added to pyridine (2 mL) and stirred at 130°C for six hours. Upon completion of the reaction, the reaction solution was directly purified by column chromatography to afford compound **44** (10 mg, yield 21.5%) as a white solid, MS(ESI, m/z): 432[M+H]⁺.

¹H NMR (400 MHz, DMSO-d6) δ 7.75 - 7.68 (m, 2H), 7.68 - 7.57 (m, 1H), 7.22 (t, *J =* 7.6 Hz, 2H), 7.12 (d, *J =* 8.0 Hz, 1H), 6.96 (t, *J =* 7.2 Hz, 1H), 5.48 - 5.36 (m, 2H).

### Example 45: Synthesis of Compound 45

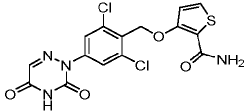

### Synthetic route:

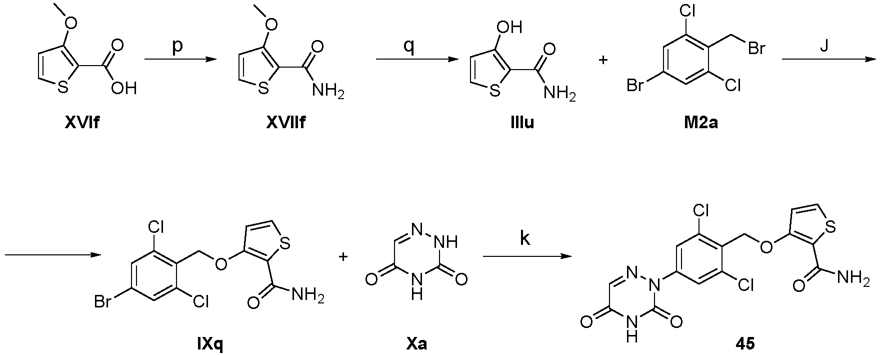

The raw material **XVIa** was replaced with **XVIf,** and then the synthetic method of Example 30 was followed to afford compound **45** (15 mg, yield 9.0%) as a white solid, MS(ESI, m/z): 413[M+H]⁺.

¹H NMR (400 MHz, DMSO-d6) δ 12.48 (s, 1H), 7.80 - 7.78 (m, 3H), 7.71 (s, 1H), 7.49 (s, 1H), 7.36 (d, *J =* 5.6 Hz, 1H), 6.64 (s, 1H), 5.47 (s, 2H).

### Example 46: Synthesis of Compound 46

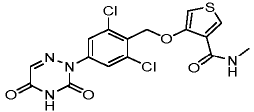

### Synthetic route:

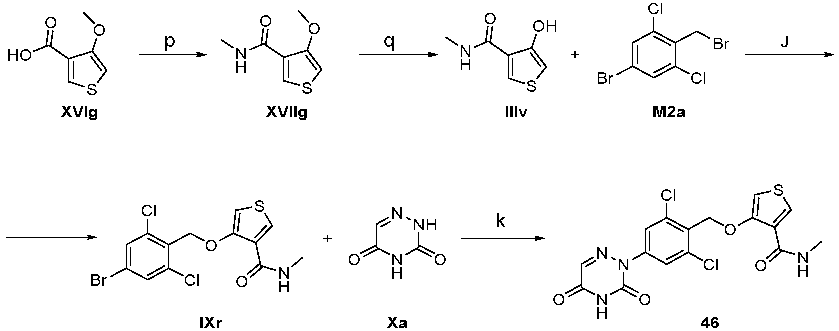

The raw material **XVIa** was replaced with **XVIg,** and then the synthetic method of Example **30** was followed to afford compound **46** (29.4 mg, yield 6.3%) as a white solid, MS(ESI, m/z): 427[M+H]⁺.

¹H NMR (400 MHz, DMSO-d6) δ 8.00 (d, *J =* 3.6 Hz, 1H), 7.79 (s, 2H), 7.71 (s, 1H), 7.46 - 7.38 (m, 1H), 7.07 (d, *J =* 3.6 Hz, 1H), 5.34 (s, 2H), 2.70 (d, *J =* 4.8 Hz, 3H).

### Example 47: Synthesis of Compound 47

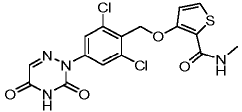

### Synthetic route:

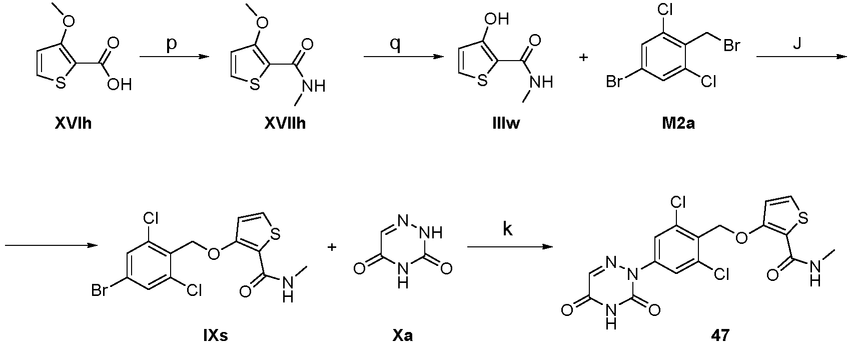

The raw material XVIa was replaced with XVIh, and then the synthetic method of Example 30 was followed to afford compound 47 (40 mg, yield 65.4%) as a white solid, MS(ESI, m/z): 427[M+H]⁺.

¹H NMR (400 MHz, DMSO-d6) δ 12.47 (s, 1H), 7.77 (s, 2H), 7.73 (d, *J =* 5.6 Hz, 1H), 7.70 (s, 1H), 7.32 (d, *J =* 5.6 Hz, 1H), 7.19 - 7.14 (m, 1H), 5.46 (s, 2H), 2.71 (d, *J =* 4.8 Hz, 3H).

### Example 48: Synthesis of Compound 48

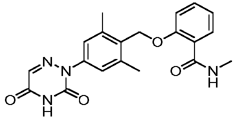

### Synthetic route:

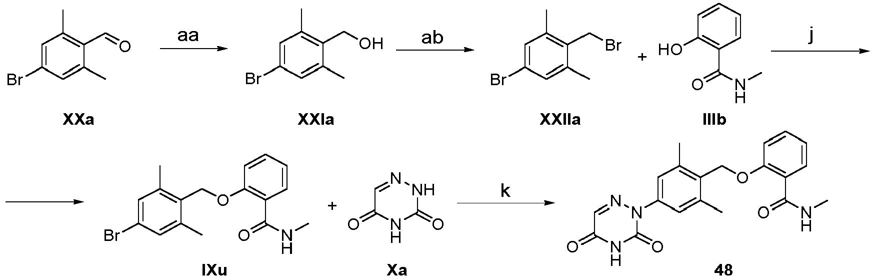

Step aa: Compound **XXa** (2 g, 9.39 mmol) was added to methanol (20 mL), and then sodium borohydride (392 mg, 10.3 mmol) was slowly added to the reaction solution at 0°C, and stirred at room temperature for 30 min. Upon completion of the reaction, a saturated ammonium chloride solution was added to quench the reaction system, and then ethyl acetate was added. The organic phases were washed with saturated saline. The organic phases were combined and concentrated to afford compound **XXIa** (1.6 g, yield 80%) as a yellow solid.

Step ab: Compound **XXIa** (1.6 g, 7.44 mmol) was added to dichloromethane (10 mL) and tetrahydrofuran (10 mL), and then phosphorus tribromide (3 g, 11.2 mmol) was slowly added to the reaction solution at 0°C and stirred at room temperature for 30 min. Upon completion of the reaction, the resultant was directly concentrated, and then purified by column chromatography to afford compound **XXIIa** (1.8 g, yield 86.5%) as a white solid.

Steps j-k: The synthetic method of Example 17 was followed to afford compound **48** (2.76 mg, yield 2.5%) as a white solid, MS(ESI, m/z): 381[M+H]⁺.

¹H NMR (400 MHz, DMSO-d6) δ 12.31 (s, 1H), 7.91 - 7.87 (m, 1H), 7.62 (s, 1H), 7.57 (d, *J =* 7.6 Hz, 1H), 7.47 (t, *J =* 8.0 Hz, 1H), 7.34 (d, *J =* 8.4 Hz, 1H), 7.22 (s, 2H), 7.05 (t, *J* = 7.6 Hz, 1H), 5.16 (s, 2H), 2.63 (d, *J =* 4.4 Hz, 3H), 2.38 (s, 6H).

### Example 49: Synthesis of Compound 49

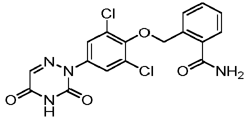

### Synthetic route:

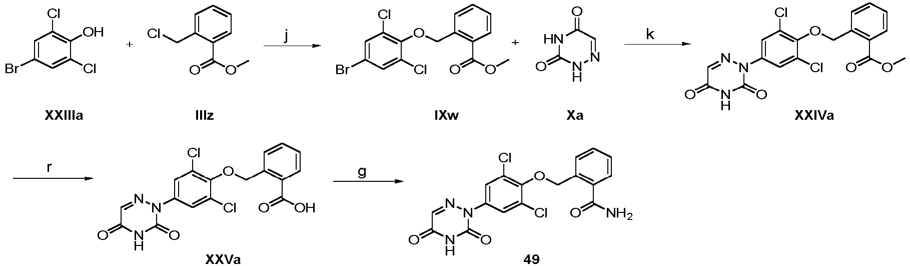

The raw material **IIIb** was replaced with **IIIz,** and then the synthetic method of Example **17** was followed to afford a yellow oily product **XXIVa** (300 mg, yield 81.5%), MS(ESI, m/z): 422[M+H]⁺.

Step r: Compound **XXIVa** (300 mg, 0.71 mmol) was added to methanol (10 mL), and then an aqueous solution (2 mL) of sodium hydroxide (28 mg, 2.13 mmol) was added, heated to 45°C, and reacted overnight. Upon completion of the reaction, the resultant was directly concentrated to afford a crude product. Thereafter, the crude product was purified by column chromatography to afford compound **XXVa** (45 mg, yield 15.5%) as a yellow solid, MS(ESI, m/z): 408[M+H]⁺.

Step g: Compound **XXVa** (45 mg, 0.11 mmol), ammonium chloride (30 mg, 0.55 mmol), and HATU (63 mg, 0.17 mmol) were dissolved into N,N-dimethylformamide (2 mL), and then N,N-diisopropylethylamine (43 mg, 0.33 mmol) was added to the reaction solution and stirred at room temperature for one hour. Upon completion of the reaction, 1N diluted hydrochloric acid solution was added to adjust the pH value to 4 to 5, and ethyl acetate (100 mL) was added. The organic phases were washed with saturated saline. The organic phases were combined and concentrated to afford a crude product. Subsequently, the crude product was purified by column chromatography to afford compound **49** (10.4 mg, yield 23.1%) as a white solid, MS(ESI, m/z): 407[M+H]⁺.

¹H NMR (400 MHz, DMSO-d6) δ 12.46 (s, 1H), 7.92 (s, 1H), 7.83 (d, *J =* 7.6 Hz, 1H), 7.72 (s, 2H), 7.69 (s, 1H), 7.58 - 7.54 (m, 2H), 7.49 (s, 1H), 7.43 (t, *J =* 7.6 Hz, 1H), 5.30 (s, 2H).

### Example 50: Synthesis of Compound 50

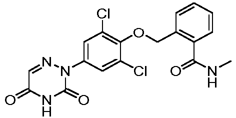

### Synthetic route:

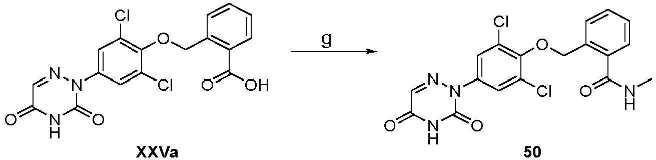

The synthetic method of Example 7 was followed to afford compound 50 (6 mg, yield 58%) as a white solid. MS(ESI, m/z): 421[M+H]⁺.

¹H NMR (400 MHz, DMSO-d6) δ 8.39 - 8.34 (m, 1H), 7.79 (d, *J =* 7.6 Hz, 1H), 7.71 (s, 2H), 7.68 (s, 1H), 7.55 (t, *J =* 7.6 Hz, 1H), 7.49 (d, *J =* 6.4 Hz, 1H), 7.44 (t, *J =* 7.6 Hz, 1H), 5.26 (s, 2H), 2.73 (d, *J =* 4.8 Hz, 3H).

### Example 51: Synthesis of Compound 51

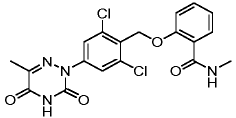

### Synthetic route:

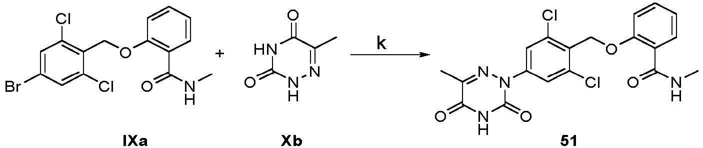

The raw material **Xa** was replaced with **Xb,** and the synthetic method of Example **17** was followed to afford compound **51** (24 mg, yield 18.0%) as a yellow solid, MS(ESI, m/z): 435[M+H]⁺.

¹H NMR (400 MHz, DMSO-d6) δ 7.87 - 7.79 (m, 3H), 7.67 (d, *J =* 6.4 Hz, 1H), 7.48 (t, *J* = 7.6 Hz, 1H), 7.37 (d, *J =* 8.4 Hz, 1H), 7.08 (t, *J =* 7.6 Hz, 1H), 5.34 (s, 2H), 2.68 (d, *J =* 4.8 Hz, 3H), 2.08 (s, 3H).

### Example 52: Synthesis of Compound 52

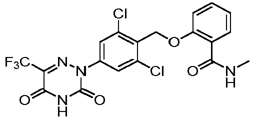

### Synthetic route:

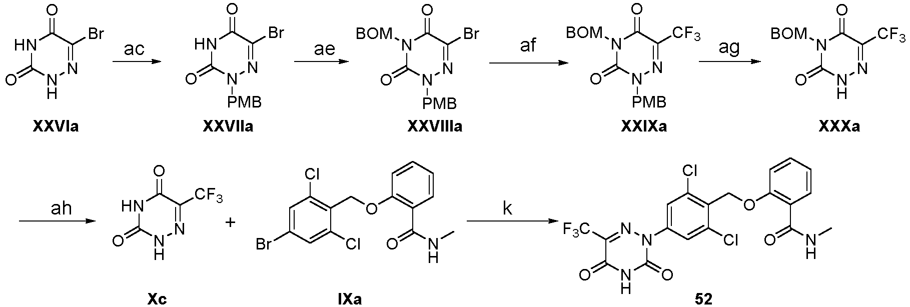

Step ac: Compound **XXVIa** (3 g, 15.6 mmol) and N,O-bis(trimethylsilyl)acetamide (6.3 g, 31.3 mmol) were added to acetonitrile (20 mL), heated to 85°C, and stirred for two hours. Thereafter, sodium iodide (2.3 g, 15.6 mmol) and 4-methoxybenzylchloride (2.9 g, 18.8 mmol) were slowly added to the reaction solution and stirred overnight at 85°C. Upon completion of the reaction, ethyl acetate (100 mL) was added. The organic phases were washed with saturated saline. The organic phases were combined and concentrated to afford a crude product. Afterwards, the crude product was purified by column chromatography to obtain a yellow solid **XXVIIa** (4.5 g, yield 92.6%), MS(ESI, m/z): 312[M+H]+.

Step ae: Compound **XXVIIa** (1.7 g, 5.47 mmol) was added to N,N-dimethylformamide (20 mL), and cooled to 0°C. Thereafter, sodium hydride (328 mg, 8.2 mmol) was added to the reaction solution and stirred at 0°C for half an hour. Thereafter, benzyl chloromethyl ether (1 g, 6.56 mmol) was slowly added to the reaction solution, and stirred at room temperature for two hours. Upon completion of the reaction, ethyl acetate (100 mL) was added. The organic phases were washed with saturated saline. The organic phases were combined and concentrated to afford a crude product. Afterwards, the crude product was purified by column chromatography to afford a white solid **XXVIIIa** (1.8 g, yield 76.3%).

1H NMR (400 MHz, DMSO-d6) δ 7.32 -7.25 (m, 7H), 6.91 (d, J = 8.4 Hz, 2H), 5.32 (s, 2H), 4.98 (s, 2H), 4.59 (s, 2H), 3.73 (s, 3H).

Step af: Compound **XXVIIIa** (1.335 g, 3.09 mmol) and methyl fluorosulfonyldifluoroacetate (2.37 g, 12.4 mmol) were added to N,N-dimethylformamide (10 mL), and then cuprous iodide (1.17 g, 6.18 mmol) was slowly added to the reaction solution and stirred overnight at 120°C. Upon completion of the reaction, ethyl acetate (100 mL) was added. The organic phases were washed with saturated saline. The organic phases were combined and concentrated to afford a crude product. Thereafter, the crude product was purified by column chromatography to afford a colorless oily product **XXIXa** (1.1 g, yield 84.6%), MS(ESI, m/z): 422[M+H]⁺.

Step ag: Compound **XXIXa** (1.1 g, 2.61 mmol) was added to acetonitrile (24 mL), and cooled to 0°C. Thereafter, an aqueous solution (8 mL) of ceric ammonium nitrate (4.3 g, 7.84 mmol) was slowly added to the reaction solution, and stirred overnight at room temperature. Upon completion of the reaction, ethyl acetate (100 mL) was added. The organic phases were washed with saturated saline. The organic phases were combined and concentrated to afford a crude product. Thereafter, the crude product was purified by column chromatography to afford a white solid **XXXa** (821 mg, yield 99%), MS(ESI, m/z): 302[M+H]⁺.

Step ah: Compound **XXXa** (821 mg, 2.73 mmol) was added to dichloromethane (20 mL), and cooled to 0°C. Thereafter, 1 mol/L boron tribromide solution (4 mL, 4.09 mmol) was slowly added to the reaction solution, and stirred at 0°C for one hour. Upon completion of the reaction, ethyl acetate (100 mL) was added. The organic phases were washed with saturated saline. The organic phases were combined and concentrated to afford a crude product. Thereafter, the crude product was purified by column chromatography to afford a colorless oily product **Xc** (400 mg, yield 81.0%), MS(ESI, m/z): 182[M+H]⁺.

Step k: The synthetic method of Example **17** was followed to afford compound **52** (100 mg, yield 53.0%) as a white solid, MS(ESI, m/z): 489[M+H]⁺.

¹H NMR (400 MHz, DMSO-d6) δ 13.05 (s, 1H), 7.87 - 7.81 (m, 1H), 7.73 (s, 2H), 7.66 - 7.61 (m, 1H), 7.47 (t, *J =* 8.8 Hz, 1H), 7.36 (d, *J =* 8.4 Hz, 1H), 7.09 (t, *J =* 7.6 Hz, 1H), 5.37 (s, 2H), 2.67 (d, *J =* 4.8 Hz, 3H).

### Example 53: Synthesis of Compound 53

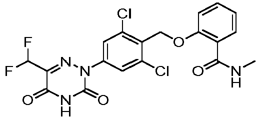

### Synthetic route:

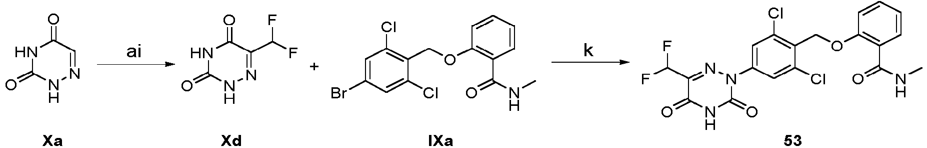

Step ai: Compound **Xa** (2 g, 17.7 mmol) and sodium difluoromethanesulfinate (4.9 g, 35.4 mmol) were added to dimethylsulfoxide (80 mL), and then Acid Red 94 (360 mg, 0.35 mmol) was added to the reaction solution and stirred at room temperature for ten hours under green light. Upon completion of the reaction, ethyl acetate (500 mL) was added. The organic phases were washed with saturated saline. The organic phases were combined and concentrated to afford a crude product. Thereafter, the crude product was purified by column chromatography to afford a red oily product **Xd** (680 mg, yield 23.6%), MS(ESI, m/z): 164[M+H]⁺.

Step k: The synthetic method of Example **17** was followed to afford compound **53** (18 mg, yield 18.0%) as a white solid, MS(ESI, m/z): 471[M+H]⁺.

¹H NMR (400 MHz, DMSO-d6) δ 12.83 (s, 1H), 7.85 - 7.83(m, 1H), 7.78 (s, 2H), 7.65 (d, *J =* 7.6 Hz, 1H), 7.50 (t, *J =* 7.6 Hz, 1H), 7.37 (d, *J =* 8.4 Hz, 1H), 7.01 (t, *J =* 52.4 Hz, 1H), 5.38 (s, 2H), 2.69 (d, *J =* 4.8 Hz, 3H).

### Example 54: Synthesis of Compound 54

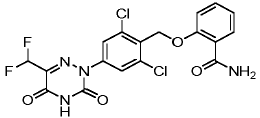

### Synthetic route:

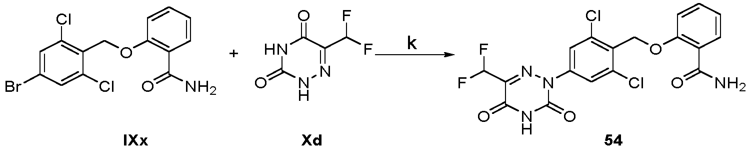

The raw material **Xa** was replaced with **Xd,** and the synthetic method of Example **17** was followed to afford compound **54** (30 mg, yield 17.0%) as a yellow solid, MS(ESI, m/z): 457[M+H]⁺.

¹H NMR (400 MHz, DMSO-d6) δ 12.86 (s, 1H), 7.81 - 7.79 (m, 3H), 7.54 (dd, J = 11.2 Hz, 4.4 Hz, 1H), 7.47 (s, 1H), 7.40 (d, *J =* 8.4 Hz, 1H), 7.22 (s,1H), 7.11 (t, *J =* 7.6 Hz, 1H), 6.92 (t, *J =* 52.4 Hz, 1H), 5.40 (s, 2H).

### Example 55: Synthesis of Compound 55

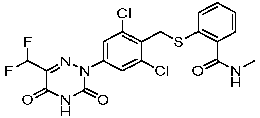

### Synthetic route:

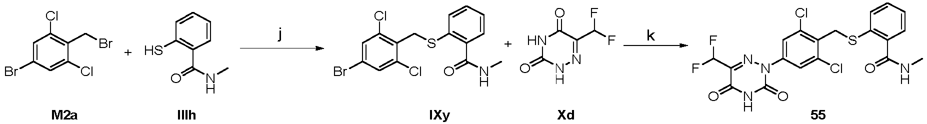

The synthetic method of Example 17 was followed to afford compound 55 (5.2 mg, yield 4.0%) as a yellow solid, MS(ESI, m/z): 487[M+H]⁺.

¹H NMR (400 MHz, DMSO-d6) δ 8.29 - 8.23 (m, 1H), 7.70 (s, 2H), 7.51 (d, *J =* 7.6 Hz, 1H), 7.47 - 7.41 (m, 2H), 7.30 (t, *J* = 7.6 Hz,1H), 6.90 (t, *J* = 52.4 Hz, 1H), 4.39 (s, 2H), 2.72 (d, *J =* 4.8 Hz, 3H).

### Example 56: Synthesis of Compound 56

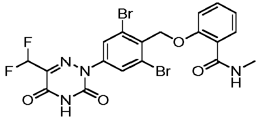

### Synthetic route:

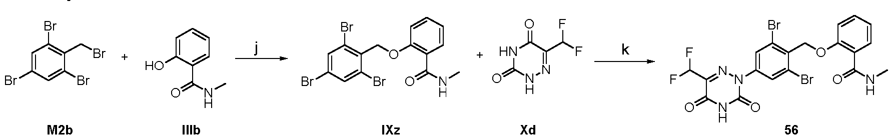

The synthetic method of Example 17 was followed to afford compound **56** (5 mg, yield 7.8%) as a yellow solid, MS(ESI, m/z): 561[M+H]⁺.

¹H NMR (400 MHz, DMSO-d6) δ 8.18 - 8.13 (m, 1H), 7.97 (s, 2H), 7.82 - 7.77 (m, 1H), 7.68 (d, *J =* 7.6 Hz, 1H), 7.55 - 7.47 (m, 1H), 7.39 (d, *J =* 8.4 Hz, 1H), 7.11 (t, *J =* 7.6 Hz, 1H), 6.97 (t, *J =* 52.4 Hz, 1H), 5.41 (s, 2H), 2.69 (d, *J =* 4.8 Hz, 3H).

### Example 57: Synthesis of Compound 57

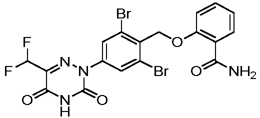

### Synthetic route:

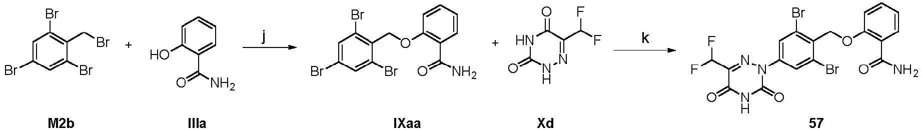

The synthetic method of Example **17** was followed to afford compound **57** (12.4 mg, yield 8.3%) as a yellow solid, MS(ESI, m/z): 547[M+H]⁺.

¹H NMR (400 MHz, DMSO-d6) δ 7.98 (s, 2H), 7.85 (d, *J =* 7.2 Hz, 1H), 7.55 (t, *J =* 7.2 Hz, 1H), 7.48 (s, 1H), 7.41 (d, *J =* 8.8 Hz, 1H), 7.17 (s, 1H), 7.12 (t, *J =* 7.6 Hz, 1H), 6.93 (t, *J* = 52.4 Hz, 1H), 5.43 (s, 2H).

### Example 58: Synthesis of Compound 58

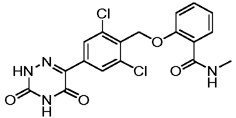

### Synthetic route:

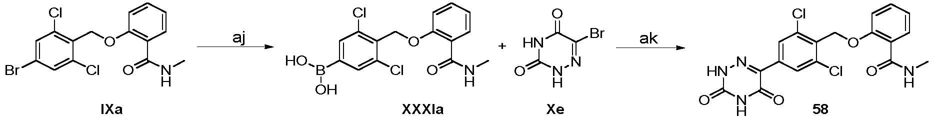

Step aj: Compound **IXa** (1.34 g, 3.44 mmol), bis(pinacolato)diboron (2.62 g, 10.3 mmol), potassium acetate (1.69 g, 17.2 mmol), and [1,1'-bis(diphenylphosphino)ferrocene] dichloropalladium (252 mg, 0.34 mmol) were added to 1,4-dioxane (20 mL), heated to 90°C, and reacted for nine hours. Upon completion of the reaction, the resultant was directly concentrated to afford a crude product. Thereafter, the crude product was purified by column chromatography to afford compound **XXXIa** (760 mg, yield 60.8%) as a gray solid, MS(ESI, m/z): 354[M+H]⁺.

Step ak: Compound **XXXIa** (70 mg, 0.198 mmol), compound **Xe** (46 mg, 0.237 mmol), tetrakis(triphenylphosphine)palladium (23 mg, 0.0198 mmol), and sodium carbonate (42 mg, 0.395 mmol) were added to a mixed solution of 1,4-dioxane (4 mL) and water (0.5 mL), and then heated to 90°C, and stirred overnight. Upon completion of the reaction, ethyl acetate (100 mL) was added. The organic phases were washed with saturated saline. The organic phases were combined and concentrated to afford a crude product. Subsequently, the crude product was purified by column chromatography to afford compound **58** (3.4 mg, yield 4.0%) as a white solid, MS(ESI, m/z): 421[M+H]⁺.

¹H NMR (400 MHz, DMSO-d6) δ 12.72 (s, 1H), 12.23 (s, 1H), 8.02 (s, 2H), 7.84 - 7.78 (m, 1H), 7.65 (d, *J =* 7.6 Hz, 1H), 7.47 (t, *J =* 8.4 Hz, 1H), 7.36 (d, *J =* 8.4 Hz, 1H), 7.08 (t, *J* = 7.6 Hz, 1H), 5.37 (s, 2H), 2.66 (d, *J =* 4.8 Hz, 3H).

### Example 59: Synthesis of Compound 59

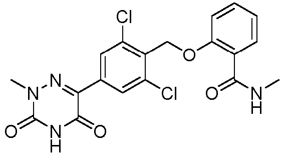

### Synthetic route:

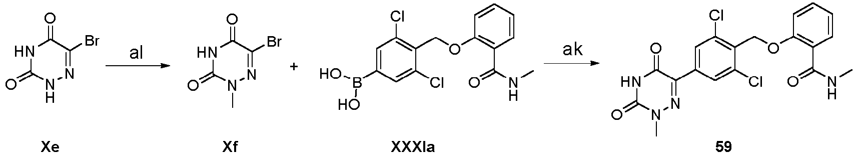

Step al: Compound **Xe** (1 g, 5.21 mmol) was added to acetonitrile (15 mL), and then N,O-bis(trimethylsilyl)acetamide (2.64 g, 13.0 mmol) was added, heated to 85°C, and stirred for two hours. Thereafter, iodomethane (1.1 g, 7.81 mmol) was slowly added dropwise to the reaction solution, and stirred overnight at 85°C. Upon completion of the reaction, ethyl acetate (100 mL) was added. The organic phases were washed with saturated saline. The organic phases were combined and concentrated to afford a crude product. Afterwards, the crude product was purified by column chromatography to afford a yellow solid **Xf** (530 mg, yield 49.5%), MS(ESI, m/z): 206[M+H]⁺.

Step ak: The raw material **Xe** was replaced with **Xf,** and then the synthetic method of Example **58** was followed to afford compound **59** (4.85 mg, yield 5.0%) as a white solid, MS(ESI, m/z): 435[M+H]⁺.

¹H NMR (400 MHz, DMSO-d6) δ 8.10 (s, 2H), 7.82 (d, *J =* 4.0 Hz, 1H), 7.65 (d, *J =* 7.6 Hz, 1H), 7.48 (t, *J =* 7.6 Hz, 1H), 7.36 (d, *J =* 8.4 Hz, 1H), 7.08 (t, *J =* 7.2 Hz, 1H), 5.36 (s, 2H), 3.55 (s, 3H), 2.66 (d, *J =* 4.8 Hz, 3H).

### Example 60: Synthesis of Compound 60

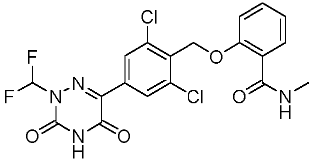

### Synthetic route:

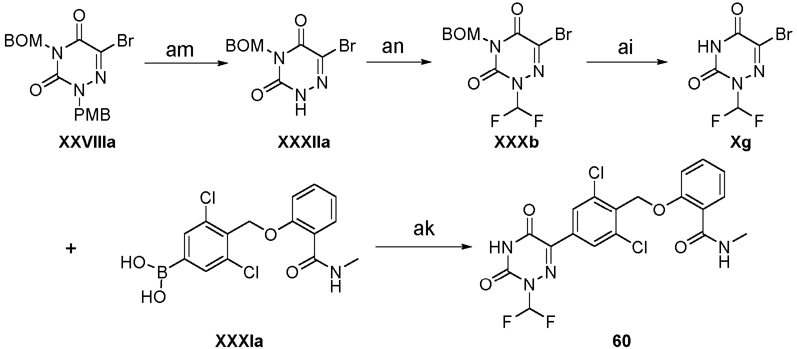

Step am: Compound **XXVIIIa** (1.8 g, 4.18 mmol) was added to acetonitrile (24 mL), and cooled to 0°C. Thereafter, an aqueous solution (8 mL) of ceric ammonium nitrate (6.6 g, 12.5 mmol) was slowly added to the reaction solution and stirred overnight at room temperature. Upon completion of the reaction, ethyl acetate (100 mL) was added. The organic phases were washed with saturated saline. The organic phases were combined and concentrated to afford a crude product. Afterwards, the crude product was purified by column chromatography to afford a white solid **XXXIIa** (830 mg, yield 63.8%), MS(ESI, m/z): 312[M+H]⁺.

Step an: Compound **XXXIIa** (830 mg, 2.66 mmol) and potassium carbonate (808 mg, 5.85 mmol) were added to N,N-dimethylformamide (10 mL), and then sodium chlorodifluoroacetate (2 g, 13.3 mmol) was slowly added to the reaction solution, and stirred at 90°C for six hours. Upon completion of the reaction, ethyl acetate (100 mL) was added. The organic phases were washed with saturated saline. The organic phases were combined and concentrated to afford a crude product. Thereafter, the crude product was purified by column chromatography to afford a colorless oily product **XXXb** (414 mg, yield 43.0%), MS(ESI, m/z): 362[M+H]⁺.

Step ai: Compound **XXXb** (414 mg, 1.14 mmol) was added to dichloromethane (15 mL), and cooled to 0°C. Thereafter, 1 mol/L boron tribromide solution (2.2 mL, 2.29 mmol) was slowly added to the reaction solution, and stirred at 0°C for one hour. Upon completion of the reaction, ethyl acetate (100 mL) was added. The organic phases were washed with saturated saline. The organic phases were combined and concentrated to afford a crude product. Thereafter, the crude product was purified by column chromatography to afford a colorless oily product **Xg** (220 mg, yield 79.7%), MS(ESI, m/z): 242[M+H]⁺.

Step ak: The raw material **Xe** was replaced with **Xg,** and then the synthetic method of Example **58** was followed to afford compound **60** (7.5 mg, yield 10.0%) as a white solid, MS(ESI, m/z): 471[M+H]⁺.

¹H NMR (400 MHz, DMSO-d6) δ 8.05 (s, 2H), 7.84 - 7.80 (m, 1H), 7.75 (t, *J =* 58.4 Hz, 1H), 7.66 (dd, *J* = 7.6 Hz, 1.6 Hz, 1H), 7.48 (t, J = 7.6 Hz, 1H), 7.36 (d, *J =* 8.0 Hz, 1H), 7.08 (t, *J* = 7.6 Hz, 1H), 5.37 (s, 2H), 2.67 (d, *J =* 4.8 Hz, 3H).

### Example 61: Synthesis of Compound 61

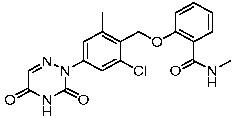

### Synthetic route:

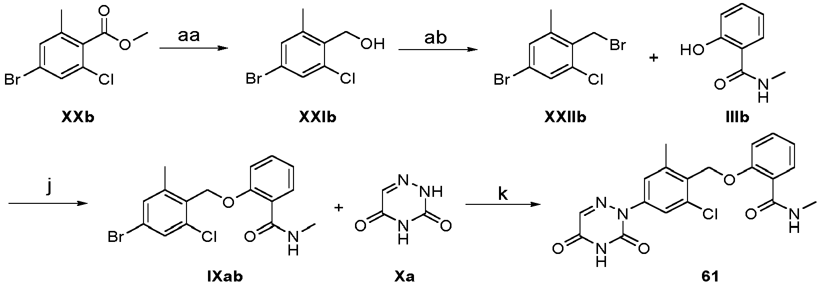

The synthetic method of Example **48** was followed to afford compound 61 (20 mg, yield 15.5%) as a white solid, MS(ESI, m/z): 401[M+H]⁺.

¹H NMR (400 MHz, DMSO-d6) δ 12.38 (s, 1H), 7.94 - 7.85 (m, 1H), 7.66 (s, 1H), 7.62 - 7.54 (m, 2H), 7.46 (t, *J =* 8.4 Hz, 1H), 7.43 - 7.41 (m, 1H), 7.34 (d, *J =* 8.4 Hz, 1H), 7.06 (t, *J* = 7.6 Hz, 1H), 5.29 (s, 2H), 2.65 (d, *J =* 4.8 Hz, 3H), 2.45 (s, 3H).

### Example 62: Synthesis of Compound 62

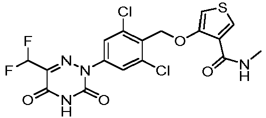

### Synthetic route:

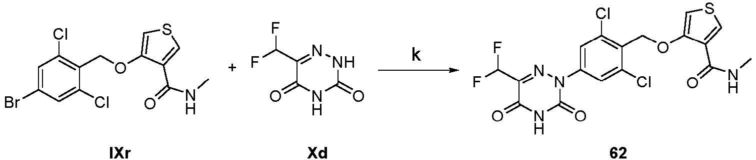

The synthetic method of Example 17 was followed to afford compound 62 (10 mg, yield 8.3%) as a yellow solid, MS(ESI, m/z): 477[M+H]⁺.

¹H NMR (400 MHz, DMSO-d6) δ 8.01 (d, *J* = 3.6 Hz, 1H), 7.79 (s, 2H), 7.43 (s, 1H), 7.08 (d, *J* = 3.6 Hz, 1H), 5.36 (s, 2H), 2.70 (d, *J =* 4.4 Hz, 3H).

### Example 63: Synthesis of Compound 63

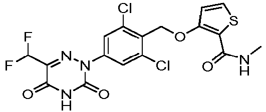

### Synthetic route:

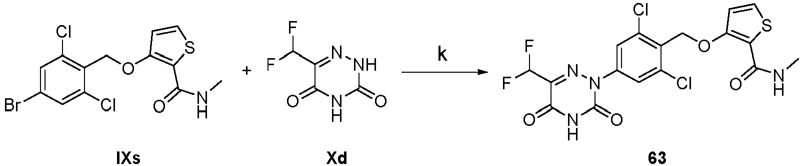

The synthetic method of Example 17 was followed to afford compound **63** (50 mg, yield 38.5%) as a yellow solid, MS(ESI, m/z): 477[M+H]⁺.

¹H NMR (400 MHz, DMSO-d6) δ 12.86 (s, 1H), 7.79 (s, 2H), 7.76 (d, *J =* 5.6 Hz, 1H), 7.34 (d, *J =* 5.6 Hz, 1H), 7.21 - 7.16 (m, 1H), 6.92 (t, *J =* 52.4 Hz, 1H), 5.48 (s, 2H), 2.74 (d, *J* = 4.4 Hz, 3H).

### Example 64: Synthesis of Compound 64

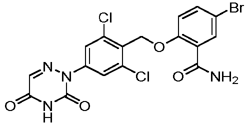

### Synthetic route:

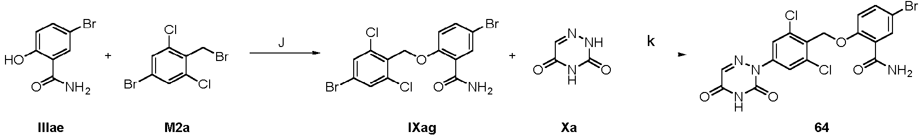

The raw material **IIIb** was replaced with **IIIae,** and then the synthetic method of Example **17** was followed to afford compound **64** (3.28 mg, yield 7.1%) as a white solid, MS(ESI, m/z): 485[M+H]⁺.

¹H NMR (400 MHz, DMSO-d6) δ 7.83 (d, *J =* 2.4 Hz, 1H), 7.79 (s, 2H), 7.71 (dd, *J =* 7.2 Hz, 2.4 Hz, 1H), 7.67(s, 1H), 7.60 (s, 1H), 7.39 (d, *J =* 8.8 Hz, 1H), 7.30 (s, 1H), 5.38 (s, 2H).

### Example 65: Synthesis of Compound 65

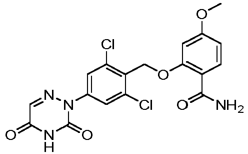

### Synthetic route:

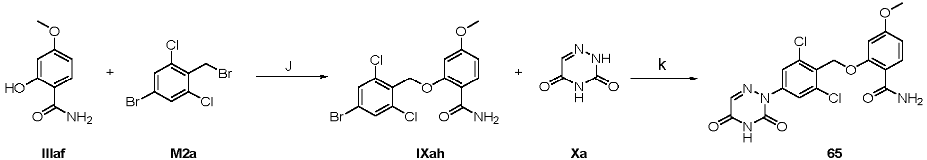

The raw material **IIIb** was replaced with **IIIaf,** and then the synthetic method of Example **17** was followed to afford compound 65 (6.04 mg, yield 9.7%) as a white solid, MS(ESI, m/z): 437[M+H]⁺.

¹H NMR (400 MHz, DMSO-d6) δ 7.87 (d, *J* = 8.8 Hz, 1H), 7.81 (s, 2H), 7.67 (s, 1H), 7.33 (s, 1H), 7.04 (s, 1H), 6.92 (s, 1H), 6.70 (d, *J =* 8.8 Hz, 1H), 5.43 (s, 2H), 3.86 (s, 3H).

### Example 66: Synthesis of Compound 66

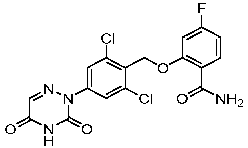

### Synthetic route:

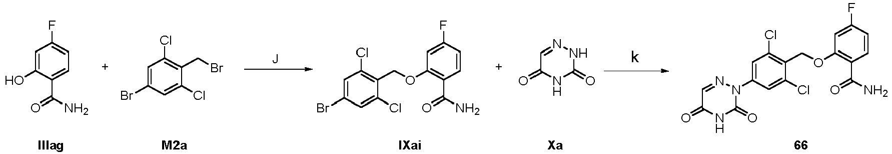

The raw material **IIIb** was replaced with **IIIag,** and then the synthetic method of Example **17** was followed to afford compound **66** (1.43 mg, yield 2.3%) as a white solid, MS(ESI, m/z): 425[M+H]⁺.

¹H NMR (400 MHz, DMSO-d6) δ 7.87 (t, *J =* 7.6 Hz, 1H), 7.81 (s, 2H), 7.64 (s, 1H), 7.49 (s, 1H), 7.37 (d, *J =* 10.8 Hz, 1H), 7.13 (s, 1H), 6.95 (d, *J =* 8.4 Hz, 1H), 5.41 (s, 2H).

### Example 67: Synthesis of Compound 67

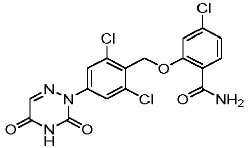

### Synthetic route:

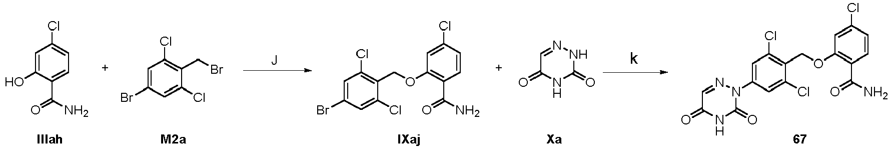

The raw material **IIIb** was replaced with **IIIah,** and then the synthetic method of Example **17** was followed to afford compound **67** (2.81 mg, yield 4.5%) as a white solid, MS(ESI, m/z): 441[M+H]⁺.

¹H NMR (400 MHz, DMSO-d6) δ 7.80 (s, 2H), 7.78 (d, *J* = 8.4 Hz, 1H), 7.71 (s, 1H), 7.54 (s, 2H), 7.18 (d, *J =* 8.4 Hz, 2H), 5.43 (s, 2H).

### Example 68: Synthesis of Compound 68

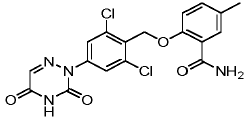

### Synthetic route:

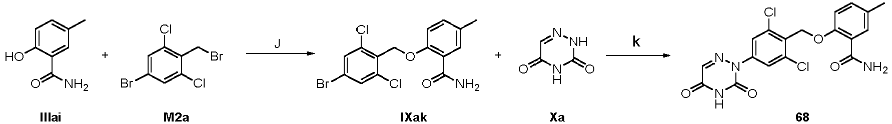

The raw material **IIIb** was replaced with **IIIai,** and then the synthetic method of Example **17** was followed to afford compound **68** (6.6 mg, yield 9.3%) as a white solid, MS(ESI, m/z): 421[M+H]⁺.

¹H NMR (400 MHz, DMSO-d6) δ 7.80 (s, 2H), 7.63 (s, 1H), 7.60 (s, 1H), 7.45 (s, 1H), 7.34 (d, *J* = 8.4 Hz, 1H), 7.28 (d, *J* = 8.4 Hz, 1H), 7.22 (s, 1H), 5.35 (s, 2H), 2.29 (s, 3H).

### Example 69: Synthesis of Compound 69

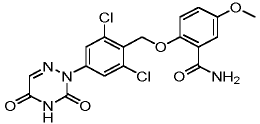

### Synthetic route:

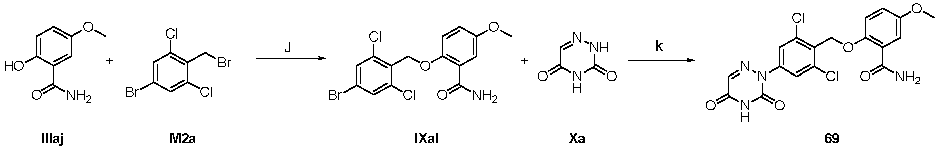

The raw material **IIIb** was replaced with **IIIaj,** and then the synthetic method of Example 17 was followed to afford compound **69** (4.01 mg, yield 11.0%) as a white solid, MS(ESI, m/z): 437[M+H]⁺.

¹H NMR (400 MHz, DMSO-d6) δ 7.79 (s, 2H), 7.64 (s, 1H), 7.54 (s, 1H), 7.36 (d, *J =* 3.2 Hz, 1H), 7.33 - 7.31 (m, 2H), 7.10 (dd, *J =* 8.8 Hz, 3.2 Hz, 1H), 5.33 (s, 2H), 3.75 (s, 3H).

### Example 70: Synthesis of Compound 70

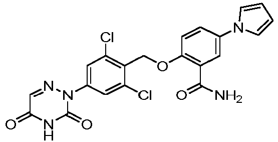

### Synthetic route:

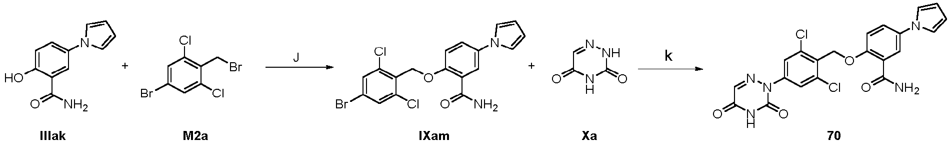

The raw material **IIIb** was replaced with IIIak, and then the synthetic method of Example **17** was followed to afford compound 70 (9.0 mg, yield 20.9%) as a white solid, MS(ESI, m/z): 472[M+H]⁺.

¹H NMR (400 MHz, DMSO-d6) δ 7.85 (d, *J =* 4.0 Hz, 1H), 7.80 (s, 2H), 7.73 - 7.70 (m, 2H), 7.61 (s, 1H), 7.48 (d, *J =* 8.8 Hz, 1H), 7.35 (s, 1H), 7.33 - 7.30 (m, 2H), 6.27 - 6.24 (m, 2H), 5.42 (s, 2H).

### Example 71: Synthesis of Compound 71

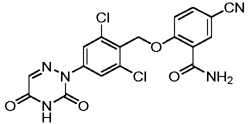

### Synthetic route:

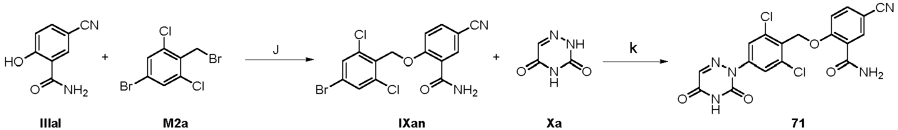

The raw material **IIIb** was replaced with **IIIal,** and then the synthetic method of Example **17** was followed to afford compound **71** (1.36 mg, yield 3.2%) as a white solid, MS(ESI, m/z): 432[M+H]⁺.

¹H NMR (400 MHz, DMSO-d6) δ 8.04 - 7.99 (m, 2H), 7.80 (s, 2H), 7.69 (s, 1H), 7.64 - 7.57 (m, 2H), 7.36 (s, 1H), 5.46 (s, 2H).

### Example 72: Synthesis of Compound 72

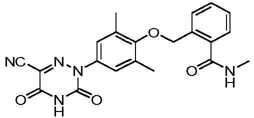

### Synthetic route:

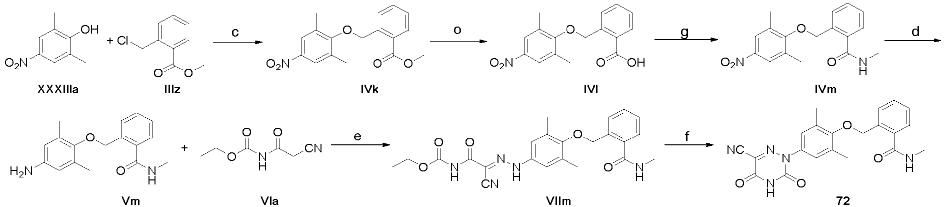

The synthetic method of Example **1** was followed to afford compound **72** (54 mg, yield 25.0%) as a yellow solid. MS(ESI, m/z): 406[M+H]⁺.

¹H NMR (400 MHz, DMSO-d6) δ 12.95 (s, 1H), 8.38 - 8.28 (m, 1H), 7.73 (d, *J =* 7.6 Hz, 1H), 7.52 (t, *J =* 7.6 Hz, 1H), 7.48 - 7.39 (m, 2H), 7.15 (s, 2H), 4.98 (s, 2H), 2.72 (d, *J =* 4.4 Hz, 3H), 2.24 (s, 6H).

### Example 73: Synthesis of Compound 73

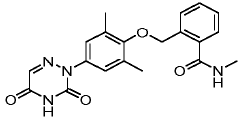

### Synthetic route:

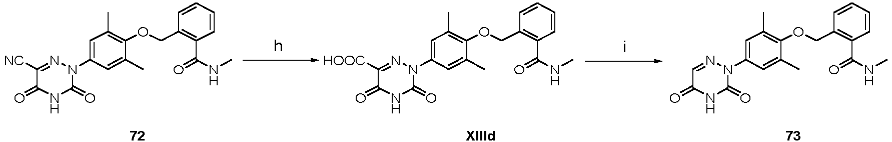

The synthetic method of Example **14** was followed to afford compound **73** (20 mg, yield 52.6%) as a white solid. MS(ESI, m/z): 381[M+H]⁺.

¹H NMR (400 MHz, DMSO-d6) δ 12.27 (s, 1H), 8.36 - 8.29 (m, 1H), 7.73 (d, *J =* 7.6 Hz, 1H), 7.58 (s, 1H), 7.55 - 7.48 (m, 1H), 7.48 - 7.37 (m, 2H), 7.15 (s, 2H), 4.97 (s, 2H), 2.72 (d, *J* = 4.8 Hz, 3H), 2.23 (s, 6H).

### Example 74: Synthesis of Compound 74

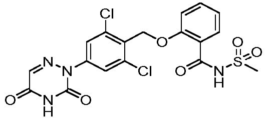

### Synthetic route:

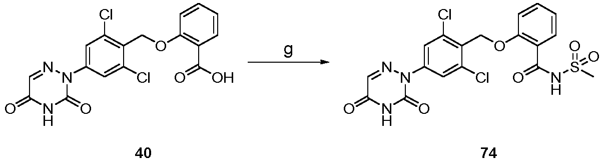

The synthetic method of Example 7 was followed to afford compound 74 (18 mg, yield 76%) as a white solid. MS(ESI, m/z): 485[M+H]⁺.

¹H NMR (400 MHz, DMSO-d6) δ 12.46 (s, 1H), 11.48 (s, 1H), 7.77 (s, 2H), 7.72 (s, 1H), 7.62-7.55 (m, 2H), 7.42 (d, *J =* 8.8 Hz, 1H), 7.13 (t, *J =* 7.6 Hz, 1H), 5.40 (s, 2H), 3.17 (s, 3H).

### Example 75: Synthesis of Compound 75

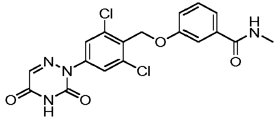

### Synthetic route:

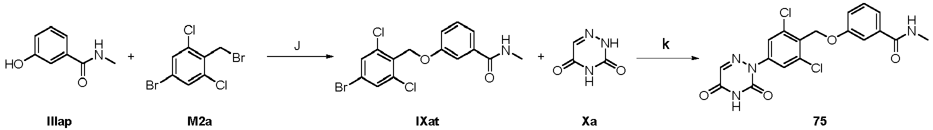

The synthetic method of Example 17 was followed to afford compound 75 (2.61 mg, yield 10.0%) as a white solid, MS(ESI, m/z): 421[M+H]⁺.

¹H NMR (400 MHz, DMSO-d6) δ 8.44 (s, 1H), 7.79 (s, 2H), 7.61 (s, 1H), 7.52 (s, 1H), 7.46 (s, 1H), 7.40 (d, *J* = 7.6 Hz, 1H), 7.23 (t, *J* = 9.6 Hz, 1H), 5.29 (s, 2H), 2.79 (d, *J* = 4.8 Hz, 2H).

### Example 76: Synthesis of Compound 76

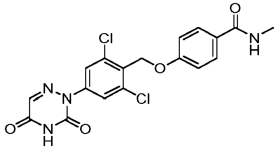

### Synthetic route:

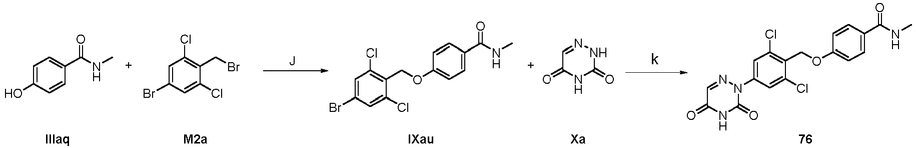

The synthetic method of Example **17** was followed to afford compound 76 (1.72 mg, yield 6.0%) as a white solid, MS(ESI, m/z): 421[M+H]⁺.

¹H NMR (400 MHz, DMSO-d6) δ 8.32 (d, *J =* 5.6 Hz, 1H), 7.83 (d, *J =* 8.4 Hz, 2H), 7.78 (s, 2H), 7.64 (s, 1H), 7.13 (d, *J =* 8.4 Hz, 2H), 5.30 (s, 2H), 2.77 (d, *J =* 4.4 Hz, 3H).

### Example 77: Synthesis of Compound 77

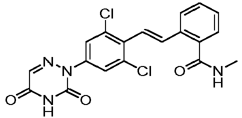

### Synthetic route:

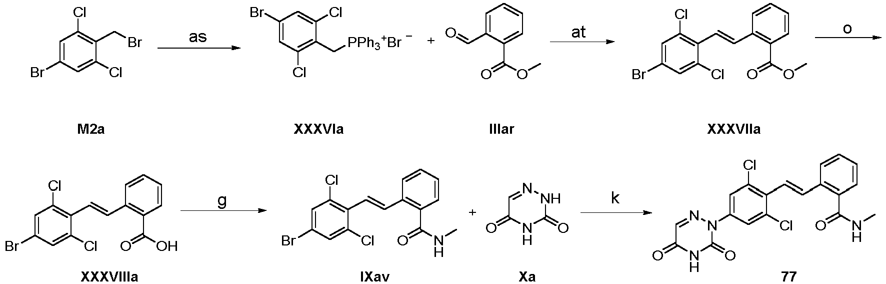

Step as: Compound **M2a** (868 mg, 2.72 mmol) and triphenylphosphine (749 mg, 2.86 mmol) were added to acetonitrile (20 mL), and then heated to 85°C, and stirred overnight. Upon completion of the reaction, the resultant was directly concentrated to afford compound **XXXVIa** (1.36 g, yield 99%) as a white solid. MS(ESI, m/z): 499[M+H]⁺.

Step at: Compound **XXXVIa** (1.36 g, 2.71 mmol) was added to tetrahydrofuran (30 mL), and then a potassium t-butoxide solution (4 mL, 4.07 mmol) was slowly added to the reaction solution under the ice bath condition, and stirred at 0°C for half an hour. Thereafter, compound **IIIar** (531 mg, 3.26 mmol) was added to the reaction solution, and heated to 60°C and stirred overnight. Upon completion of the reaction, ethyl acetate (200 mL) was added. The organic phases were washed with saturated saline. The organic phases were combined and concentrated to afford a crude product. Afterwards, the crude product was purified by column chromatography to afford a yellow oily product **XXXVIIa** (840 mg, yield 80.0%). MS(ESI, m/z): 385[M+H]⁺.

Step o, step g, and step k: The synthetic method of Example **17** was followed to afford compound 77 (6 mg, yield 5.6%) as a white solid, MS(ESI, m/z): 417[M+H]⁺.

¹H NMR (400 MHz, DMSO-d6) δ 8.36 - 8.33 (m, 1H), 7.89 (d, *J =* 8.0 Hz, 1H), 7.74 (s, 2H), 7.71 (s, 1H), 7.53 - 7.48 (m, 1H), 7.45 (d, *J =* 16.8 Hz, 1H), 7.42 - 7.40 (m, 2H), 7.14 (d, *J =* 16.8 Hz, 1H), 2.74 (d, *J =* 4.4 Hz, 3H).

### Example 78: Synthesis of Compound 78

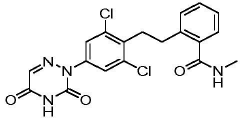

### Synthetic route:

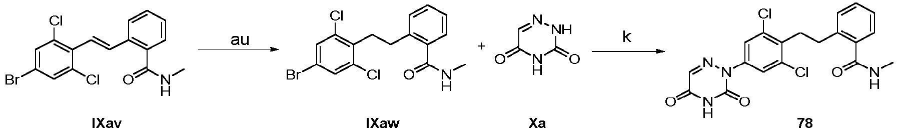

Step au: Compound **IXav** (100 mg, 0.21 mmol), p-toluenesulfonyl hydrazide (384 mg, 2.1 mmol), and sodium acetate (169 mg, 2.1 mmol) were added to ethanol (20 mL), and then heated to 95°C and stirred overnight. Upon completion of the reaction, the resultant was directly concentrated to afford a crude product. Subsequently, the crude product was purified by column chromatography to afford compound **IXaw** (30 mg, yield 30%) as a white solid. MS(ESI, m/z): 386[M+H]⁺.

Step k: The synthetic method of Example **17** was followed to afford compound **78** (9.3 mg, yield 36.0%) as a white solid, MS(ESI, m/z): 419[M+H]⁺.

¹H NMR (400 MHz, DMSO-d6) δ 8.26 - 8.22 (m, 1H), 7.68 (s, 1H), 7.65 (s, 2H), 7.43 - 7.36 (m, 2H), 7.30 - 7.27 (m, 2H), 3.13 - 3.09 (m, 2H), 2.95 - 2.90 (m, 2H), 2.75 (d, *J =* 4.4 Hz, 3H).

### Example 79: Synthesis of Compound 79

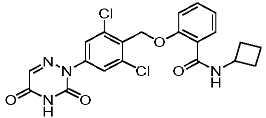

### Synthetic route:

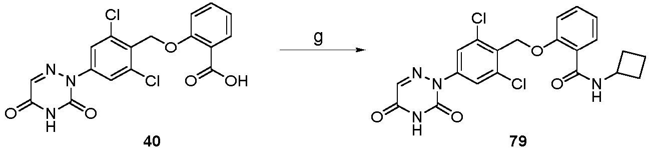

The synthetic method of Example 7 was followed to afford compound **79** (7 mg, yield 30%) as a white solid. MS(ESI, m/z): 461[M+H]⁺.

¹H NMR (400 MHz, DMSO-d6) δ 12.43 (s, 1H), 7.95 (d, *J =* 8.0 Hz, 1H), 7.82 (s, 2H), 7.73 (d, *J =* 8.0 Hz, 2H), 7.53 (t, *J =* 7.6 Hz, 1H), 7.39 (d, J = 8.0 Hz, 1H), 7.11 (t, *J =* 7.6 Hz, 1H), 5.42 (s, 2H), 4.31 - 4.15 (m, 1H), 2.15 - 2.07 (m, 2H), 1.60 - 1.49 (m, 4H).

### Example 80: Synthesis of Compound 80

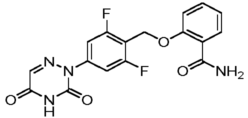

### Synthetic route:

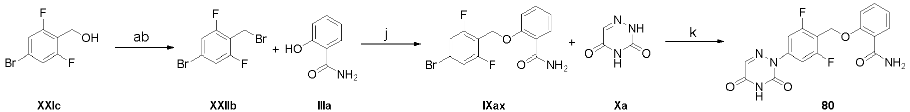

The synthetic method of Example **48** was followed to afford compound **80** (400 mg, yield 46.8%) as a white solid. MS(ESI, m/z): 375[M+H]⁺.

¹H NMR (400 MHz, DMSO-d6) δ 12.48 (s, 1H), 7.76 (d, *J* = 7.6 Hz, 2H), 7.57 - 7.39 (m, 4H), 7.36 (s, 1H), 7.32 (d, *J* = 8.4 Hz, 1H), 7.07 (t, *J* = 7.6 Hz, 1H), 5.31 (s, 2H).

### Example 81: Synthesis of Compound 81

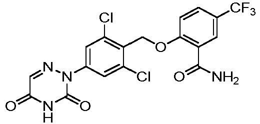

### Synthetic route:

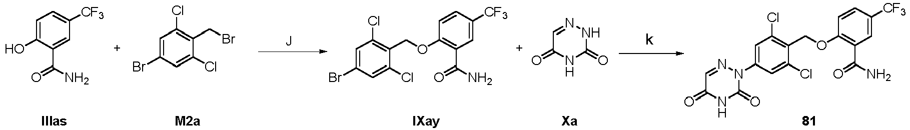

The synthetic method of Example **17** was followed to afford compound **81** (3 mg, yield 1.5%) as a white solid. MS(ESI, m/z): 475[M+H]⁺.

¹H NMR (400 MHz, DMSO-d6) δ 8.01 (d, *J* = 2.4 Hz, 1H), 7.92 (dd, *J* = 8.4 Hz, 2.4 Hz, 1H), 7.80 (s, 2H), 7.70 (s, 1H), 7.67 (s, 1H), 7.62 (d, *J* = 9.2 Hz, 1H), 7.34 (s, 1H), 5.48 (s, 2H).

### Example 82: Synthesis of Compound 82

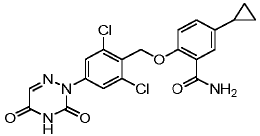

### Synthetic route:

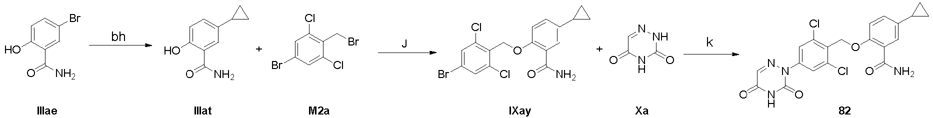

Step bh: Compound **IIIae** (264 mg, 1.22 mmol), cyclopropylboronic acid (136 mg, 1.58 mmol), palladium acetate (14 mg, 0.061 mmol), tricyclohexylphosphine (34 mg, 0.122 mmol), and potassium phosphate (905 mg, 4.27 mmol) were added to toluene (10 mL) and water (0.5 mL), and then heated to 100°C and stirred overnight. Upon completion of the reaction, ethyl acetate (100 mL) was added. The organic phases were washed with saturated saline. The organic phases were combined and concentrated to afford a crude product. Subsequently, the crude product was purified by column chromatography to afford compound **IIIat** (100 mg, yield 46.3%) as a white solid. MS(ESI, m/z): 453[M+H]⁺.

The synthetic method of Example **17** was followed to afford compound **82** (16 mg, yield 38.1%) as a white solid. MS(ESI, m/z): 447[M+H]⁺.

¹H NMR (400 MHz, DMSO-d6) δ 7.79 (s, 2H), 7.67 (s, 1H), 7.52 (s, 1H), 7.45 (s, 1H), 7.29 - 7.20 (m, 3H), 5.35 (s, 2H), 1.98 -1.89 (m, 1H), 0.96 - 0.90 (m, 2H), 0.65 - 0.60 (m, 2H).

### Example 83: Synthesis of Compound 83

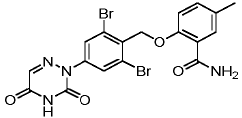

### Synthetic route:

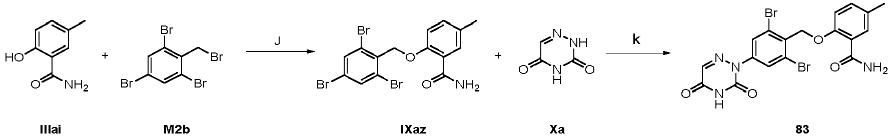

The synthetic method of Example **17** was followed to afford compound **83** (36.7 mg, yield 31.4%) as a white solid. MS(ESI, m/z): 511[M+H]⁺.

¹H NMR (400 MHz, DMSO-*d*6) δ 7.97 (s, 2H), 7.77 - 7.66 (m, 3H), 7.45 (s, 1H), 7.36 - 7.34 (m, 1H), 7.30 (s, 1H), 7.17 (s, 1H), 5.39 (s, 2H), 2.29 (s, 3H).

### Example 84: Synthesis of Compound 84

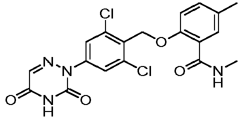

### Synthetic route:

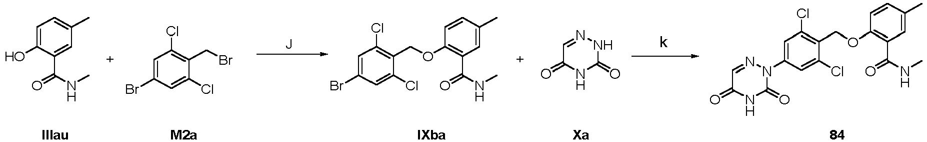

The synthetic method of Example **17** was followed to afford compound **84** (1.69 mg, yield 3.1%) as a white solid. MS(ESI, m/z): 435[M+H]⁺.

¹H NMR (400 MHz, DMSO-d6) δ 7.86 - 7.81 (m, 1H), 7.78 (s, 2H), 7.66 (s, 1H), 7.49 (s, 1H), 7.31 - 7.25 (m, 2H), 5.33 (s, 2H), 2.68 (d, *J =* 4.8 Hz, 3H), 2.28 (s, 3H).

### Example 85: Synthesis of Compound 85

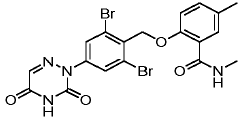

### Synthetic route:

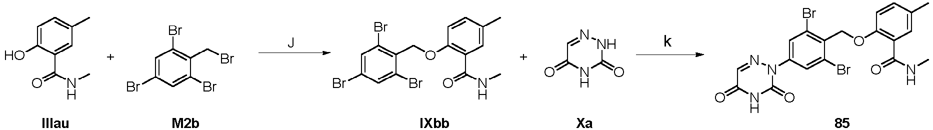

The synthetic method of Example **17** was followed to afford compound **85** (2.43 mg, yield 4.6%) as a white solid. MS(ESI, m/z): 525[M+H]⁺.

¹H NMR (400 MHz, DMSO-d6) δ 8.16 (s, 1H), 7.96 (s, 2H), 7.80 - 7.74 (m, 1H), 7.52 (s, 1H), 7.32 - 7.27 (m, 2H), 5.37 (s, 2H), 2.69 (d, *J* = 4.4 Hz, 3H), 2.29 (s, 3H).

### Example 86: Synthesis of Compound 86

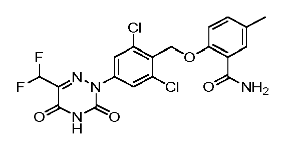

### Synthetic route:

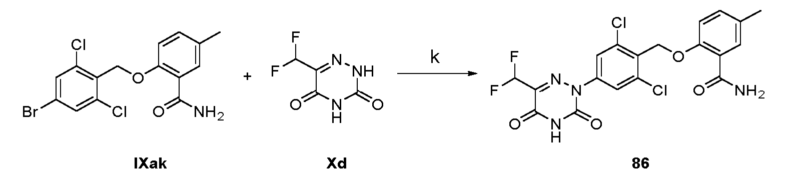

The synthetic method of Example 17 was followed to afford compound 86 (35.7 mg, yield 21.0%) as a white solid. MS(ESI, m/z): 471[M+H]⁺.

¹H NMR (400 MHz, DMSO-d6) δ 7.79 (s, 2H), 7.62 (s, 1H), 7.43 (s, 1H), 7.34 (d, *J =* 8.0 Hz, 1H), 7.28 (d, *J =* 8.4 Hz, 1H), 7.22 (s, 1H), 6.91 (t, *J =* 52.8 Hz, 1H), 5.37 (s, 2H), 2.29 (s, 3H).

### Example 87: Synthesis of Compound 87

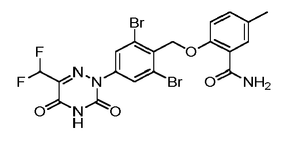

### Synthetic route:

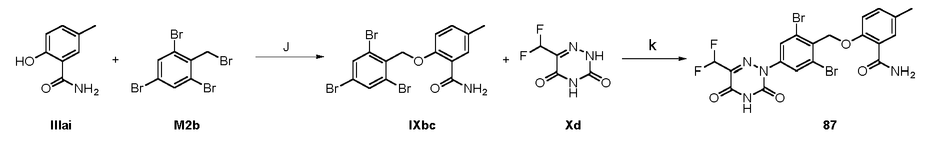

The synthetic method of Example 17 was followed to afford compound 87 (18.8 mg, yield 14.6%) as a white solid. MS(ESI, m/z): 561[M+H]⁺.

¹H NMR (400 MHz, DMSO-d6) δ 7.97 (s, 2H), 7.66 (s, 1H), 7.44 (s, 1H), 7.35 (d, *J =* 8.0 Hz, 1H), 7.29 (d, *J =* 8.4 Hz, 1H), 7.17 (s, 1H), 6.91 (t, *J =* 52.4 Hz, 1H), 5.40 (s, 2H), 2.30 (s, 3H).

### Example 88: Synthesis of Compound 88

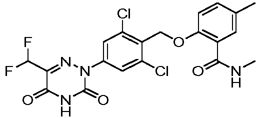

### Synthetic route:

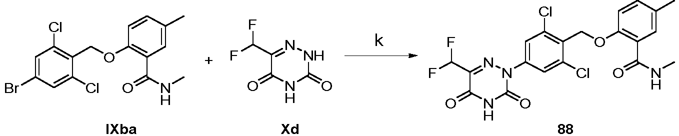

The synthetic method of Example **17** was followed to afford compound **88** (21 mg, yield 35.0%) as a white solid. MS(ESI, m/z): 485[M+H]⁺.

¹H NMR (400 MHz, DMSO-d6) δ 7.86 - 7.80 (m, 1H), 7.77 (s, 2H), 7.48 (s, 1H), 7.31 - 7.25 (m, 2H), 6.92 (t, *J =* 52.8 Hz, 1H), 5.35 (s, 2H), 2.69 (d, *J =* 4.8 Hz, 3H), 2.28 (s, 3H).

### Example 89: Synthesis of Compound 89

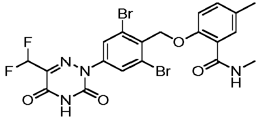

### Synthetic route:

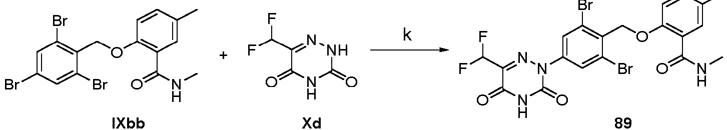

The synthetic method of Example **17** was followed to afford compound **89** (8 mg, yield 13.6%) as a white solid. MS(ESI, m/z): 575[M+H]⁺.

¹H NMR (400 MHz, DMSO-d6) δ 7.96 (s, 2H), 7.81 - 7.74 (m, 1H), 7.51 (s, 1H), 7.32 - 7.26 (m, 2H), 6.91 (t, *J =* 52.4 Hz, 1H), 5.37 (s, 2H), 2.69 (d, *J =* 4.4 Hz, 3H), 2.29 (s, 3H).

### Example 90: Synthesis of Compound 90

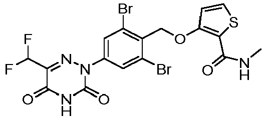

### Synthetic route:

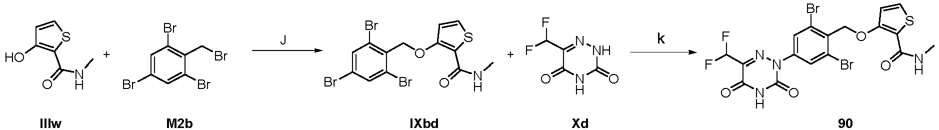

The synthetic method of Example 17 was followed to afford compound 90 (4.05 mg, yield 4.9%) as a white solid. MS(ESI, m/z): 567[M+H]⁺.

¹H NMR (400 MHz, DMSO-d6) δ 7.97 (s, 2H), 7.76 (d, *J =* 5.2 Hz, 1H), 7.35 (d, *J =* 5.6 Hz, 1H), 7.18 - 7.13 (m, 1H), 6.91 (t, *J =* 52.4 Hz, 1H), 5.49 (s, 2H), 2.74 (d, *J =* 4.8 Hz, 3H).

### Example 91: Synthesis of Compound 91

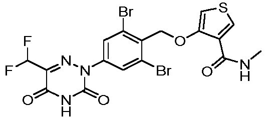

### Synthetic route:

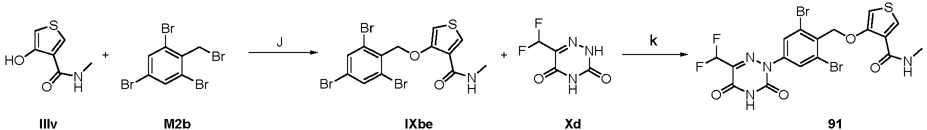

The synthetic method of Example **17** was followed to afford compound **91** (31.6 mg, yield 27.0%) as a white solid. MS(ESI, m/z): 567[M+H]⁺.

¹H NMR (400 MHz, DMSO-d6) δ 8.02 (d, *J =* 3.2 Hz, 1H), 7.97 (s, 2H), 7.42 - 7.36 (m, 1H), 7.10 (d, *J =* 3.2 Hz, 1H), 6.90 (t, *J =* 52.4 Hz, 1H), 5.37 (s, 2H), 2.70 (d, *J =* 4.8 Hz, 3H).

### Example 92: Synthesis of Compound 92

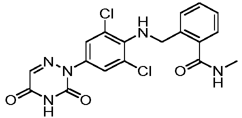

### Synthetic route:

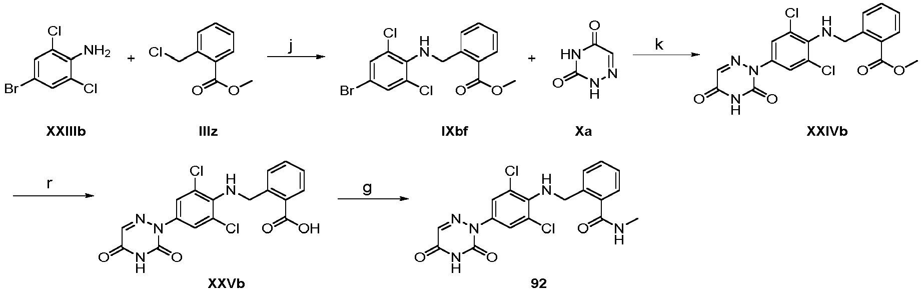

The synthetic method of Example **49** was followed to afford compound **92** (25.7 mg, yield 13.4%) as a white solid. MS(ESI, m/z): 420[M+H]⁺.

¹H NMR (400 MHz, DMSO-d6) δ 8.41 - 8.37 (m, 1H), 7.59 (s, 1H), 7.49 - 7.43 (m, 3H), 7.37 - 7.29 (m, 2H), 7.25 (d, *J =* 7.2 Hz, 1H), 5.82 (t, *J =* 7.2 Hz, 1H), 4.61 (d, *J =* 7.2 Hz, 2H), 2.78 (d, *J =* 4.8 Hz, 3H).

### Example 93: Synthesis of Compound 93

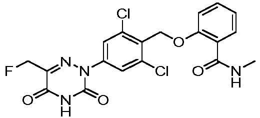

### Synthetic route:

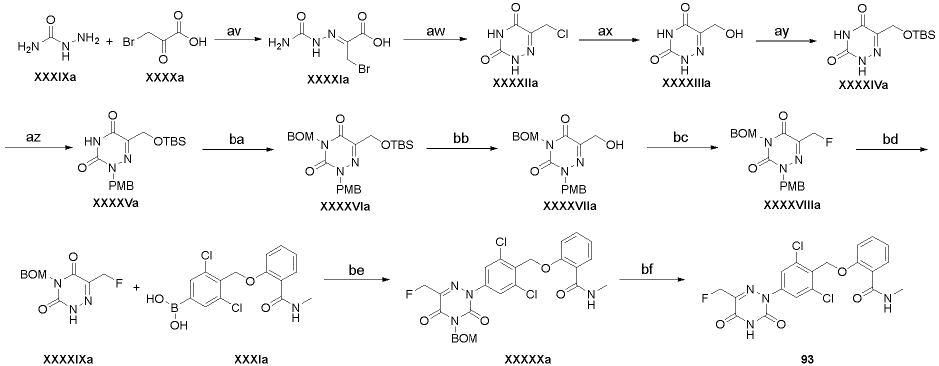

Step av: Compound **XXXIXa** (25 g, 224.2 mmol) was added to 1N diluted hydrochloric acid (200 mL), and then compound **XXXXa** (25 g, 149.7 mmol) was added to the reaction solution at 0°C, and stirred for two hours. Upon completion of the reaction, the resultant was directly filtered to afford compound **XXXXIa** (28.3 g, yield 84.5%) as a white solid.

¹H NMR (400 MHz, DMSO-d6) δ 10.50 (s, 1H), 7.37 (s, 1H), 6.92 (s, 1H), 4.44 (s, 2H).

Step aw: Compound **XXXXIa** (28.3 g, 126.3 mmol) was added to thionyl chloride (200 mL), and then heated to 80°C and stirred overnight. Upon completion of the reaction, the resultant was directly concentrated to afford compound **XXXXIIa** (32 g, yield 99%) as a yellow solid. MS(ESI, m/z): 162[M+H]⁺.

Step ax: Compound **XXXXIIa** (32 g, 197.5 mmol) was added to water (300 mL), and then heated to 100°C and stirred for four hours. Upon completion of the reaction, the resultant was directly concentrated to afford compound **XXXXIIIa** (23 g, yield 81.0%) as a gray solid. MS(ESI, m/z): 144[M+H]⁺.

Step ay: Compound **XXXXIIIa** (23 g, 159.7 mmol), imidazole (32.6 g, 479.2 mmol), and t-butyldimethylchlorosilane (36.1 g, 239.6 mmol) were added to N,N-dimethylformamide (200 mL), and stirred at room temperature for two hours. Upon completion of the reaction, ethyl acetate (500 mL) was added. The organic phases were washed with saturated saline. The organic phases were combined and concentrated to afford a crude product. Thereafter, the crude product was purified by column chromatography to afford compound **XXXXIVa** (14 g, yield 34.1%) as a yellow solid. MS(ESI, m/z): 258[M+H]⁺.

Step az: Compound **XXXXIVa** (1.4 g, 5.45 mmol) and N,O-bis(trimethylsilyl)acetamide (2.21 g, 10.9 mmol) were added to acetonitrile (15 mL), and then heated to 85°C, and stirred for two hours. Thereafter, 4-methoxybenzylchloride (935 mg, 5.99 mmol) and sodium iodide (817 mg, 5.45 mmol) were added to the reaction solution and stirred overnight at 85°C. Upon completion of the reaction, ethyl acetate (100 mL) was added. The organic phases were washed with saturated saline. The organic phases were combined and concentrated to afford a crude product. Afterwards, the crude product was purified by column chromatography to afford compound **XXXXVa** (972 mg, yield 47.4%) as a yellow solid. MS(ESI, m/z): 378[M+H]⁺.

Step ba: Compound **XXXXVa** (972 mg, 2.58 mmol) was dissolved into N,N-dimethylformamide (10 mL), and then sodium hydride (155 mg, 3.87 mmol) was added at 0°C and stirred for 30 min. Thereafter, benzyl chloromethyl ether (442 mg, 2.83 mmol) was added to the reaction solution and reacted at room temperature for one hour. Upon completion of the reaction, ethyl acetate (100 mL) was added. The organic phases were washed with saturated saline. The organic phases were combined and concentrated to afford a crude product. Afterwards, the crude product was purified by column chromatography to afford compound **XXXXVIa** (1.25 g, yield 97%) as a yellow solid. MS(ESI, m/z): 498[M+H]⁺.

Step bb: Compound **XXXXVIa** (1.25 g, 2.51 mmol) was dissolved into tetrahydrofuran (10 mL), and then a tetrabutylammonium fluoride solution (2.51 mL, 2.51 mmol, 1 mol/L in THF) was added to the reaction solution and stirred at room temperature for one hour. Upon completion of the reaction, the resultant was directly concentrated to afford a crude product. Subsequently, the crude product was purified by column chromatography to afford compound **XXXXVIIa** (900 mg, yield 100%) as a white solid. MS(ESI, m/z): 384[M+H]⁺.

Step bc: Compound **XXXXVIIa** (500 mg, 1.14 mmol) was dissolved into dichloromethane (10 mL), and then diethylaminosulphur trifluoride (296 mg, 1.84 mmol) was added at 0°C and stirred at room temperature for one hour. Upon completion of the reaction, the resultant was directly concentrated to afford a crude product. Subsequently, the crude product was purified by column chromatography to afford compound **XXXXVIIIa** (340 mg, yield 68%) as a white solid. MS(ESI, m/z): 386[M+H]⁺.

Step bd: Compound **XXXXVIIIa** (340 mg, 0.955 mmol) was added to a mixed solution of acetonitrile (15 mL) and water (5 mL), and then ceric ammonium nitrate (2.5 g, 4.78 mmol) was added to the reaction solution, heated to 80°C, and stirred for three hours. Upon completion of the reaction, ethyl acetate (100 mL) was added. The organic phases were washed with saturated saline. The organic phases were combined and concentrated to afford a crude product. Subsequently, the crude product was purified by column chromatography to afford compound **XXXXIXa** (110 mg, yield 43%) as a white solid. MS(ESI, m/z): 266[M+H]⁺.

Step be: Compound **XXXXIXa** (50 mg, 0.177 mmol) and compound **XXXIa** (70 mg, 0.161 mmol) were added to N,N-dimethylformamide (1 mL), and then copper acetate (29 mg, 0.161 mmol) and pyridine (25 mg, 0.321 mmol) were added to the reaction solution, and stirred overnight at room temperature in an open state. Upon completion of the reaction, ethyl acetate (100 mL) was added. The organic phases were washed with saturated saline. The organic phases were combined and concentrated to afford a crude product. Subsequently, the crude product was purified by column chromatography to afford compound **XXXXXa** (60 mg, yield 65%) as a white solid. MS(ESI, m/z): 573[M+H]⁺.

Step bf: Compound **XXXXXa** (30 mg, 0.0524 mmol) was added to dichloromethane (2 mL), and then boron tribromide (0.052 mL, 0.052 mmol) was slowly added to the reaction solution at 0°C and stirred at room temperature for two hours. Upon completion of the reaction, methanol (1 mL) was added to quench the system and the resultant was directly concentrated to afford a crude product. Subsequently, the crude product was purified by column chromatography to afford compound 93 (6 mg, yield 31%) as a white solid. MS(ESI, m/z): 453[M+H]⁺.

¹H NMR (400 MHz, DMSO-d6) δ 12.69 (s, 1H), 7.88 - 7.83 (m, 1H), 7.81 (s, 2H), 7.66 (d, *J =* 7.6 Hz, 1H), 7.50 (t, *J =* 8.0 Hz, 1H), 7.38 (d, *J =* 8.4 Hz, 1H), 7.11 (t, *J =* 7.6 Hz, 1H), 5.38 (s, 2H), 5.31 (d, *J =* 46.8 Hz, 2H), 2.69 (d, *J =* 4.8 Hz, 3H).

### Example 94: Synthesis of Compound 94

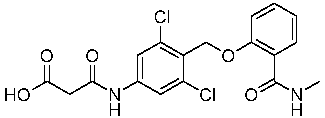

### Synthetic route:

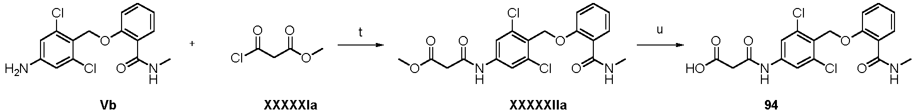

Step t: Compound **Vb** (20 mg, 0.062 mmol), compound **XXXXXIa** (7.6 µL, 0.068 mmol), and triethylamine (10.3 µL, 0.074 mmol) were added to dichloromethane (1mL) under the ice bath condition, and stirred overnight at room temperature. Upon completion of the reaction, the resultant was directly concentrated to afford a crude product. Subsequently, the crude product was purified by column chromatography to afford compound **XXXXXIIa** (9 mg, yield 34%) as a white solid. MS(ESI, m/z): 425[M+H]⁺.

Step u: Compound **XXXXXIIa** (9 mg, 0.021 mmol) was added to a mixed solvent of tetrahydrofuran (0.4 mL), methanol (0.4 mL), and water (0.1 mL), and then lithium hydroxide (1.5 mg, 0.063 mmol) was added, and stirred at room temperature for one hour. Upon completion of the reaction, the resultant was directly concentrated to afford a crude product. Thereafter, the crude product was purified by reverse-phase column chromatography to afford compound 94 (7.9 mg, yield 91%) as a white solid, MS(ESI, m/z): 411[M+H]⁺.

¹H NMR (400 MHz, DMSO-d6) δ 13.22 (s, 1H), 7.85 -7.77 (m, 1H), 7.75 (s, 2H), 7.72 (dd, *J* = 7.6, 2.0 Hz, 1H), 7.52 - 7.46 (m, 1H), 7.36 (d, *J =* 8.4 Hz, 1H), 7.09 (t, J = 7.2 Hz, 1H), 5.29 (s, 2H), 2.88 (s, 2H), 2.69 (d, *J =* 4.8 Hz, 3H).

### Example 95: Synthesis of Compound 95

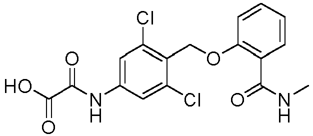

### Synthetic route:

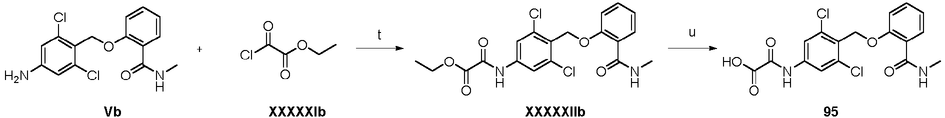

The synthetic method of Example 94 was followed to afford compound 95 (4.1 mg, yield 71%) as a white solid, MS(ESI, m/z): 397[M+H]⁺.

¹H NMR (400 MHz, DMSO-d6) δ10.68 - 10.47 (m, 1H), 8.09 - 8.03 (m, 2H), 7.85 - 7.80 (m, 1H), 7.73 - 7.68 (m, 1H), 7.52 - 7.46 (m, 1H), 7.38 - 7.34 (m, 1H), 7.09 (t, *J =* 7.2 Hz, 1H), 5.29 (s, 2H), 2.68 (d, *J =* 4.8 Hz, 2H).

### Example 96: Synthesis of Compound 96

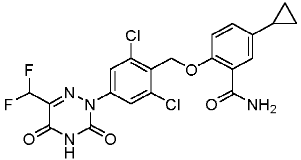

### Synthetic route:

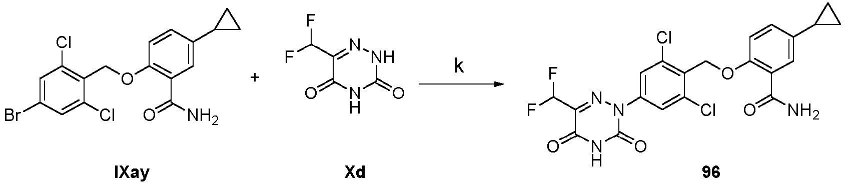

The synthetic method of Example **17** was followed to afford compound 96 (4.4 mg, yield 8.9%) as a white solid. MS(ESI, m/z): 497[M+H]+.

1H NMR (400 MHz, DMSO-d6) 12.83 (s, 1H), 7.78 (s, 2H), 7.51 (s, 1H), 7.44 (s, 1H), 7.28 - 7.22 (m, 3H), 6.91 (t, J = 52.4 Hz, 1H), 5.35 (s, 2H), 1.97 - 1.90 (m, 1H), 0.95 - 0.89 (m, 2H), 0.64 - 0.59 (m, 2H).

### Example 97: Synthesis of Compound 97

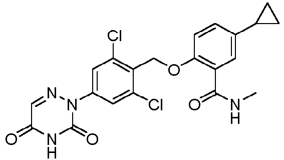

### Synthetic route:

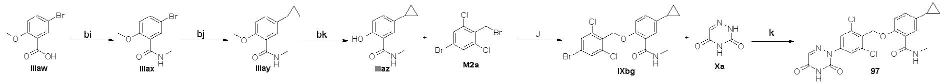

The synthetic methods of Example **30** and Example **82** were followed to afford compound 97 (4.02 mg, yield 12.2%) as a white solid. MS(ESI, m/z): 461[M+H]+.

¹H NMR (400 MHz, DMSO-d6) 12.47 (s, 1H), 7.85 - 7.79 (m, 1H), 7.76 (s, 2H), 7.71 (s, 1H), 7.35 (s, 1H), 7.26 - 7.16 (m, 2H), 5.31 (s, 2H), 2.67 (d, J = 4.4 Hz, 3H), 1.96 - 1.88 (m, 1H), 0.95 - 0.88 (m, 2H), 0.64 - 0.58 (m, 2H).

### Example 98: Synthesis of Compound 98

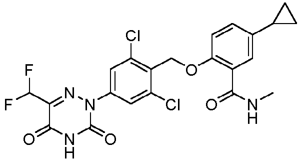

### Synthetic route:

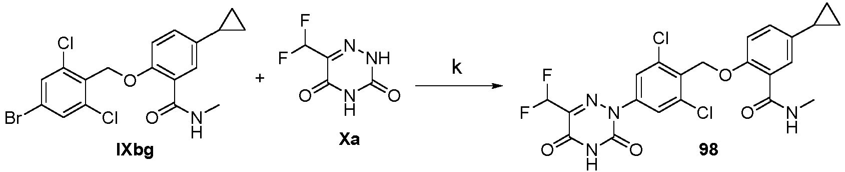

The synthetic method of Example **17** was followed to afford compound **98** (36 mg, yield 14.0%) as a white solid. MS(ESI, m/z): 511[M+H]⁺.

¹H NMR (400 MHz, CDCl₃) 9.42 (s, 1H), 7.92 (s, 1H), 7.75 (s, 2H), 7.73 - 7.67 (m, 1H), 7.21 (d, *J =* 8.4 Hz, 1H), 7.13 (d, *J =* 8.4 Hz, 1H), 6.68 (d, *J =* 52.4 Hz, 1H), 5.41 (s, 2H), 2.93 - 2.87 (m, 3H), 1.97 - 1.87 (m, 1H), 0.98 - 0.92 (m, 2H), 0.75 - 0.68 (m, 2H).

### Example 99: Synthesis of Compound 99

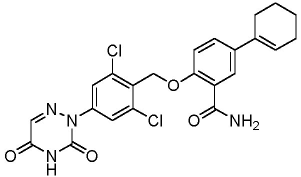

### Synthetic route:

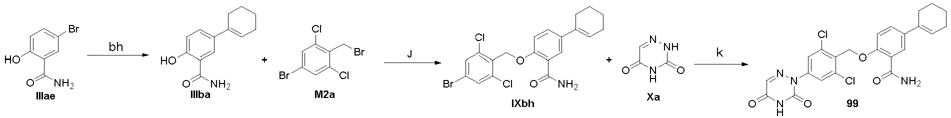

The synthetic method of Example **82** was followed to afford compound **99** (4.5 mg, yield 12.9%) as a white solid. MS(ESI, m/z): 487[M+H]⁺.

¹H NMR (400 MHz, DMSO-d6) 7.82 (s, 1H), 7.79 (s, 2H), 7.71 (s, 1H), 7.56 (d, *J =* 8.0 Hz, 1H), 7.47 (s, 1H), 7.33 (d, *J =* 8.8 Hz, 1H), 7.23 (s, 1H), 6.14 - 6.10 (m, 1H), 5.38 (s, 2H), 2.38 - 2.31 (m, 2H), 2.20 - 2.14 (m, 2H), 1.76 - 1.69 (m, 2H), 1.63 - 1.55 (m, 2H).

### Example 100: Synthesis of Compound 100

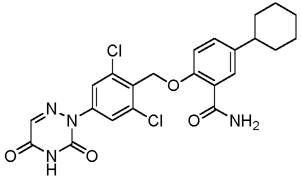

### Synthetic route:

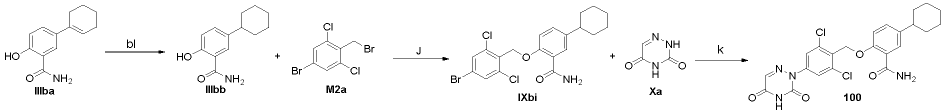

Step bl: Compound **IIIba** (120 mg, 0.55 mmol) was added to methanol (10 mL). Thereafter, Pd/C (12 mg, 10%) was added, and then the mixture was stirred at room temperature for three hours under hydrogen condition. Upon completion of the reaction, the resultant was directly filtered and concentrated to afford a pink solid **IIIbb** (120 mg, yield 99%), MS(ESI, m/z): 220[M+H]⁺.

The synthetic method of Example **82** was followed to afford compound **100** (7.8 mg, yield 22.3%) as a white solid. MS(ESI, m/z): 489[M+H]⁺.

¹H NMR (400 MHz, DMSO-d6) 7.79 (s, 2H), 7.70 (s, 1H), 7.67 (s, 1H), 7.44 (s, 1H), 7.38 (d, *J =* 8.4 Hz, 1H), 7.30 (d, *J =* 8.4 Hz, 1H), 7.20 (s, 1H), 5.36 (s, 2H), 2.55 - 2.52 (m, 1H), 1.83 - 1.68 (m, 6H), 1.40 - 1.33 (m, 4H).

### Example 101: Synthesis of Compound 101

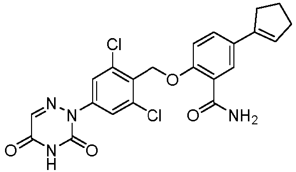

### Synthetic route:

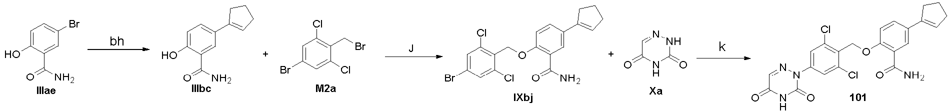

The synthetic method of Example **82** was followed to afford compound **101** (17.7 mg, yield 52.4%) as a white solid. MS(ESI, m/z): 473[M+H]⁺.

¹H NMR (400 MHz, DMSO-d6) 7.84 (s, 1H), 7.79 (s, 2H), 7.68 (s, 1H), 7.63 (d, *J =* 7.6 Hz, 1H), 7.49 (s, 1H), 7.33 (d, *J =* 8.8 Hz, 1H), 7.24 (s, 1H), 6.24 - 6.19 (m, 1H), 5.39 (s, 2H), 2.69 - 2.61 (m, 2H), 2.37 - 2.31 (m, 1H), 2.01 - 1.93 (m, 3H).

### Example 102: Synthesis of Compound 102

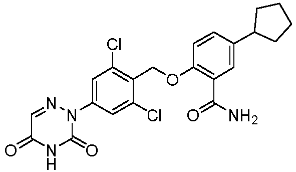

### Synthetic route:

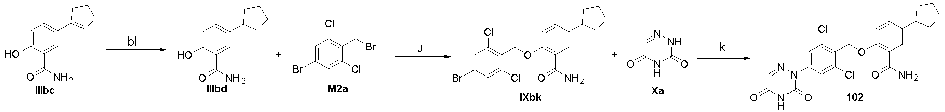

The synthetic methods of Example **82** and Example **100** were followed to afford compound **102** (12.3 mg, yield 33.5%) as a white solid. MS(ESI, m/z): 475[M+H]⁺.

¹H NMR (400 MHz, DMSO-d6) 7.78 (s, 2H), 7.71 - 7.67 (m, 2H), 7.45 (s, 1H), 7.40 (d, *J* = 8.4 Hz, 1H), 7.29 (d, *J =* 8.4 Hz, 1H), 7.21 (s, 1H), 5.35 (s, 2H), 3.00 - 2.93 (m, 1H), 2.04 - 1.96 (m, 2H), 1.79 - 1.72 (m, 2H), 1.68 - 1.59 (m, 2H), 1.55 - 1.43 (m, 2H).

### Example 103: Synthesis of Compound 103

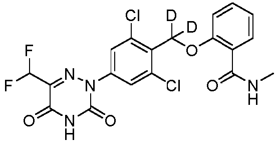

### Synthetic route:

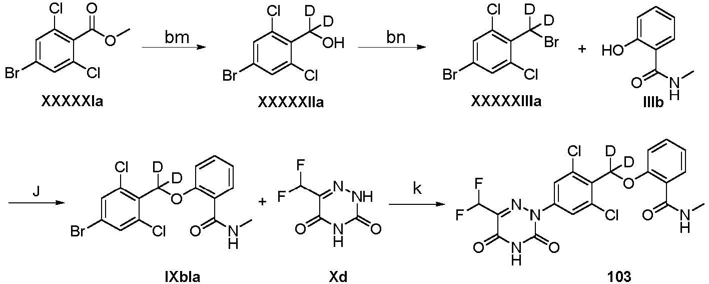

Step bm: Compound **XXXXXIa** (1.7 g, 6.05 mmol) was added to tetrahydrofuran (10 mL), and then deuterated lithium aluminum tetrahydride (508 mg, 12.1 mmol) was slowly added to the reaction solution at 0°C, and stirred at room temperature for two hours. Upon completion of the reaction, 0.5 mL of water, 0.5 mL of 15% sodium hydroxide solution, and 1.5 mL of water were added in this order to the reaction solution and stirred for half an hour. The resultant was directly filtered and concentrated to afford compound **XXXXXIIa** (1.27 g, yield 82.5%) as a yellow solid.

Step bn: Compound **XXXXXIIa** (1.27 g, 5 mmol) was added to dichloromethane (15 mL), and then phosphorus tribromide (678 mg, 2.5 mmol) was slowly added to the reaction solution at 0°C and stirred at room temperature for two hours. Upon completion of the reaction, the resultant was directly concentrated to afford a crude product. Subsequently, the crude product was purified by column chromatography to afford compound **XXXXXIIIa** (1.11 g, yield 70.3%) as a white solid.

The synthetic method of Example **82** was followed to afford compound **103** (110 mg, yield 45.0%) as a white solid. MS(ESI, m/z): 473[M+H]⁺.

¹H NMR (400 MHz, DMSO-d6) 12.82 (s, 1H), 7.87 - 7.81 (m, 1H), 7.78 (s, 2H), 7.65 (d, *J* = 7.6 Hz, 1H), 7.49 (t, *J =* 7.6 Hz, 1H), 7.37 (d, *J =* 8.4 Hz, 1H), 7.10 (t, *J =* 7.6 Hz, 1H), 6.90 (t, *J =* 52.4 Hz, 1H), 2.68 (d, *J =* 4.8 Hz, 3H).

### Example 104: Synthesis of Compound 104

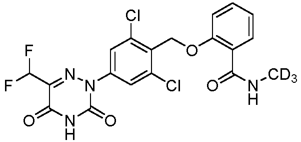

### Synthetic route:

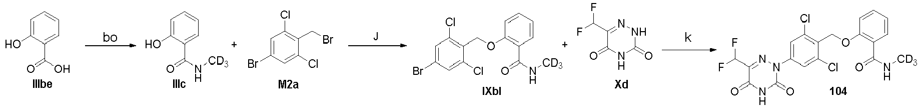

Step bo: Compound **IIIbe** (1.96 g, 14.2 mmol), deuterated methylamine hydrochloride (1 g, 14.2 mmol), and HATU (7 g, 18.4 mmol) were added to N,N-dimethylformamide (15 mL). Thereafter, triethylamine (4.3 g, 42.6 mmol) was added to the reaction solution, and then stirred at room temperature for three hours. Upon completion of the reaction, ethyl acetate (200 mL) was added. The organic phases were washed with saturated saline. The organic phases were combined and concentrated to afford a crude product. Subsequently, the crude product was purified by column chromatography to afford a yellow oily product **IIIc** (600 mg, yield 27.4%), MS(ESI, m/z): 155[M+H]⁺.

The synthetic method of Example **82** was followed to afford compound **104** (78.4 mg, yield 36.1%) as a white solid. MS(ESI, m/z): 474[M+H]⁺.

¹H NMR (400 MHz, DMSO-d6) 12.80 (s, 1H), 7.81 (s, 1H), 7.78 (s, 2H), 7.65 (d, *J =* 7.6 Hz, 1H), 7.49 (t, *J =* 7.6 Hz, 1H), 7.37 (d, *J =* 8.0 Hz, 1H), 7.10 (t, *J =* 7.2 Hz, 1H), 6.90 (t, *J* = 52.4 Hz, 1H), 5.38 (s, 2H).

### Example 105: Synthesis of Compound 105

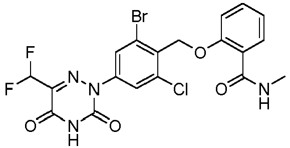

### Synthetic route:

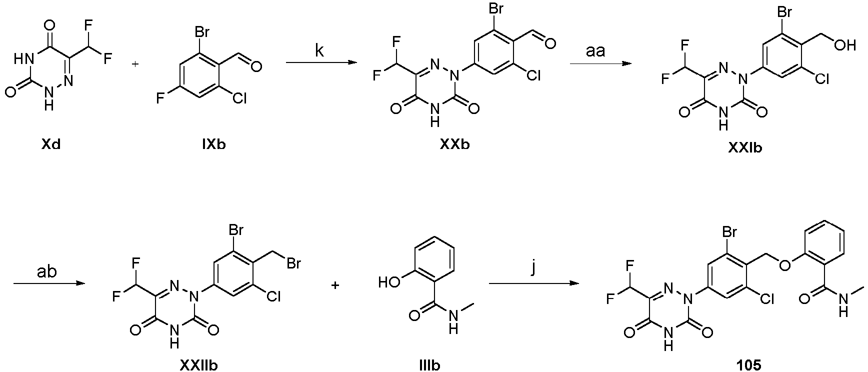

Step **K** of Example **53** was followed to synthesize compound **XXb** (290 mg, yield 68.6%) as a yellow solid, MS(ESI, m/z): 382[M+H]⁺.

With reference to the synthetic route of Example **48,** compound **XXa** was replaced with compound **XXb** to synthesize compound 105 (20 mg, yield 33.4%) as a white solid, MS(ESI, m/z): 517[M+H]⁺.

¹H NMR (400 MHz, DMSO-*d*₆) δ 7.97 - 7.90 (m, 1H), 7.88 - 7.77 (m, 2H), 7.67 (d, *J =* 7.6 Hz, 1H), 7.56 - 7.45 (m, 1H), 7.39 (d, *J =* 8.4 Hz, 1H), 7.11 (t, *J =* 7.6 Hz, 1H), 6.92 (t, *J =* 52.4 Hz, 1H), 5.39 (s, 2H), 2.69 (d, *J =* 4.8 Hz, 3H).

### Example 106: Synthesis of Compound 106

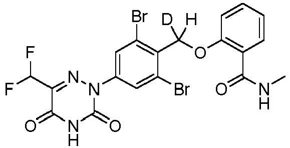

### Synthetic route:

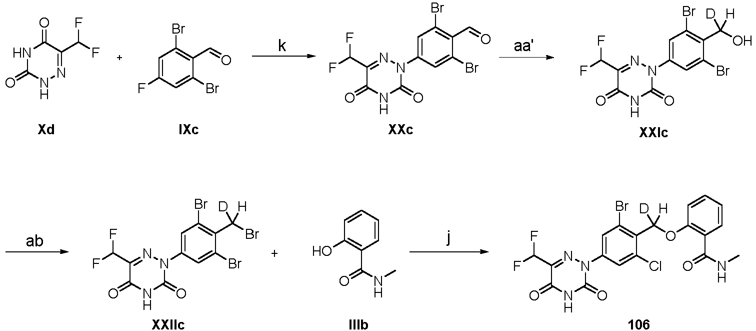

With reference to the synthetic route of Example **105,** compound **IXb** was replaced with compound IXc, and the sodium borohydride in step aa was replaced with deuterated sodium borohydride to synthesize compound 106 (92 mg, yield 53.0%) as a yellow solid, MS(ESI, m/z): 561.7[M+H]⁺.

¹H NMR (400 MHz, DMSO-*d*₆) δ 12.84 (s, 1H), 7.97 (s, 2H), 7.83 - 7.77 (m, 1H), 7.69 (dd, *J= 7.6* Hz, 1.6 Hz, 1H), 7.53 - 7.49 (m, 1H), 7.40 (d, *J =* 8.4 Hz, 1H), 7.13 - 7.09 (m, 1H), 6.92 (t, *J =* 52.4 Hz, 1H), 5.40 (d, *J =* 8.4 Hz, 1H), 2.69 (d, *J =* 4.8 Hz, 3H).

### Example 107: Synthesis of Compound 107

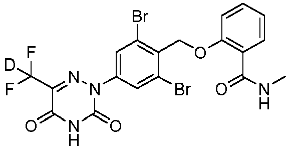

### Synthetic route:

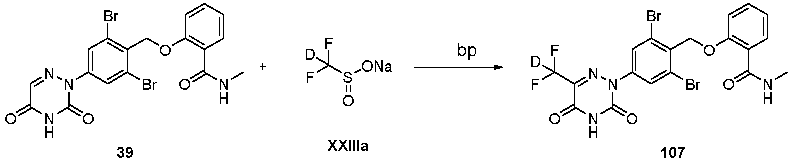

Compound **39** (100 mg, 0.20 mmol), compound **XXIIIa** (55 mg, 0.39 mmol), and Acid Red **94** (4 mg, 0.0039 mmol) were added to dimethylsulfoxide (6 mL), and heated to 50°C under green light, and stirred for one hour. Upon completion of the reaction, the resultant was directly purified by column chromatography to afford compound **107** (29 mg, yield 26.5%) as a white solid, MS(ESI, m/z): 561.7[M+H]⁺.

¹H NMR (400 MHz, DMSO-*d*₆) δ 12.83 (s, 1H), 7.97 (s, 2H), 7.82 - 7.77 (m, 1H), 7.69 (dd, *J =* 7.6 Hz, 1.6 Hz, 1H), 7.53 - 7.49 (m, 1H), 7.40 (d, *J =* 8.0 Hz, 1H), 7.11 (t, *J =* 7.6 Hz, 1H), 5.41 (s, 2H), 2.69 (d, *J =* 4.8 Hz, 3H).

### Example 108: Synthesis of Compound 108

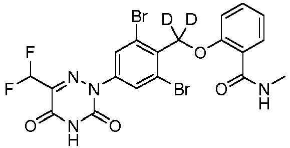

### Synthetic route:

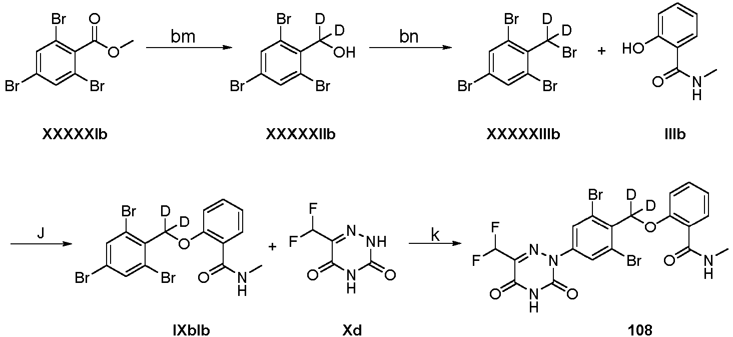

With reference to the synthetic route of Example **103,** compound **XXXXXIa** was replaced with compound **XXXXXIb** to synthesize compound **108** (3 mg, yield 3.8%) as a white solid, MS(ESI, m/z): 562.8[M+H]⁺.

¹H NMR (400 MHz, DMSO-*d*₆) δ 12.83 (s, 1H), 7.97 (s, 2H), 7.83 - 7.77 (m, 1H), 7.69 (dd, *J= 7.6* Hz, 2.0 Hz, 1H), 7.53 - 7.49 (m, 1H), 7.40 (d, *J =* 8.4 Hz, 1H), 7.13 - 7.09 (m, 1H), 6.91 (t, *J=* 52.4 Hz, 1H), 2.69 (d, *J* = 4.8 Hz, 3H).

### Example 109: Synthesis of Compound 109

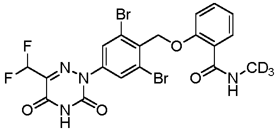

### Synthetic route:

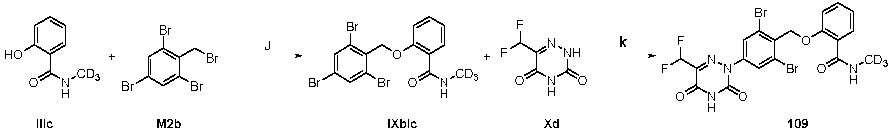

With reference to the synthetic method of Example **104,** compound **M2a** was replaced with compound **M2b** to synthesize compound 109 (76.5 mg, yield 42.1%) as a white solid. MS(ESI, m/z): 563.8[M+H]⁺.

¹H NMR (400 MHz, DMSO-d6) 12.84 (s, 1H), 7.97 (s, 2H), 7.77 (s, 1H), 7.69 (d, *J =* 7.4 Hz, 1H), 7.51 (t, *J =* 7.2 Hz, 1H), 7.40 (d, *J =* 8.4 Hz, 1H), 7.11 (t, *J =* 7.4 Hz, 1H), 6.91 (t, *J =* 52.3 Hz, 1H), 5.41 (s, 2H).

### Example 110: Synthesis of Compound 110

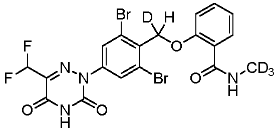

### Synthetic route:

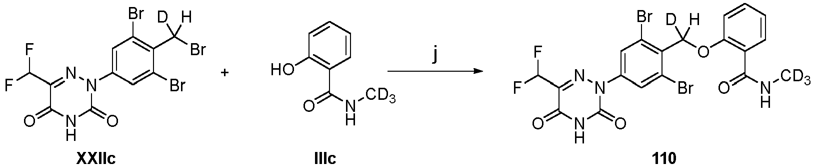

With reference to the synthetic route of Example 106, compound IIIb was replaced with compound IIIc to synthesize compound 110 (32.2 mg, yield 25.0%) as a yellow solid, MS(ESI, m/z): 564.7[M+H]⁺.

¹H NMR (400 MHz, DMSO-*d*₆) δ 12.84 (s, 1H), 7.97 (s, 2H), 7.80 - 7.76 (m, 1H), 7.69 (dd, *J =* 7.6 Hz, 1.6 Hz, 1H), 7.54 - 7.49 (m, 1H), 7.40 (d, *J =* 8.0 Hz, 1H), 7.11 (t, *J =* 7.6 Hz, 1H), 6.92 (t, *J* = 52.4 Hz, 1H), 5.40 (d, *J* = 8.4 Hz, 1H).

### Example 111: Synthesis of Compound 111

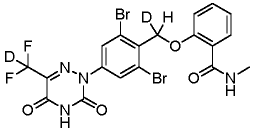

### Synthetic route:

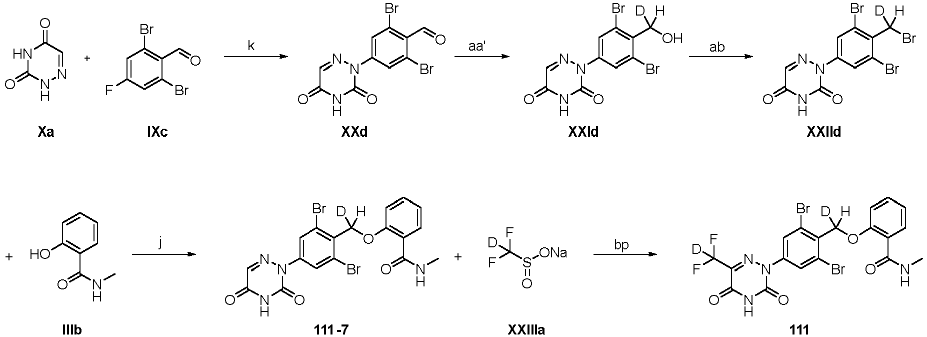

With reference to the synthetic route of Example **106,** compound **Xd** was replaced with compound **Xa** to afford compound **111-7.** Thereafter, the synthetic route of Example **107** was followed to synthesize compound **111** (12.3 mg, yield 12.4%) as a yellow solid, MS(ESI, m/z): 562.7[M+H]⁺.

¹H NMR (400 MHz, DMSO-d6) δ 12.83 (s, 1H), 7.97 (s, 2H), 7.84 - 7.78 (m, 1H), 7.69 (d, *J* = 7.6 Hz, 1H), 7.54 - 7.50 (m, 1H), 7.40 (d, *J =* 8.4 Hz, 1H), 7.12 (t, *J =* 7.6 Hz, 1H), 5.40 (d, *J =* 8.4 Hz, 1H), 2.69 (d, *J* = 4.8 Hz, 3H).

### Example 112: Synthesis of Compound 112

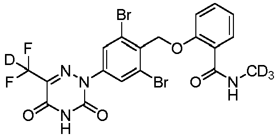

### Synthetic route:

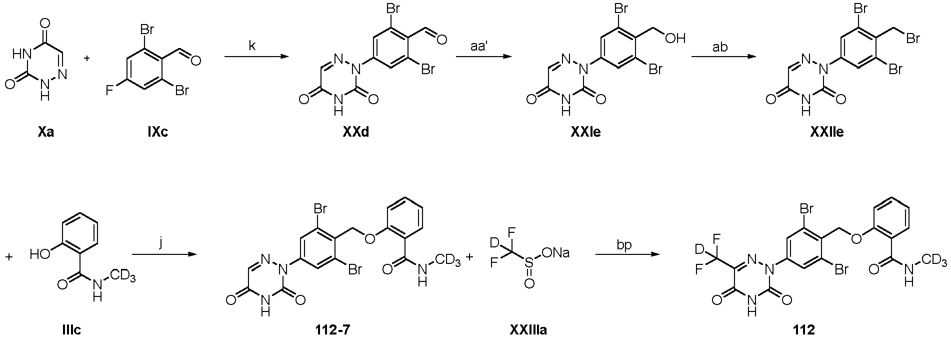

With reference to the synthetic route of Example **111,** the deuterated sodium borohydride in step **aa'** was replaced with sodium borohydride, and compound **IIIb** was replaced with compound **IIIc** to synthesize compound **112** (11.2 mg, yield 8.4%) as a white solid, MS(ESI, m/z): 564.7[M+H]⁺.

¹H NMR (400 MHz, DMSO-d6) δ 12.85 (s, 1H), 7.97 (s, 2H), 7.78 (s, 1H), 7.70 (dd, *J =* 8.0 Hz, 2.0 Hz, 1H), 7.54 - 7.50 (m, 1H), 7.40 (d, *J =* 8.4 Hz, 1H), 7.12 (t, *J =* 7.6 Hz, 1H), 5.42 (s, 3H).

### Example 113: Synthesis of Compound 113

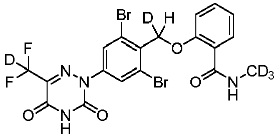

### Synthetic route:

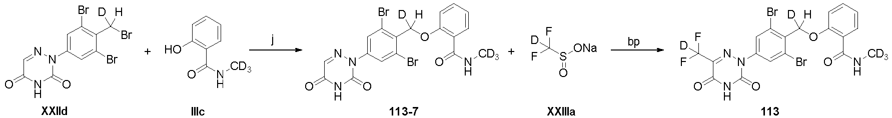

With reference to the synthetic route of Example **112,** compound **XXIIe** was replaced with compound **XXIId** to synthesize compound **113** (12.7 mg, yield 10.6%) as a white solid, MS(ESI, m/z): 565.7[M+H]⁺.

¹H NMR (400 MHz, DMSO-d6) δ 12.85 (s, 1H), 7.97 (s, 2H), 7.83 - 7.75 (m, 1H), 7.70 (d, *J* = 7.6 Hz, 1H), 7.56 - 7.48 (m, 1H), 7.44 - 7.36 (m, 1H), 7.16 - 7.08 (m, 1H), 5.43 - 5.37 (m, 1H).

### Comparative Example 1: Synthesis of Compound 114

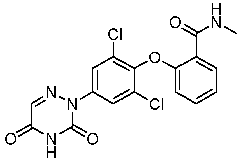

### Synthetic route:

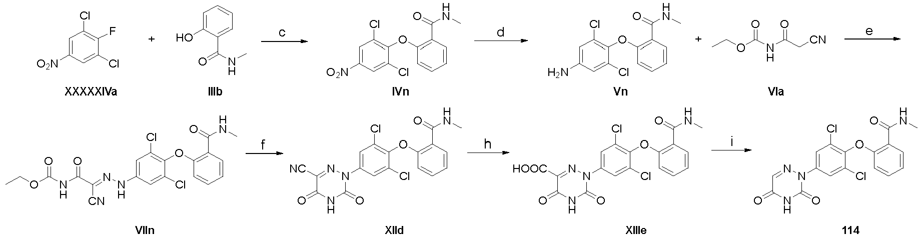

The synthetic methods of Example 1 and Example **14** were followed to afford compound **114** (83.3 mg, yield 46.0%) as a white solid. MS(ESI, m/z): 407[M+H]+.

1H NMR (400 MHz, DMSO-d6) 12.48 (s, 1H), 8.17 (s, 1H), 7.87 - 7.79 (m, 3H), 7.70 (s, 1H), 7.37 (t, *J =* 6.8 Hz, 1H), 7.16 (t, *J =* 6.8 Hz, 1H), 7.49 (d, *J =* 6.8 Hz, 1H), 2.82 (s, 3H).

### Bioactivity Assay

### Experimental Example 1: Detection of THRα and THRβ Agonist Activities of Compounds Based on Reporter Gene Activity Assay Method

### 1. Method

### 1.1 Construction and preparation of plasmids pGAL4-THR-LBD and pG5-Luc

The pGAL4-THRα-LBD and pGAL4-THRβ-LBD plasmids used in the reporter gene assay system were constructed by a conventional molecular cloning method comprising the following main steps: the cDNA sequences of THRα (NM_003250) and THRβ (NM_000461) corresponding to the amino acid sequences of THRα (163-407AA) and THRβ (217-461AA) were inserted into the BamHI and NotI enzyme digestion sites of the pGAL4 vector by the PCR technique respectively to obtain the pGAL4-THRα-LBD and pGAL4-THRβ-LBD plasmids; the pG5-Luc (#E249A) and pRL-TK (#E2241) plasmids were purchased from Promega; the plasmids were transfected into the *E. coli* DH5α by the CaCl₂ method, and after further culture and amplification, the plasmids were purified by Plasmid Extraction Kits (TIANGEN, #D107) to obtain the corresponding plasmid DNAs.

### 1.2 Co-transfection of plasmids into HEK293T cells and treatment with compounds

The HEK293T cells were inoculated into a 96-well plate at a density of 1×10⁴/well the day before plasmid transfection. Cell transfection was conducted according to the instructions of the transfection reagent FuGENE^{®} HD (Promega, # E2311). The main steps were as follows: taking one well as an example here, the plasmids pGAL4-THRα-LBD or pGAL4-THRβ-LBD, pG5-Luc, and pRL-TK were added in the proportions of 20 ng, 50 ng, and 5 ng to 10 uL of Opti-MEM^{™} I medium (Gibco, #11058021) and mixed well; subsequently, 0.25 uL of FuGENE^{®} HD was added, mixed well, and then left at room temperature for 5 min; afterwards, 10 uL of the mixture was added to the cell wells containing 100 uL of culture medium. After cell co-transfection for 6 h, the compound was diluted in a 3-fold gradient with dimethyl sulfoxide at the highest concentration of 1 uM. The compound was added to the cell culture medium at a total of 10 concentrations for treatment for 24 h. There were a total of two duplicate wells for each concentration. Triiodothyronine (T3) was used as the positive control.

### 1.3 Dual-Glo Luciferase Assay

After the cells were treated with the compound for 24 h, the cells were assayed according to the instructions of Dual-Glo^{®} Luciferase Assay System (Promega, # E2940). The main steps were as follows: 50 uL of culture medium was aspirated from each well and discarded, and then 50 uL of Dual-Glo^{®} Luciferase reagent was added and oscillated at room temperature for 10 min; 80 uL of lysis reaction solution was measured into a white opaque optiPlate-96-well plate, and the luminescence signal value (Firefly-Luc) of the Firefly luciferase was detected using an MD i3x multi-mode microplate reader; 40 uL of Dual-Glo^{®} Stop & Glo^{®} reagent was then added, and oscillated at room temperature for 10 min; the luminescence signal value (Renilla-Luc) of the Renilla luciferase was then detected using an MD i3x multi-mode microplate reader. The Firefly-Luc/Renilla-Luc ratio was used as the activation activity of the compound against THR, and the ratio in the solvent DMSO group was used for normalization; the dose-response curve was fitted with four parameters using the GraphPad Prism6.0 software, and the EC50 value was calculated.

### 2. Results

Resmetirom (MGL-3196) was an oral liver-targeted and highly selective THR-β agonist, so MGL-3196 was used as a control compound herein for illustrating the bioactivities of the compounds of the present application.

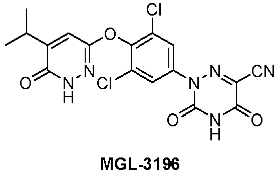

The experimental data suggested that the compounds of the present disclosure exhibited a stronger THRβ agonist activity and certain THRα/β selectivity. The specific data were listed in Table 1.

**Table 1**

| **Name of Compound** | THRα Agonist Activity EC50 (µM) | THRβ Agonist Activity EC50 (µM) | Selectivity (THRα/THRβ) |
|---|---|---|---|
| **MGL3196** | * | ** | † |
| **14** | *** | **** | † |
| **17** | **** | ***** | † |
| **25** | *** | ***** | † |
| **29** | ** | *** | † |
| **32** | ** | ** | † |
| **37** | ** | *** | † |
| **38** | *** | ***** | †† |
| **39** | ***** | ***** | † |
| **43** | **** | ***** | † |
| **45** | *** | **** | † |
| **46** | *** | ***** | † |
| **47** | **** | ***** | † |
| **51** | **** | ***** | † |
| **52** | * | ** | † |
| **53** | **** | ***** | † |
| 54 | ** | *** | † |
| **56** | **** | ***** | †† |
| **57** | **** | ***** | † |
| **59** | **** | ***** | † |
| **60** | ** | ** | † |
| **61** | *** | **** | † |
| **62** | *** | ***** | †† |
| **63** | **** | ***** | † |
| **64** | **** | **** | † |
| **68** | *** | ***** | † |
| **69** | *** | **** | † |
| **70** | * | *** | †† |
| **76** | **** | ***** | † |
| **82** | ** | ***** | †† |
| **83** | ** | ***** | †††† |
| **84** | ***** | ***** | † |
| **85** | ** | ***** | †††† |
| **86** | *** | ***** | †† |
| **87** | **** | ***** | † |
| **88** | ***** | ***** | † |
| **89** | ***** | ***** | † |
| **90** | **** | ***** | ††† |
| **91** | ***** | ***** | ††† |
| **93** | ***** | ***** | † |
| **99** | * | ***** | †††† |
| **100** | * | ***** | †††† |
| **101** | *** | ***** | † |
| **102** | *** | ***** | †† |
| **103** | ***** | ***** | † |
| **104** | **** | ***** | †† |
| **105** | ***** | ***** | † |
| **106** | ***** | ***** | † |
| **107** | **** | ***** | † |
| **108** | **** | ***** | † |
| **109** | ***** | ***** | † |
| **110** | ***** | ***** | † |
| **111** | ***** | ***** | † |
| **112** | ***** | ***** | † |
| 113 | **** | ***** | † |

| | | | |
|---|---|---|---|
| *: 150 µM ≥ EC50 > 40 µM; **: 40 µM ≥ EC50 > 20 µM; ***: 20 µM ≥ EC50 > 10 µM; ****: 10 µM ≥ EC50 > 5 µM; *****: 5 µM ≥ EC50 †: 5 ≥ THRα/β; ††: 10 ≥ THRα/β > 5; †††: 20 ≥ THRα/β > 10; ††††: 100 ≥ THRα/β > 20 | | | |

### Experimental Example 2: Detection of Agonist Activities of Compounds Against THRα/β Based on Time-Resolved Fluorescence Resonance Energy Transfer (THR-FRET)

### 1. Construction of THRα/β overexpression vectors

By visiting NCBI, the THRα/β LBD domain sequences were found. The pET21-His-GST-dLBT-THRα LBD and pET21-His-GST-dLBT-THRβ LBD overexpression vectors were constructed by the fusion method, and sequenced to confirm the accuracy of the sequences.

### 2. Prokaryotic expression of recombinant proteins in E. coli

The correctly sequenced **THRα** LBD and THRβ LBD overexpression vectors were transfected into *E. coli* cells BL21 (DE3), and spread on an agar plate with ampicillin. Monoclones were picked out and amplified in an LB medium, and transferred into 1L LB at a ratio of 1:100 for large-scale culture. When the OD value was at 0.8 to 1.2, 0.5 mM isopropyl-beta-D-thiogalactopyranoside (IPTG) was added, and induced overnight at 18°C. The bacteria were harvested, disrupted, and purified via the GST column and the molecular sieve to obtain two proteins His-GST-dLBT-THRα LBD and His-GST-dLBT-THRβ LBD. The protein concentrations were determined to be 24 µM and 23 µM respectively by Bradford Protein Assay Kit of Sangon Biotech.

### 3. Preparation of compounds and formulation of reaction system

The proteins were taken out from a refrigerator at -80°C. The proteins with the GST-tagged THRα/β LBD domain and the Eu-labelled GST antibodies were thawed slowly on ice, and an assay buffer containing a final concentration of 5 mM dithiothreitol (DTT) was formulated.

### 3.1 Preparation of compounds

The starting concentration of the compound was 100 µM (in DMSO). The compound (100 µM in DMSO) was subjected to 3-fold isogradient dilution with DMSO to obtain a total of 11 isogradient concentrations. Subsequently, the above compounds at the isogradient concentration were further subjected to 50-fold dilution with an assay buffer containing 5 mM DTT.

### 3.2 Formulation of THR-FRET reaction system

The final concentrations of all components were calculated based on a system with a final volume of 20 µL per well. To 18 µL of assay buffer containing 5 mM DTT, the GST-tagged THRα/β proteins, the SRC2 (LKEKHKILHRLLQDSSSPV) polypeptide, XL665 (Cisobio, #610SAXLB), and the Eu-labeled GST antibody were added, and the final concentrations were 2 nM, 200 nM, 0.05 nM, and 7.6 nM respectively to formulate 18 µL of reaction mixture of the proteins, polypeptide, and antibody per well.

18 µL of reaction mixture and 2 µL of diluted compound were added into an optiplate-384-well plate, and reacted at room temperature for 24 h.

### 3.3 Plate reading

The plate was read using an MD i3X multi-mode microplate reader with the excitation and emission wavelengths of 340 nm and 665 nm, respectively. The intensity of light at a wavelength of 616 nm generated by europium excited by 340 nm wavelength light from the MD i3X multi-mode microplate reader was used as the background. According to the different activation degrees of different compounds against THRα and THRβ, the intensity of the emission light at a wavelength of 665 nm generated by exciting XL665 with the exciting light at a wavelength of 616 nm was varied. The intensity ratio between these two light wavelengths (665 nm, 616 nm) was used as the activation activity of the compound against THRα or THRβ, and the ratio in the solvent DMSO group was used for normalization. The dose-response curve was fitted with four parameters using the GraphPad Prism 6.0 software, and the EC50 value was calculated.

### 4. Results

The experimental data suggested that the compounds of the present disclosure exhibited a stronger THRβ agonist activity and certain THRα/β selectivity. The specific data were listed in Table 2.

**Table 2**

| **Name of Compound** | THRα Agonist Activity EC50 (µM) | THRβ Agonist Activity EC50 (µM) | Selectivity (THRα/THRβ) |
|---|---|---|---|
| **MGL3196** | *** | **** | †† |
| **1** | *** | **** | †† |
| **2** | *** | **** | †† |
| **6** | * | *** | ††† |
| 7 | * | **** | ††† |
| **9** | **** | ***** | †† |
| **10** | * | ** | ††† |
| **11** | **** | ***** | †† |
| **12** | **** | ***** | †† |
| **13** | ** | **** | †† |
| **14** | **** | **** | †† |
| **17** | **** | ***** | †† |
| **18** | ** | **** | ††† |
| **19** | * | ** | ††† |
| **20** | * | **** | ††† |
| **22** | * | ** | ††† |
| **24** | * | **** | †† |
| **25** | **** | ***** | ††† |
| **26** | * | **** | ††† |
| **29** | *** | **** | ††† |
| **33** | ** | **** | ††† |
| **38** | **** | ***** | †† |
| **42** | **** | **** | †† |
| **45** | **** | ***** | †† |
| **53** | **** | ***** | †† |
| **54** | **** | ***** | †† |
| **55** | * | **** | †† |
| **69** | * | **** | ††† |
| **106** | ***** | ***** | †† |
| **107** | ***** | ***** | †† |
| **108** | ***** | ***** | †† |
| **109** | ***** | ***** | †† |
| **110** | ***** | ***** | †† |
| **111** | ***** | ***** | †† |
| **112** | ***** | ***** | †† |
| **113** | ***** | ***** | †† |
| **114** | * | * | †† |

| | | | |
|---|---|---|---|
| *: 250 µM ≥ EC50 > 20 µM; **: 20 µM ≥ EC50 > 10 µM; ***: 10 µM ≥ EC50 > 5 µM; ****: 5 µM ≥ EC50 > 0.5 µM; *****: 0.5 µM ≥ EC50 ††: 10 ≥ THRα/β; †††: 20 ≥ THRα/β > 10 | | | |

## Claims

1. A compound having a structure of formula (1) or a pharmaceutically acceptable form thereof: 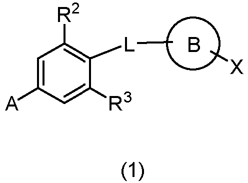 wherein
A is 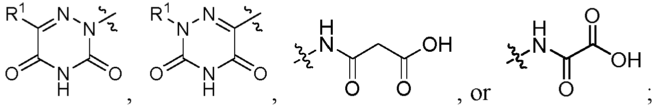
R¹ is H, halogen, -CN, -NH₂, -NO₂, -OH, or C₁₋₆ alkyl, said C₁₋₆ alkyl is optionally substituted with one or more substituents that are independently deuterium, halogen, -CN, -NH₂, -NO₂, or -OH;
R² and R³ are independently H, halogen, -CN, -NH₂, -NO₂, -OH, or C₁₋₆ alkyl, said C₁₋₆ alkyl is optionally substituted with one or more substituents that are independently halogen, -CN, -NH₂, -NO₂, or -OH;
L is -(C₁₋₄ alkylene)-, -(C₁₋₄ alkylene)-O-, -(C₁₋₄ alkylene)-S-, -(C₁₋₄ alkylene)-NH-, -O-(C₁₋₄ alkylene)-, -S-(C₁₋₄ alkylene)-, -NH-(C₁₋₄ alkylene)-, or -CH=CH-; said alkylene is optionally substituted with one or more substituents that are independently deuterium, halogen, -CN, -NH₂, -NO₂, or -OH;
ring B is a benzene ring, a naphthalene ring, a furan ring, a thiophene ring, or a pyrrole ring; ring B is optionally substituted with one or more R⁴;
each R⁴ is independently H, halogen, -CN, -NH₂, -NO₂, -OH, C₁₋₆ alkyl, C₁₋₆ alkoxy, 5- to 10-membered heteroaryl, C₅₋₈ cycloalkenyl, or C₃₋₈ cycloalkyl, said C₁₋₆ alkyl, C₁₋₆ alkoxy, 5-to 10-membered heteroaryl, or C₃₋₈ cycloalkyl is optionally substituted with one or more substituents that are independently halogen, -CN, -NH₂, -NO₂, or -OH;
X is -C(=O)NR⁵R⁶, -COOH, or 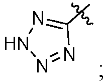
R⁵ and R⁶ are independently H, -OH, -S(=O)₂R⁷, C₁₋₆ alkyl, C₁₋₆ alkoxy, C₆₋₁₀ aryl, or C₃₋₈ cycloalkyl, said -S(=O)₂R⁷, C₁₋₆ alkyl, C₁₋₆ alkoxy, C₆₋₁₀ aryl, or C₃₋₈ cycloalkyl is optionally substituted with one or more substituents that are independently deuterium, halogen, -CN, -NH₂, -NO₂, or -OH;
R⁷ is H or C₁₋₆ alkyl; and
the pharmaceutically acceptable form is selected from the group consisting of a pharmaceutically acceptable salt, ester, stereoisomer, tautomer, solvate, nitrogen oxide, isotope-labelled compound, metabolite, and prodrug.

2. The compound or the pharmaceutically acceptable form thereof according to claim 1, wherein
R¹ is H, F, Cl, Br, -CN, -NH₂, or C₁₋₄ alkyl, said C₁₋₄ alkyl is optionally substituted with one or more substituents that are independently deuterium, F, Cl, Br, -CN, -NH₂, or -OH;
preferably, R¹ is H, -CN, -NH₂, -CH₃, -CH₂F, -CHF₂, -CDF₂, or -CF₃.

3. The compound or the pharmaceutically acceptable form thereof according to claim 1 or 2, wherein

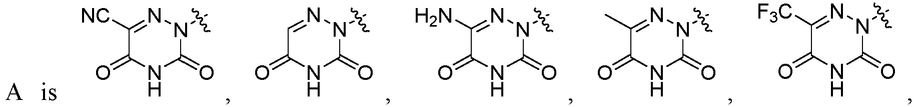

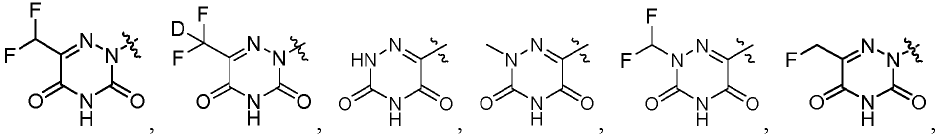

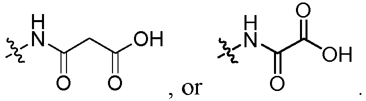

4. The compound or the pharmaceutically acceptable form thereof according to any one of claims 1 to 3, wherein
R² and R³ are independently H, F, Cl, Br, -CN, -NH₂, or C₁₄ alkyl, said C₁₋₄ alkyl is optionally substituted with one or more substituents that are independently F, Cl, Br, -CN, -NH₂, -NO₂, or -OH;
preferably, R² and R³ are independently H, F, Cl, Br, or -CH₃.

5. The compound or the pharmaceutically acceptable form thereof according to any one of claims 1 to 4, wherein
L is -(C₁₋₃ alkylene)-, -(C₁₋₃ alkylene)-O-, -(C₁₋₃ alkylene)-S-, -(C₁₋₃ alkylene)-NH-, -O-(C₁₋₃ alkylene)-, -S-(C₁₋₃ alkylene)-, -NH-(C₁₋₃ alkylene)-, or -CH=CH-; said alkylene is optionally substituted with one or more substituents that are independently deuterium, F, Cl, Br, or -OH;
preferably, L is -C(D)H-O-, -CD₂-O-, -CH₂-O-, -CH₂-S-, -CH₂-NH-, -CH₂-CH₂-, -O-CH₂-, -S-CH₂-, -NH-CH₂-, or -CH=CH-.

6. The compound or the pharmaceutically acceptable form thereof according to any one of claims 1 to 5, wherein
ring B is a benzene ring, a naphthalene ring, or a thiophene ring; ring B is optionally substituted with one or more R⁴;
preferably, ring B is 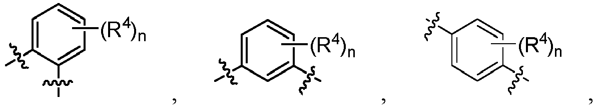 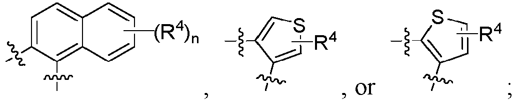 n is 0, 1, 2 or 3;
each R⁴ is independently H, F, Cl, Br, -CN, -NH₂, C₁₋₄ alkyl, C₁₋₄ alkoxy, 5- to 8-membered heteroaryl, C₅₋₈ cycloalkenyl, or C₃₋₆ cycloalkyl, said C₁₋₄ alkyl, C₁₋₄ alkoxy, 5- to 8-membered heteroaryl, C₅₋₈ cycloalkenyl, or C₃₋₆ cycloalkyl is optionally substituted with one or more substituents that are independently F, Cl, Br, -CN, -NH₂, or -OH;
preferably, each R⁴ is independently H, F, Cl, Br, -CN, -CH₃, -OCH₃, -CF₃,

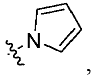

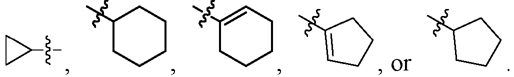

7. The compound or the pharmaceutically acceptable form thereof according to any one of claims 1 to 6, wherein
R⁵ and R⁶ are independently H, -OH, -S(=O)₂R⁷, C₁₋₄ alkyl, C₁₋₄ alkoxy, C₆₋₁₀ aryl, or C₃₋₆ cycloalkyl, said -S(=O)₂R⁷, C₁₋₄ alkyl, C₁₋₄ alkoxy, C₆₋₁₀ aryl, or C₃₋₆ cycloalkyl is optionally substituted with one or more substituents that are independently deuterium, F, Cl, Br, -CN, -NH₂, or -OH; R⁷ is H or C₁₋₄ alkyl;
preferably, R⁵ and R⁶ are independently H, -CH₃, -CD₃, -CH(CH₃)₂, -CH₂CH₃, -OCH₃, -OH, -S(=O)₂CH₃,

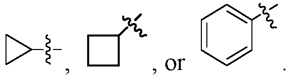

8. The compound or the pharmaceutically acceptable form thereof according to any one of claims 1 to 7, which is a compound having a structure of formula (2), formula (3), formula (4) or formula (5), or a pharmaceutically acceptable form thereof: 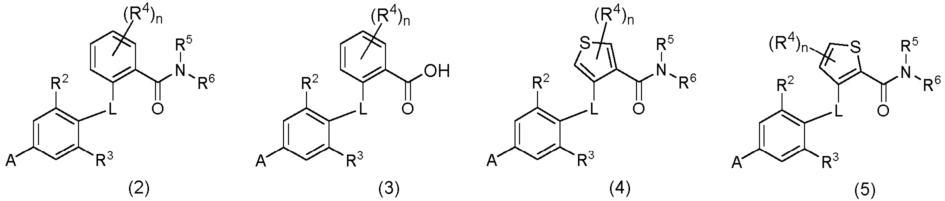 wherein A, R², R³, R⁴, L, n, R⁵*,* and R⁶ are as defined in any one of claims 1 to 7.

9. The compound or the pharmaceutically acceptable form thereof according to any one of claims 1 to 8, which is a compound having a structure of formula (6), formula (7), formula (8), formula (9) or formula (10), or a pharmaceutically acceptable form thereof: 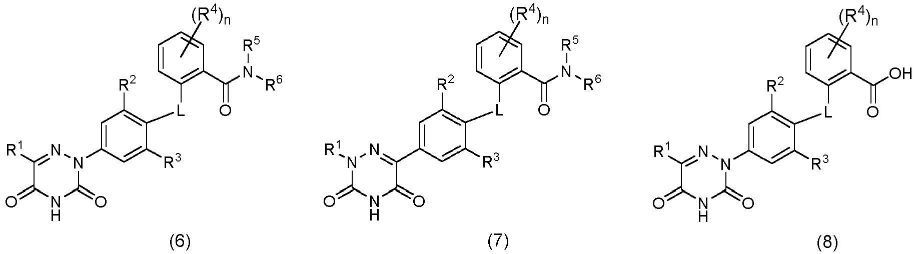 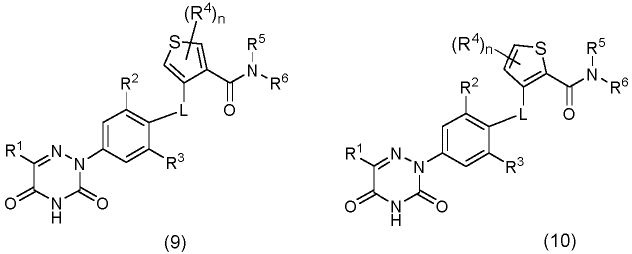 wherein R¹, R², R³, R⁴, L, n, R⁵, and R⁶ are as defined in any one of claims 1 to 8.

10. A compound or a pharmaceutically acceptable form thereof, wherein the compound is selected from the group consisting of: 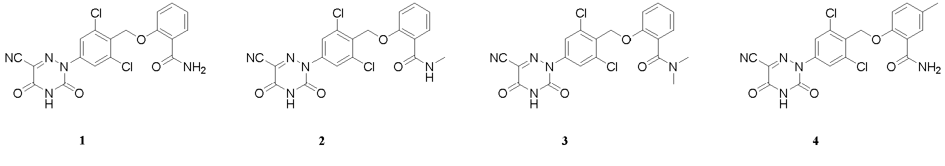 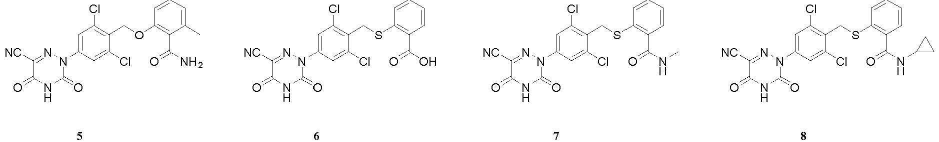 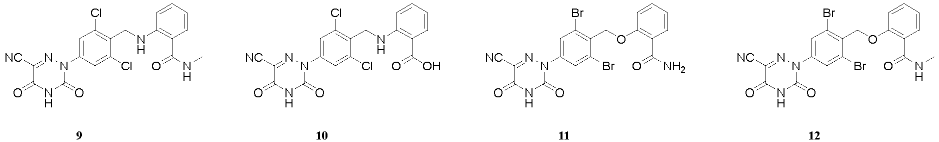 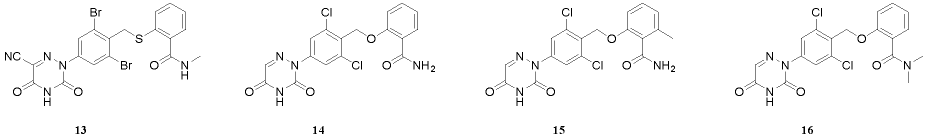 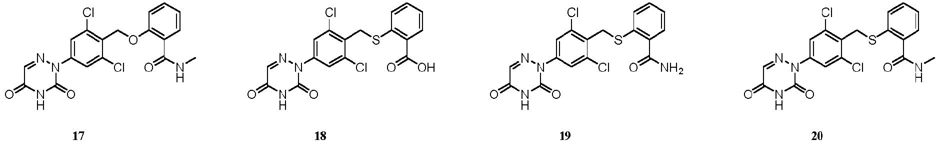 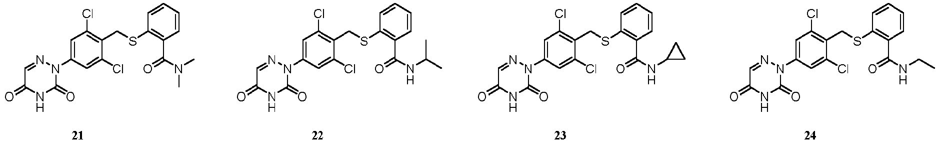 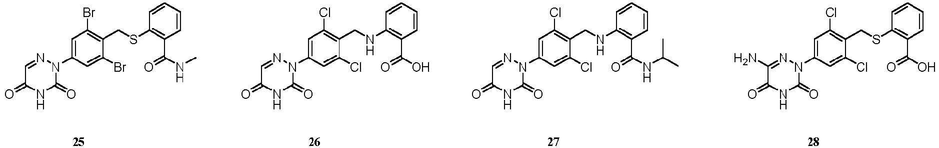 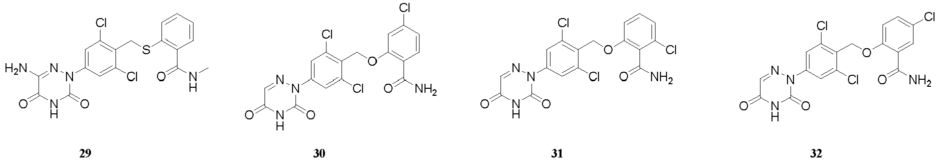 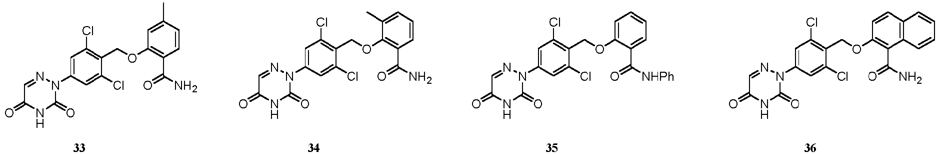 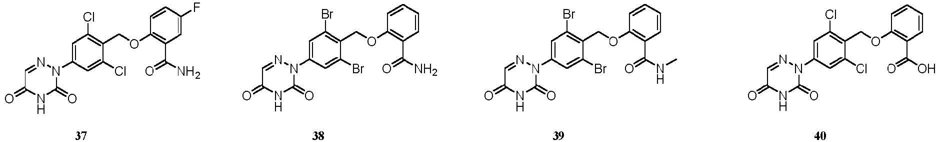 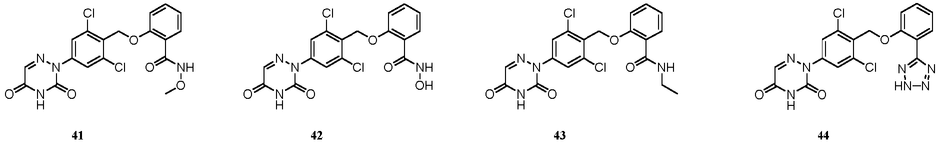 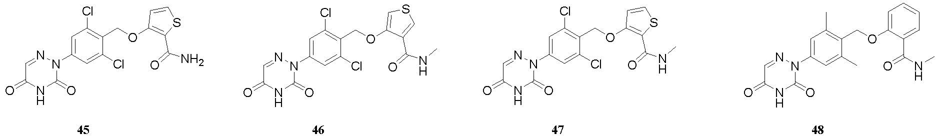 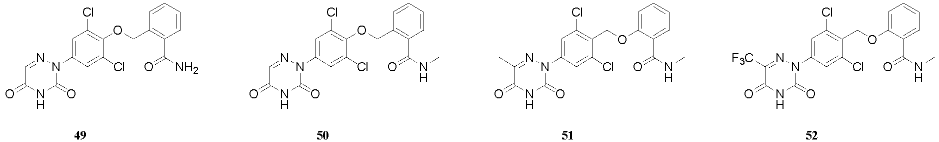 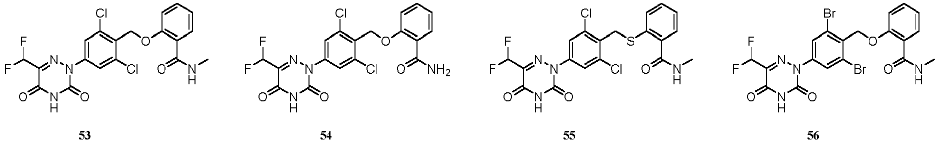 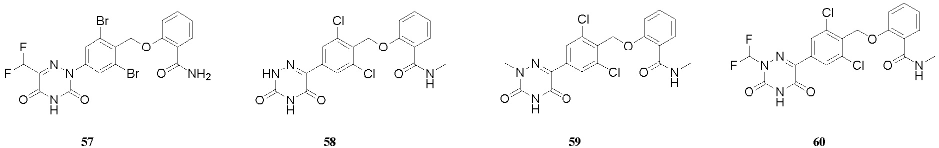 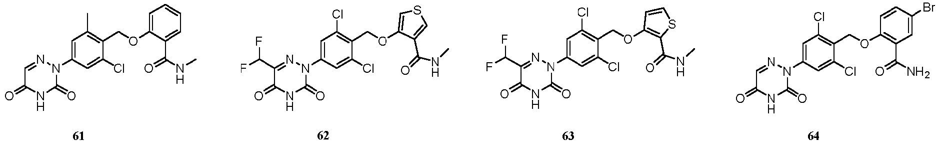 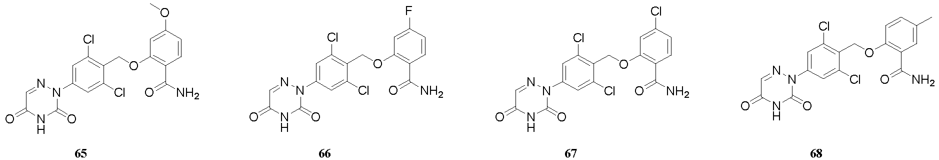 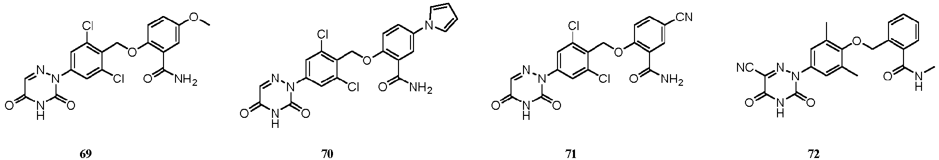 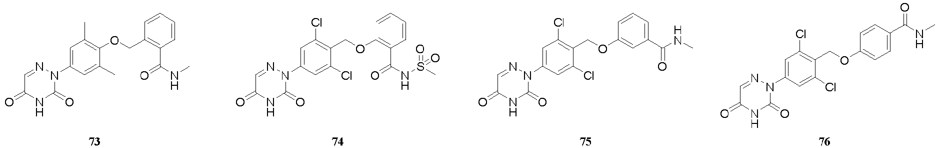 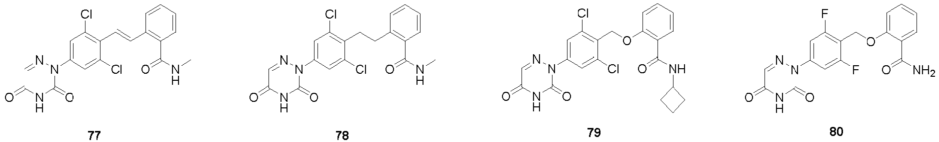 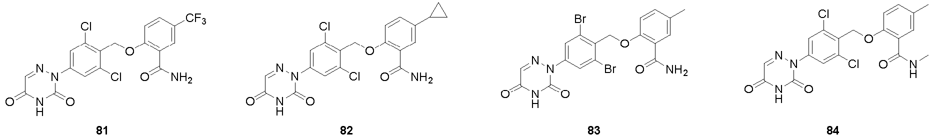 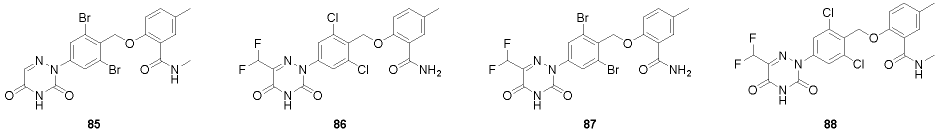 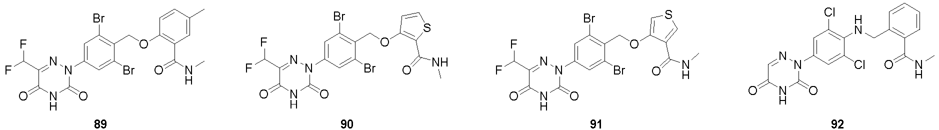 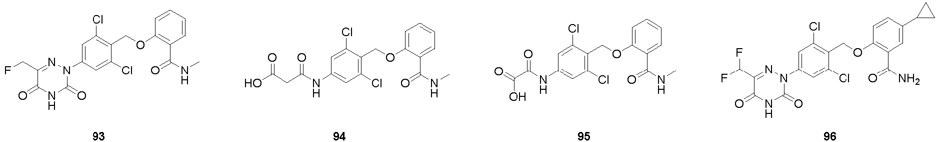 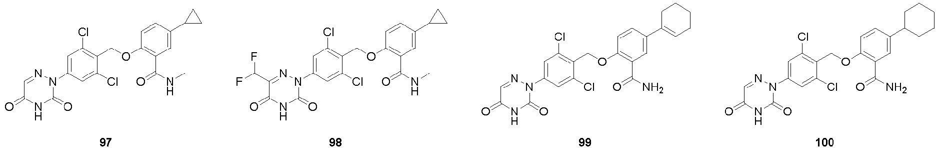 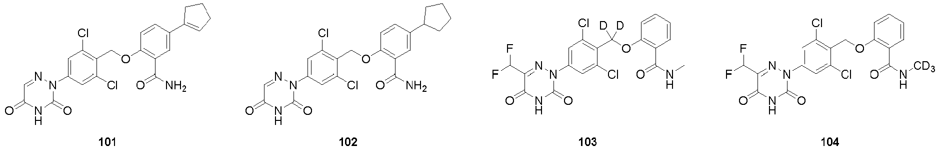 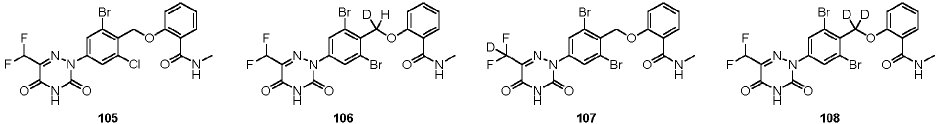 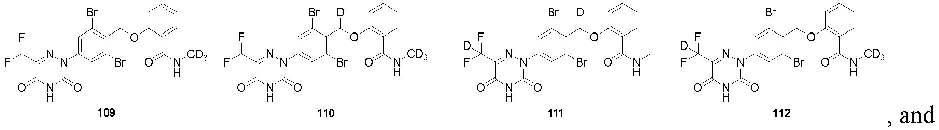 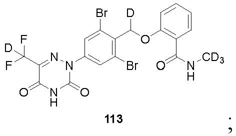 the pharmaceutically acceptable form is selected from the group consisting of a pharmaceutically acceptable salt, ester, stereoisomer, tautomer, solvate, nitrogen oxide, isotope-labelled compound, metabolite, and prodrug.

11. A pharmaceutical composition, comprising the compound or the pharmaceutically acceptable form thereof according to any one of claims 1 to 10, and one or more pharmaceutically acceptable carriers.

12. Use of the compound or the pharmaceutically acceptable form thereof according to any one of claims 1 to 10, or the pharmaceutical composition according to claim 11 in the preparation of a medicament for preventing and/or treating a disease or condition at least partially mediated by a thyroid hormone β receptor.

13. The use according to claim 12, wherein the disease is a metabolic disease.

14. The use according to claim 13, wherein the disease is a nonalcoholic fatty liver disease, dyslipidemia, atherosclerosis, or hypothyroidism.
